# EUROPEAN PATENT APPLICATION

(11) **EP 4 480 493 A1**
(43) Date of publication of application: **25.12.2024**
(21) Application number: 23181084.7
(22) Date of filing: 22.06.2023
(51) Int. Cl.: A61K 38/55, C12N 15/81, A61P 11/00

(54) **A RECOMBINANT HUMAN ALPHA-1 ANTITRYPSIN GLYCOPROTEIN FOR TREATING NON-VIRAL INFLAMMATORY DISEASES OF THE LUNG**

(71) Applicant: AATEC Medical GmbH, 80639 München (DE)
(72) Inventor: Jankowsky, Rüdiger, 80997 München (DE); Stangl, Manfred, 82054 Sauerlach (DE); Strassmair, Michael, 85662 Hohenbrunn (DE)
(74) Representative: Hertin und Partner Rechts- und Patentanwälte PartG mbB

(57) **Abstract**

The invention relates to a human recombinant AAT (rhAAT) protein or fragment thereof, expressed in a genetically modified yeast, for use in the treatment and/or prevention of a non-viral disease of the lung associated with inflammation and/or a pathological immune response. In embodiments the rhAAT or fragment has one or more N-linked glycosylations, comprising at least one HexNAc1 glycosylation. In other embodiments, the rhAAT glycoprotein or fragment thereof comprises at least one Hex9HexNAc2, Hex10HexNAc2, Hex11HexNAc2, Hex12HexNAc2, Hex13HexNAc2, Hex14HexNAc2, Hex15HexNAc2 and/or Hex16HexNAc2 glycosylation. In embodiments the invention further relates to a protein preparation comprising recombinant human alpha1-antitrysin (rhAAT) glycoproteins or fragments thereof with heterologous N-linked glycosylations. The invention further relates to a pharmaceutical composition comprising the rhAAT protein or fragment thereof or a protein preparation for use in the treatment of a non-viral disease of the lung associated with inflammation and/or a pathological immune response.

## Description

The invention is in the field of recombinant glycoprotein production, including methods of yeast fermentation, recombinant protein expression and purification, and medical uses of said recombinant glycoproteins. In particular the invention relates to the use of said recombinant glycoproteins in the treatment and/or prevention of non-viral, inflammatory diseases of the lung.

The invention therefore relates to a human recombinant AAT (rhAAT) protein or fragment thereof, expressed in a genetically modified yeast, for use in the treatment and/or prevention of a nonviral disease of the lung associated with inflammation and/or a pathological immune response.

In embodiments the rhAAT or fragment has one or more N-linked glycosylations, comprising at least one HexNAc1 glycosylation. In other embodiments, the rhAAT glycoprotein or fragment thereof comprises at least one Hex9HexNAc2, Hex10HexNAc2, Hex11HexNAc2, Hex12HexNAc2, Hex13HexNAc2, Hex14HexNAc2, Hex15HexNAc2 and/or Hex16HexNAc2 glycosylation. In embodiments the invention further relates to a protein preparation comprising recombinant human alpha1-antitrysin (rhAAT) glycoproteins or fragments thereof with heterologous N-linked glycosylations.

The invention further relates to a pharmaceutical composition comprising the rhAAT protein or fragment thereof or a protein preparation for use in the treatment of a non-viral disease of the lung associated with inflammation and/or a pathological immune response.

### BACKGROUND OF THE INVENTION

Alpha-1 antitrypsin (also known as A1AT, AAT, PI, SERPINA1, AAT protein) is a ~52kDa glycoprotein that is one of the most abundant endogenous serine protease inhibitors (SERPIN superfamily). AAT is considered to be an acute phase protein, whereby AAT concentration can increase many fold upon acute inflammation.

Although known primarily for its anti-protease and anti-inflammatory activities, studies conducted over the past decade have cumulatively demonstrated that AAT is also an immune-modulator and a cytoprotective molecule. As such, microenvironments enriched with AAT were shown to contain reduced levels of pro-inflammatory cytokines, such as IL-1, IL-6, and TNF-α, and increased levels of anti-inflammatory mediators such as IL-1 receptor antagonist and IL-10. This phenomenon was also depicted in human PBMC in vitro studies, as well as in samples obtained from cystic fibrosis patients who received inhaled AAT. Concomitantly, AAT has been demonstrated to directly bind IL-8, and danger-associated molecular pattern molecules (DAMPs), such as extracellular HSP70 and gp96, which otherwise act as adjuvants to the associated immune responses (Lior et al., Expert Opinion on Therapeutic Patents, 2016).

AAT further has a significant effect on neutrophils via the IL-8/CXCR1 signaling pathway (Curley et al., Clinical Implications. Chest. 2016). It binds to IL-8 and thus prevents CXCR1 activation. At the same time, it reduces soluble immune complexes which cause chemotaxis of neutrophils via binding to the ADAM-17 (Bergin et al., J Clin Invest., 2010). Reduction of TNF-α, free radicals as well as neutrophil elastase in tissues, in addition to modulating the immune response, reduces destruction of the pulmonary court. AAT further reduces the secretion of IL-1β from monocytes by down-regulating the receptor P2X7 and increases the level of cAMP. Simultaneously, it stimulates the secretion of the anti-inflammatory IL-1R from macrophages (Janciauskiene et al., J Biol Chem. 2007). The immunomodulatory effect of AAT is not limited to macrophages and neutrophils. It has been shown to reduce the effect of TNF-α on endothelial cells of lung micro vessels. This slows down a breakdown of the fluid barrier of the alveoli with consequent development of pulmonary oedema (Lockett et al., Am J Respir Cell Mol Biol. 2013).

In vitro, the neutrophil extracellular trap (NET) has been shown to disrupt the integrity of the cell membrane of lung epithelial cells. This results in cell death via apoptosis. At the same time, the cellular connections of the epithelial cells are disrupted. This results not only in damage to the lung tissue but also in the risk of micro thrombi forming. AAT counteracts this mechanism by forming a complex with NET as well as neutrophil elastase (Hudock et al., Front Immunol. 2023).

Surprisingly, the anti-inflammatory qualities of AAT still allow innate immune cells to respond to authentic threats; macrophages readily phagocytose bacteria, neutrophils decontaminate infected sites, and antigen-loaded dendritic cells migrate to draining lymph nodes. T lymphocytes, on the other hand, respond to an AAT-rich environment indirectly, pending stimulation from innate immunocytes. For example, AAT has been shown to elicit semi-mature antigen presenting cells that favor the expansion of protective regulatory T cells. B lymphocytes, which belong to both the innate and adaptive immune system, appear to exert a modified response in the presence of AAT, in the form of diminished isotype switching, which results in enhanced protective IgM production. It has been suggested that the reduction in bacterial burden, as depicted in CF patients for instance, during treatment with AAT may relate to enhanced anti-pathogen immune responses; at the same time, local tissues are spared from inappropriate excessive injury that might promote deleterious adaptive responses by elevating the levels of local DAMPs (Lior et al., Expert Opinion on Therapeutic Patents, 2016).

Inflammatory lung diseases and diseases associated with an unwanted, pathological immune response represent a significant cause of health problems and mortality worldwide and represent medical conditions that require improved modulation of inflammation in order to provide effective treatment. Nonviral inflammatory diseases of the lung may seriously impact the well-being of patients and encompass a number of pathological conditions affecting the tissues of the lung.

Asthma is a chronic inflammatory disease of the lung, characterized by coughing, wheezing, chest tightness and breathlessness and by reversible airway obstruction and bronchospasm as the most common symptoms (Huang et al., Cytokine 2021). Asthma is affecting over 300 million people worldwide including children and adults. Symptomatic treatment of asthma includes several drugs such as beta-2-agonist and glucocorticoids. However, there is no curative treatment available to date as the pathology of asthma is still not fully understood. In general oxygen radicals and autophagy have been reported to be involved in the pathophysiology of asthma.

Acute respiratory distress syndrome (ARDS) is a life-threatening condition. It is characterized by a massive disturbance of gas exchange due to impaired permeability of the pulmonary capillaries and tissue oedema due to an inflammatory reaction by neutrophil elastase. The severity of the disease varies depending on the degree of hyperinflammation. Especially the strong immune response in the early phase of the disease associated with a rapid and significant increase of proinflammatory cytokines and the infiltration of immune cells into the lung tissue requires early intervention in terms of immunomodulation. Thereby, the rapid increase of pro-inflammatory cytokines correlates with a poor prognosis for patients (Pugin et al., Am J Respir Crit Care Med. 1996). If the patient survives the early phase of the disease, a persistent, severe disturbance of lung function develops along this pathway.

Bronchiolitis obliterans (BO) also termed Bronchiolitis obliterans syndrome (BOS) is a rare, chronic, pulmonary disease characterized by inflammation and fibrous wall thickening of the bronchioles, narrowing their lumens and restricting air circulation (Jerkic et al., Can Respir J. 2020). BO emerges subsequent to pulmonary infection (Post infectious Bronchiolitis obliterans (PiBO)), or in combination with associated conditions, such as lung or allogeneic hematopoietic stem cell transplantations (chronic graft-versus-host disease) and is in this context termed Bronchiolitis obliterans syndrome (BOS) (Walther et al., Pediatr Pulmonol. 2020). The pathogenesis of BO is still largely unclear. However, a pathomechanism is conceivable in which an infiltration of the submucosa of the airways through leukocytes occurs. A cascade of inflammatory cytokines and mediators leads to an accumulation of inflammatory cells. Rosewich et al. showed a predominant neutrophilic inflammatory reaction in the bronchioles with increased concentrations of inflammatory cytokines such as IL-1β, IL-6, IL-8 and TNF-α (Rosewich et al., Cytokine 2015). In summary BO develops due to a complex reaction of the immune system involving cytokines, growth factors, chemokines and cells of the alloimmune reactivity, humoral immunity, autoimmunity and innate immunity.

Observations in induced sputum of PiBO patients demonstrated ongoing neutrophilic inflammation over time without termination (Eckrich et al., Lung 2016). Recent investigations also showed an upregulation of IL-33 in the sputum of the patients pointing to an involvement of alveolar epithelial type II cells (AEC-II) in the inflammatory process (Kriszeleit, doctoral thesis at the department of medicine of the Johann Wolfgang Goethe-Universität Frankfurt am Main 2023). AEC-II cells are damaged by many cellular and humoral inflammatory mediators in the development and progression of inflammatory induced lung injury. Vice versa, AEC-II are themselves able to amplify lung inflammation by producing inflammatory cytokines and chemokines, leading to the activation and recruitment of phagocytes and neutrophils (Standiford et al., J Clin Invest 1990).

BOS after lung transplantation is the leading cause of death after the first year posttransplant, whereby 48% and 76% of patients develop BOS within 5 and 10 years after transplantation, respectively (Weigt et al., Semin Respir Crit Care Med 2013). In addition to its impact on longterm survival, BOS causes significant morbidity, impairs quality of life, and increases costs for the healthcare system.

Given the uncontrolled inflammation in BO lung diseases, resolution of the chronic inflammatory response is of critical importance (Jerkic et al., Clin Transl Immunology. 2022). To date the most established therapy of BO is the treatment with Azithromycin, which is however associated with several side effects such as headache, diarrhoea and abdominal pain and further shows severe interactions with several drugs including antacids, digoxin, zidovudin, ergotamine, statins, marcumar, ciclosporin, terfenadine, cisaprid, astemizol, alfenatil and substances that prolong the QT time interval.

AAT reduced airway inflammation in other immune-mediated pulmonary diseases such as cystic fibrosis by decreasing the levels of neutrophile elastase activity (Griese et al., European Respiratory Journal 2007). However, to date, obtaining AAT still largely relies on purification from human plasma. For example, available methods include protein precipitation by addition of ammonium sulfate. The logical evolution in AAT purification was the combination of the ammonium sulfate fractionation with other procedures that could take advantage of the physicochemical properties of AAT. To make easier the separation of the large pool of proteins (typically the result of ammonium sulfate precipitation) into several smaller pools, one (or more) of which was enriched in AAT, the first parameter considered by investigators was protein charge. More recently, an increased purification level was observed with the advent of a wide range of sophisticated materials in the ion-exchange and affinity chromatography field. For example, in Morihara et al, after saturating human plasma with ammonium sulfate (80%), the pellet was dissolved in phosphate buffer pH 8.0, dialyzed, and loaded on an Affi-GEL Blue column. Two subsequent steps on a Zn-chelate column followed by a DE-ion exchange chromatography allowed producing homogeneous AAT.

Kwon et al. also disclose purification of recombinant AAT from yeast that was secreted in the medium as a glycosylated form. They developed a procedure that involved the precipitation of the protein with ammonium sulfate (60-75% saturation) followed by a series of subsequent chromatographic steps consisting of anion exchange (DEAE and mono Q columns) and affinity (A -Gel Blue column) chromatography. Although the yeast-produced AAT was fully functional as a protease inhibitor (compared with the plasma form), the molecular mass of this protein (unlike plasma AAT), once treated with endoglycosidase H, decreased to that of recombinant AAT produced in Escherichia coli. This indicated that the N-linked glycosylation of this form was a high mannose-type. However, Saccharomyces diastaticus is typically not ideal for secretory production of recombinant proteins (Purification and characterization of alpha 1-antitrypsin secreted by recombinant yeast Saccharomyces diastaticus. J. Biotechnol. 1995, 42, 191-195.).

Arjmand etal. 2011 (Avicenna J Med Biotechnol. 2011, 3(3): 127-134) discloses the expression and purification of recombinant human AAT in a methylotrophic Yeast *Pichia pastoris.* Human AAT was expressed in a secretary manner and under the control of inducible alcohol oxidase 1 (AOX1) promoter. The amount of AAT protein in medium was measured as 60 mg/L, 72 hours after induction with methanol. Arjmand et al. 2013 (Electronic Journal of Biotechnology, 2013, vol. 16, no. 1, 1-14) discloses the use of *P. pastoris* as a host for efficient production and secretion of recombinant AAT. The findings revealed that optimizing codon usage of the AAT gene for P. *pastoris* had positive effects on the level of secreted AAT under the control of inducible alcohol oxidase 1 (AOX1) and constitutive glycerol aldehyde phosphate dehydrogenase (GAP) promoters.

There are still many drawbacks to address regarding AAT isolation from human plasma, for example the abundance of albumin in human plasma is challenging, and the yield of AAT from human plasma is limited, thus optimized methods for producing secreted recombinant proteins are needed. Furthermore, recombinant production of AAT in yeast has until now been limited by relatively low yields, poor characterization of glycosylation and clinical applications are lacking.

Despite advances in purification of AAT from human plasma, and advances in recombinant production of AAT, to the knowledge of the inventors there has been no convincing solution to date to address the disadvantages of the prior art. Methods and means are required that reduce or avoid the problems associated with isolating AAT from human plasma, and novel means and uses of recombinant AAT are required. Means are still required to obtain a desired amount and purity level of AAT suitable for therapeutic use. Another problem which needs to be addressed in light of the prevalence and severity of non-viral lung diseases associated with inflammation and/or a pathological immune response, is the provision of effective means for prevention and or treatment of these medical conditions.

### SUMMARY OF THE INVENTION

In light of the prior art one technical problem underlying the present invention is the provision of improved or alternative means for treating and/or preventing non-viral medical conditions of the lung which are associated with inflammation and/or a pathological immune response.

Another problem underlying the invention was the provision of a disease modifying treatment of non-viral medical conditions of the lung which are associated with inflammation and/or a pathological immune response in a subject.

In light of the prior art, another technical problem underlying the invention was the provision of improved or alternative means for producing recombinant human AAT.

Another problem underlying the invention was the provision of means for producing recombinant human AAT providing higher yields than previously established with reduced cost and reduced limitations on production capacity.

These problems are solved by the features of the independent claims. Preferred embodiments of the present invention are provided by the dependent claims.

In one aspect the invention therefore relates to a human recombinant AAT (rhAAT) protein or fragment thereof, expressed in a genetically modified yeast, for use in the treatment and/or prevention of a non-viral disease of the lung associated with inflammation and/or a pathological immune response.

Although AAT production in yeast has been described previously, to the knowledge of the inventors the efficacy of rhAAT glycoprotein from yeast in treating a non-viral disease of the lung associated with inflammation and/or a pathological immune response has not been previously assessed. The present invention therefore represents a novel and surprisingly effective rhAAT glycoprotein preparation using yeast as an expression system. Correspondingly, the isolated rhAAT glycoprotein itself, the genetically modified yeast used to produce the recombinant AAT and methods of preparing and using the AAT produced in yeast may, in embodiments, be defined by the inventive finding of efficacy in treating a non-viral disease of the lung associated with inflammation and/or a pathological immune response such as asthma, acute respiratory distress syndrome (ARDS) or bronchiolitis obliterans syndrome (BOS).

In one embodiment the human recombinant AAT (rhAAT) protein is a glycoprotein.

In one embodiment the human recombinant AAT (rhAAT) protein is a human recombinant Alpha-1 antitrypsin (rhAAT) protein.

In one embodiment the human recombinant AAT (rhAAT) glycoprotein is a human recombinant Alpha-1 antitrypsin (rhAAT) glycoprotein.

In one embodiment the disease of the lung associated with inflammation and/or a pathological immune response is associated with an increased level of inflammatory cytokines, such as IL-1β, IL-1Ra, IL-4, IL-5, IL-6, IL-8, IFN-γ and/or TNF-α, an increased level of one or more transcription factors such as NF-κB and/or increased immune cell infiltration in the lung tissue of a subject compared to a healthy subject.

In one embodiment the disease of the lung associated with inflammation and/or a pathological immune response is associated with an increased level of inflammatory cytokines, such as IL-1β, IL-1Ra, IL-4, IL-5, IL-6, IL-8, IFN-γ and/or TNF-α and/or an increased level of one or more transcription factors such as NF-κB in the full blood, serum and/or plasma of a subject compared to a healthy subject.

In one embodiment the disease of the lung associated with inflammation and/or a pathological immune response is associated with an increased level of inflammatory cytokines, such as IL-1β, IL-1Ra, IL-4, IL-5, IL-6, IL-8, IFN-γ and/or TNF-α and/or an increased level of one or more transcription factors such as NF-κB in the bronchoalveolar lavage (BALB) of a subject compared to a healthy subject.

In one embodiment the level of inflammatory cytokines, such as IL-1β, IL-1Ra, IL-4, IL-5, IL-6, IL-8, IFN-γ and/or TNF-α in the lung tissue of a subject is increased at least 1.2-fold, preferably at least 2-fold, more preferably at least 10-fold compared to a healthy subject. In one embodiment the level of inflammatory cytokines, such as IL-1β, IL-1Ra, IL-4, IL-5, IL-6, IL-8, IFN-γ and/or TNF-α in the lung tissue of a subject is increased 1.2, 1.5, 1.6, 2, 2.5, 4, 5, 10, 12, 14, 15 or 20-fold compared to a healthy subject.

In one embodiment the level of inflammatory cytokines, such as IL-1β, IL-1Ra, IL-4, IL-5, IL-6, IL-8, IFN-γ and/or TNF-α in the full blood, serum and/or plasma of a subject is increased at least 1.2-fold, preferably at least 2-fold, more preferably at least 10-fold compared to a healthy subject. In one embodiment the level of inflammatory cytokines, such as IL-1β, IL-1Ra, IL-4, IL-5, IL-6, IL-8, IFN-γ and/or TNF-α in the full blood, serum and/or plasma of a subject is increased 1.2, 1.5, 1.6, 2, 2.5, 4, 5, 10, 12, 14, 15 or 20-fold compared to a healthy subject.

In one embodiment the level of inflammatory cytokines, such as IL-1β, IL-1Ra, IL-4, IL-5, IL-6, IL-8, IFN-γ and/or TNF-α in bronchoalveolar lavage (BALB) of a subject is increased at least 1.2-fold, preferably at least 2-fold, more preferably at least 10-fold compared to a healthy subject. In one embodiment the level of inflammatory cytokines, such as IL-1β, IL-1Ra, IL-4, IL-5, IL-6, IL-8, IFN-γ and/or TNF-α in the bronchoalveolar lavage (BALB) of a subject is increased 1.2, 1.5, 1.6, 2, 2.5, 4, 5, 10, 12, 14, 15 or 20-fold compared to a healthy subject.

In one embodiment the level of one or more transcription factors such as NF-κB in the lung tissue of a subject is increased at least 1.2-fold, preferably at least 2-fold, more preferably at least 10-fold compared to a healthy subject. In one embodiment the level of one or more transcription factors such as NF-κB -a in the lung tissue of a subject is increased 1.2, 1.5, 1.6, 2, 2.5, 4, 5, 10, 12, 14, 15 or 20-fold compared to a healthy subject.

In one embodiment the level of one or more transcription factors such as NF-κB in the full blood, serum and/or plasma of a subject is increased at least 1.2-fold, preferably at least 2-fold, more preferably at least 10-fold compared to a healthy subject. In one embodiment the level of one or more transcription factors such as NF-κB in the full blood, serum and/or plasma of a subject is increased 1.2, 1.5, 1.6, 2, 2.5, 4, 5, 10, 12, 14, 15 or 20-fold compared to a healthy subject.

In one embodiment the level of one or more transcription factors such as NF-κB in bronchoalveolar lavage (BALB) of a subject is increased at least 1.2-fold, preferably at least 2-fold, more preferably at least 10-fold compared to a healthy subject. In one embodiment the level of one or more transcription factors such as NF-κB in the bronchoalveolar lavage (BALB) of a subject is increased 1.2, 1.5, 1.6, 2, 2.5, 4, 5, 10, 12, 14, 15 or 20-fold compared to a healthy subject.

In one embodiment the disease of the lung associated with inflammation and/or a pathological immune response is associated with an increased level of infiltration of macrophages, monocytes and/or neutrophils in the lung tissue of a subject compared to a healthy subject.

In one embodiment the disease of the lung is asthma.

In one embodiment the disease of the lung is acute respiratory distress syndrome (ARDS).

In one embodiment the disease of the lung is bronchiolitis obliterans (BO).

In one embodiment the disease of the lung is bronchiolitis obliterans syndrome (BOS).

In one embodiment the bronchiolitis obliterans syndrome (BO) is associated with lung transplantation and/or allogenic hematopoietic stem cell transplantation (chronic graft-versus host disease).

In one embodiment the disease of the lung is bronchiolitis obliterans syndrome (BOS) associated with lung transplantation and/or allogenic hematopoietic stem cell transplantation (chronic graft-versus host disease).

In one embodiment the rhAAT protein or fragment thereof comprises a sequence according to SEQ ID NO 4, or is encoded by SEQ ID NO 1, 2 or 3, or a sequence with an at least 80% identity thereto.

In one embodiment the rhAAT protein or fragment thereof has a serum half-life, pulmonary half-life and/or activity not less than AAT purified from human plasma.

In one embodiment the rhAAT protein or fragment thereof has a bronchial half-life and/or activity not less than AAT purified from human plasma.

In one embodiment the rhAAT protein or fragment thereof has a alveolar half-life and/or activity not less than AAT purified from human plasma.

In one embodiment the rhAAT protein or fragment thereof is expressed in a yeast of the family Saccharomycetaceae, preferably the family Pichia.

In one embodiment the rhAAT protein or fragment thereof comprises post-translational modifications.

In one embodiment the post-translation modification is N-glycosylation, O-glycosylation, N-terminal methionine removal, N-acetylation and/or phosphorylation, or any combination thereof, preferably comprising one or more N-linked glycosylations, comprising at least one HexNAc1 glycosylation.

In one embodiment the rhAAT protein or fragment thereof is administered by inhalation, nasal administration, and/or injection.

In one embodiment the rhAAT protein or fragment thereof is administered by inhalation, preferably as a solution suitable for inhalation, soluble powder suitable for inhalation or dry powder suitable for inhalation.

In one embodiment the rhAAT protein or fragment thereof is administered by inhalation of a nebulized solution.

In one embodiment the rhAAT protein or fragment thereof is administered by injection, preferably as a solution suitable for injection. In one embodiment the rhAAT protein or fragment thereof is administered by intravenous injection, preferably as a solution suitable for intravenous injection. In one embodiment the rhAAT protein or fragment thereof is administered by subcutaneous injection, preferably as a solution suitable for subcutaneous injection.

In embodiments of the invention, the recombinant AAT is administered for a plurality of consecutive days. In embodiments, the patient may receive treatment via inhalation multiple times within one day, multiple times within one week, or even on an ongoing basis over multiple weeks.

In embodiments, the patient is subjected additionally to a standard medical treatment for a nonviral disease of the lung associated with inflammation and/or a pathological immune response. For example, standard medical treatments comprise oxygen support, non-invasive ventilation, high flow oxygen, mechanical ventilation and extracorporeal membrane oxygenation or the administration of anzithromycinm, cyclophosphamide, ciclosporin, azathioprine, anti-CD3-antibodies, tacrolimus, mycophenolate or statins for the treatment of Bronchiolitis obliterans (BO), the administration of beta-2-agonists such as salbutamol, bambuterol and indacaterol, glucocorticoids such as beclomethasone, budesonide and fluticasone, theophylline, leukotriene antagonist such as montelukast, cromoglicic acid, nedocromil and lodoxamid for the treatment of asthma.

In embodiments of the invention, the recombinant AAT is administered as preventative treatment, for example for the prevention of Bronchiolitis obliterans syndrome (BOS) associated with lung transplantation and/or allogenic hematopoietic stem cell transplantation by administration of rhAAT directly after lung transplantation and/or allogenic hematopoietic stem cell transplantation. In embodiments of the invention, the treatment results in at least one outcome of reduced hospitalization, reduced oxygen dependence, reduced intensive care or mechanical ventilation need, reduced healthcare utilization or burden, reduced absences from school or work, decreased need for medication such as beta-2-agonist and/or glucocorticoid need, decreased relapse frequency and/or decreased morbidity or risk of morbidity.

In one embodiment, the rhAAT glycoprotein is administered to treat a non-viral disease of the lung associated with inflammation and/or a pathological immune response, wherein the rhAAT protein or fragment thereof is expressed from *Pichia pastoris* and the rhAAT protein or fragment thereof is administered by inhalation of a nebulized solution.

In one embodiment, the rhAAT glycoprotein is administered to treat a non-viral disease of the lung associated with inflammation and/or a pathological immune response, and the rhAAT protein or fragment thereof is expressed from *Pichia pastoris* and isolated using chromatography, such as affinity, size exclusion, ion exchange and/or hydrophobic interaction chromatography, and the rhAAT protein or fragment thereof is administered by inhalation of a nebulized solution.

In one aspect the invention relates to a pharmaceutical composition comprising the rhAAT protein or fragment thereof according to any one of the preceding claims and a pharmaceutically acceptable carrier, for use in the treatment of a non-viral disease of the lung associated with inflammation and/or a pathological immune response.

In one aspect the invention relates to a pharmaceutical composition comprising the rhAAT protein or fragment thereof or a protein preparation according to any one of the preceding claims and a pharmaceutically acceptable carrier, for use in the treatment of a non-viral disease of the lung associated with inflammation and/or a pathological immune response.

In one embodiment, the pharmaceutical composition is a solution, a soluble powder or a dry powder. In embodiments, the composition is suitable for inhalation, nasal administration and/or injection.

In one embodiment the composition is a solution suitable for inhalation, a soluble powder suitable for inhalation or a dry powder suitable for inhalation.

### Embodiments relating to human alpha1-antitrysin (rhAAT) glycoprotein:

In one embodiment, the invention relates to a recombinant human alpha1-antitrysin (rhAAT) glycoprotein or fragment thereof, with one or more N-linked glycosylations, comprising at least one HexNAc1 glycosylation. This rhAAT is suitable for the medical treatments disclosed herein.

As described in more detail below, the present invention provides a novel method of manufacturing rhAAT from *P. pastoris,* leading to the production of a rhAAT glycoprotein with a unique glycosylation pattern that has, to the knowledge of the inventors, not been previously described. Furthermore, this novel glycoprotein and protein preparation comprising the same represent a therapeutic agent with exhibit unexpected advantages in preventing respiratory viral entry into target cells.

Recombinant protein expression in P. pastoris is an established technology, leading to protein glycosylation typically of shorter chain length than found in Saccharomyces cerevisiae.

Expression in yeast, including P. pastoris, typically leads to N-linked oligosaccharides originating from an oligosaccharide (Glc3Man9GlcNAc2) that is assembled in the endoplasmic reticulum (ER). In most yeasts that have been studied, the three glucose residues and one specific a-1,2-linked mannose residue are removed by specific glycosidases in the ER, giving a protein N-linked Man8GlcNAc2 core structure that is further processed in the Golgi complex. This can involve addition of further mannose or other sugar residues in a linear or branched manner, leading to complex elongated polysaccharides bound to the glycosylated protein via a GIcNAc2 disaccharide unit as the protein-proximal unit. In other words, P. pastoris can typically N-glycosylate proteins via a mannose oligosaccharide linked to asparagine through two N-acetylglucosamines.

The present invention provides a rhAAT glycoprotein with unique glycosylations, not previously observed for AAT or in AAT proteins expressed from P. pastoris. As outlined in the Examples below, the inventive rhAAT glycoprotein exhibits at least one HexNAc1 glycosylation. This structure preferably occurs at an amide nitrogen of asparagine residues in AAT, found in the consensus sequence Asn-Xaa-Thr/Ser, providing an N-linked glycosylation of HexNAc1.

In embodiments, the rhAAT glycoprotein or fragment thereof comprises at least two or at least three HexNAc1 glycosylations.

As shown in the Examples below, the three N-linked glycosylation sites of AAT may be completely or partially occupied by HexNAc1, which represents an unexpected finding considering typical glycosylation patterns or recombinant proteins, especially those expressed in yeast.

In embodiments, the rhAAT glycoprotein or fragment thereof comprises at least one Hex9HexNAc2, Hex10HexNAc2, Hex11HexNAc2, Hex12HexNAc2, Hex13HexNAc2, Hex14HexNAc2, Hex15HexNAc2 and/or Hex16HexNAc2 glycosylation.

As shown in the Examples below, any one or more of the N-linked glycosylation sites of AAT may be completely or partially occupied by Hex9HexNAc2, Hex10HexNAc2, Hex11HexNAc2, Hex12HexNAc2, Hex13HexNAc2, Hex14HexNAc2, Hex15HexNAc2 and/or Hex16HexNAc2. These glycosylations appear to be prevalent although typically to a lesser degree than HexNAc1.

As used herein, the term Hex refers to a hexose. In embodiments, Hex may be a mannose, for example Hex9HexNAc2 may relate to Man9HexNAc2. In embodiments, Hex may be a glucose, for example Hex9HexNAc2 may relate to Hex9GlcNAc2. In preferred embodiments, the HexNAc2 nomenclature refers to two N-acetylglucosamines, in other words, to GlcNAc2.

In preferred embodiments, the glycans HexNAc1, Hex9HexNAc2, Hex10HexNAc2, Hex11HexNAc2, Hex12HexNAc2, Hex13HexNAc2, Hex14HexNAc2, Hex15HexNAc2 and/or Hex16HexNAc2 may be expressed as GlcNAc1, Man9GlcNAc2, Man10GlcNAc2, Man11GlcNAc2, Man12GlcNAc2, Man13GlcNAc2, Man14GlcNAc2, Man15GlcNAc2 and/or Man16GlcNAc2, respectively.

Such "Man(n)GlcNAc2" nomenclature (wherein n is the number of mannose) may apply to any given embodiment where the nomenclature "Hex(n)HexNAc2" is disclosed. In embodiments, a nomenclature may be applied for the glycans as Hex(n)HexNAc2 or Man(n)GlcNAc2, wherein n is 0-20, more preferably 1-19, 2-18, 5-18, more preferably 9-16.

Considering the typically complex and long polysaccharides appended to recombinant proteins expressed in yeast, the detected glycans in rhAAT as shown herein represent a surprising glycosylation pattern, with the unexpected presence of HexNAc1 as a predominant glycan and other glycans with relatively short Hexose (Mannose) chains compared to expected results obtained from expression in yeast. Without being bound by theory, such a glycosylation pattern may show advantages over other rhAAT protein preparations, with respect to protein secretion, protein production yield, glycoprotein stability and enzymatic and/or therapeutic efficacy.

It has been shown that N-glycosylation and N-glycan structures can affect the biophysical and pharmacokinetic properties of therapeutic glycoproteins. Furthermore, N-glycans on different sites may play a role in the secretion and ultimately yield of a therapeutic recombinant protein. The report provided below shows high protein yields with assumedly good protein stability based on the N-linked glycosylation modifications, which appear optimal for biotechnological production of a therapeutic rhAAT product.

Taken together, the present invention is based on developing a novel production method for rhAAT in yeast, in particular P. pastoris, leading to good yields of a stable rhAAT glycoprotein that exhibits a unique glycosylation pattern and shows potential improvements in therapeutic efficacy.

### Embodiments relating to the quantities of each glycosylation:

In one embodiment, HexNAc1 glycosylation comprises 50-100% of total N-glycans, preferably 60-90%, more preferably 70-80%.

As shown in the Examples below, the HexNAc1 glycosylation at any one or more of the three N-linked glycosylation sites represents an unexpected modification after rhAAT production in yeast (P. pastoris). Without being bound by theory, this modification, being highly prevalent, also appears to convey improved properties in secretion and/or enzymatic and/or therapeutic efficacy over other AAT preparations, such as Prolastin.

In one embodiment, Hex10HexNAc2, Hex11HexNAc2, Hex12HexNAc2, Hex13HexNAc2 and Hex14HexNAc2 glycosylation comprises 5-50% of total N-glycans, preferably 10-40%, more preferably 15-35%.

In one embodiment, Hex12HexNAc2, Hex13HexNAc2 and Hex14HexNAc2 glycosylation comprises 5-30% of total N-glycans, preferably 10-25%, more preferably 12-20%.

In one embodiment, Hex11HexNAc2, Hex12HexNAc2, Hex13HexNAc2, Hex14HexNAc2 and Hex15HexNAc2 glycosylation comprises 5-50% of total N-glycans, preferably 10-40%, more preferably 15-35%.

In one embodiment, Hex9HexNAc2 glycosylation comprises 0.05-5% of total N-glycans, preferably 0.1-2%, more preferably 0.2-1%.

In one embodiment, Hex10HexNAc2 glycosylation comprises 0.1-10% of total N-glycans, preferably 0.5-5%, more preferably 1-3%.

In one embodiment, Hex11HexNAc2 glycosylation comprises 0.1-10% of total N-glycans, preferably 0.5-5%, more preferably 1-4%.

In one embodiment, Hex12HexNAc2 glycosylation comprises 1-20% of total N-glycans, preferably 2-10%, more preferably 3-8%.

In one embodiment, Hex13HexNAc2 glycosylation comprises 1-20% of total N-glycans, preferably 2-10%, more preferably 3-8%.

In one embodiment, Hex14HexNAc2 glycosylation comprises 1-20% of total N-glycans, preferably 2-10%, more preferably 3-8%.

In one embodiment, Hex15HexNAc2 glycosylation comprises 0.1-10% of total N-glycans, preferably 0.5-7%, more preferably 1-5%.

In one embodiment, Hex16HexNAc2 glycosylation comprises 0.1-10% of total N-glycans, preferably 0.5-7%, more preferably 1-5%.

### Embodiments relating to AAT sequence and position of N-linked glycosylation:

The rhAAT glycoprotein according to any one of the preceding claims, comprising an amino acid sequence according to SEQ ID NO 4 or fragment thereof. SEQ ID NO 4 relates to the primary amino acid sequence of human AAT as commonly known from protein databases and the like.

In embodiments, the rhAAT or fragment thereof comprises an amino acid sequence according to SEQ ID NO 4 and at least one N-linked glycosylation at N46.

In embodiments, the rhAAT or fragment thereof comprises an amino acid sequence according to SEQ ID NO 4 and at least two N-linked glycosylations at N46 and N247.

In embodiments, the rhAAT or fragment thereof comprises an amino acid sequence according to SEQ ID NO 4 and at least three N-linked glycosylations at N46, N83 and N247.

In embodiments, the rhAAT or fragment thereof comprises an amino acid sequence according to SEQ ID NO 4 or fragment thereof and at least one N-linked glycosylation at N46, N83 or N247, at least two N-linked glycosylations at N46 and N83, N46 and N247, or N83 und N247, or three N-linked glycosylations at N46, N83 and N247.

In embodiments, the rhAAT or fragment thereof comprises an amino acid sequence according to SEQ ID NO 4 and at least one N-linked glycosylation comprising HexNAc1 at N46.

In embodiments, the rhAAT or fragment thereof comprises an amino acid sequence according to SEQ ID NO 4 and at least one N-linked glycosylation comprising Hex10HexNAc2, Hex11HexNAc2, Hex12HexNAc2, Hex13HexNAc2, Hex14HexNAc2, Hex15HexNAc2 and/or Hex16HexNAc2 at N83.

In embodiments, the rhAAT or fragment thereof comprises an amino acid sequence according to SEQ ID NO 4 and at least one N-linked glycosylation comprising Hex9HexNAc2, Hex10HexNAc2, Hex11HexNAc2, Hex12HexNAc2, Hex13HexNAc2, Hex14HexNAc2, Hex15HexNAc2 and/or Hex16HexNAc2 at N247.

### Embodiments relating to AAT sequence, position and type of N-linked glycosylation:

In embodiments, the rhAAT or fragment thereof comprises an amino acid sequence according to SEQ ID NO 4 or fragment thereof and at least one N-linked glycosylation comprising HexNAc1 at N46, N83 or N247, at least two N-linked glycosylations comprising HexNAc1 at N46 and N83, at N46 and N247, or N83 und N247, or at least three N-linked glycosylations comprising HexNAc1 at N46, N83 and N247.

In embodiments, the rhAAT or fragment thereof comprises an amino acid sequence according to SEQ ID NO 4 or fragment thereof and comprising at least one N-linked glycosylation comprising Hex10HexNAc2, Hex11HexNAc2, Hex12HexNAc2, Hex13HexNAc2, Hex14HexNAc2, Hex15HexNAc2 or Hex16HexNAc2 at N83, and/or

In embodiments, the rhAAT or fragment thereof comprises an amino acid sequence according to SEQ ID NO 4 or fragment thereof and comprising at least one N-linked glycosylation comprising Hex9HexNAc2, Hex10HexNAc2, Hex11HexNAc2, Hex12HexNAc2, Hex13HexNAc2, Hex14HexNAc2, Hex15HexNAc2 or Hex16HexNAc2 at N247.

### Embodiments relating to AAT sequence, position, type of N-linked glycosylation, with indications regarding occupancy (total) and frequency of each modification at each glycan site:

The invention further relates to a protein preparation comprising recombinant human alpha1-antitrysin (rhAAT) glycoproteins or fragments thereof with heterologous N-linked glycosylations, comprising at least one HexNAc1 glycosylation.

As described in the Examples below, expression, secretion and isolation of rhAAT leads to a protein preparation with various glycan structures. These can be homogenous or heterogeneous. In preferred embodiments, the preparation comprises rhAAT glycoproteins or fragments thereof with heterologous N-linked glycosylations. The quantities (relative or absolute) of each glycosylation may be used to define the protein preparation. Quantitative (or semi-quantitative) peptide mapping has identified rhAAT protein sequences with varying degrees of glycosylation at different sites with the rhAAT protein. These glycosylation patterns may be used to define the rhAAT protein or protein preparation and appear beneficial for secretion, yield, enzymatic activity and/or therapeutic activity.

In embodiments, the protein preparation comprises a rhAAT glycoprotein with an amino acid sequence according to SEQ ID NO 4 or fragment thereof and at least one N-linked glycosylation comprising HexNAc1 at N46, wherein said HexNAc1 at N46 is present in more than 50% of said glycoproteins in the preparation, preferably more than 80%, more preferably more than 90%.

In embodiments, the protein preparation comprises a rhAAT glycoprotein with an amino acid sequence according to SEQ ID NO 4 or fragment thereof and at least one N-linked glycosylation comprising HexNAc1 at N83, wherein said HexNAc1 at N83 is present in 20-60% of said glycoproteins in the preparation, preferably 30-50%, more preferably 35-45%.

In embodiments, the protein preparation comprises a rhAAT glycoprotein with an amino acid sequence according to SEQ ID NO 4 or fragment thereof and at least one N-linked glycosylation comprising HexNAc1 at N247, wherein said HexNAc1 at N247 is present in more than 60% of said glycoproteins in the preparation, preferably 75-95%, more preferably 80-90%.

In embodiments, the protein preparation comprises a rhAAT glycoprotein with an amino acid sequence according to SEQ ID NO 4 or fragment thereof and at least one N-linked glycosylation comprising Hex10HexNAc2, Hex11HexNAc2, Hex12HexNAc2, Hex13HexNAc2, Hex14HexNAc2, Hex15HexNAc2 and/or Hex16HexNAc2 at N83, wherein any one or more of said glycosylations, alone or in combination, is present in 0.5-30% of said glycoproteins in the preparation, preferably 1-20%, more preferably 2-18%.

In embodiments, the protein preparation comprises a rhAAT glycoprotein with an amino acid sequence according to SEQ ID NO 4 or fragment thereof and at least one N-linked glycosylation comprising Hex9HexNAc2, Hex10HexNAc2, Hex11HexNAc2, Hex12HexNAc2, Hex13HexNAc2, Hex14HexNAc2, Hex15HexNAc2 and/or Hex16HexNAc2 at N247, wherein any one or more of said glycosylations, alone or in combination, is present in 0.1-15% of said glycoproteins in the preparation, preferably 0.5-10%, more preferably 1-5%.

In embodiments, the protein preparation comprises a rhAAT glycoprotein with an amino acid sequence according to SEQ ID NO 4 or fragment thereof and at least one N-linked glycosylation at N46 in more than 80% of said glycoproteins in the preparation, preferably more than 90%.

In embodiments, the protein preparation comprises a rhAAT glycoprotein with an amino acid sequence according to SEQ ID NO 4 or fragment thereof and at least one N-linked glycosylation at N83 in more than 80% of said glycoproteins, preferably more than 90%.

In embodiments, the protein preparation comprises a rhAAT glycoprotein with an amino acid sequence according to SEQ ID NO 4 or fragment thereof and at least one N-linked glycosylation at N247 in more than 50% of said glycoproteins, preferably 60-80%, of said glycoproteins in the preparation.

In embodiments, the protein preparation comprises a rhAAT glycoprotein with an amino acid sequence according to SEQ ID NO 4 and at least one N-linked glycosylation comprising HexNAc1 at N46, wherein said HexNAc1 at N46 is present in more than 50% of said glycoproteins in the preparation, preferably more than 80%, more preferably more than 90%.

In embodiments, the protein preparation comprises a rhAAT glycoprotein with an amino acid sequence according to SEQ ID NO 4 and at least one N-linked glycosylation comprising HexNAc1 at N83, wherein said HexNAc1 at N83 is present in 20-60% of said glycoproteins in the preparation, preferably 30-50%, more preferably 35-45%.

In embodiments, the protein preparation comprises a rhAAT glycoprotein with an amino acid sequence according to SEQ ID NO 4 and at least one N-linked glycosylation comprising HexNAc1 at N247, wherein said HexNAc1 at N247 is present in more than 60% of said glycoproteins in the preparation, preferably 75-95%, more preferably 80-90%.

In embodiments, the protein preparation comprises a rhAAT glycoprotein with an amino acid sequence according to SEQ ID NO 4 and at least one N-linked glycosylation comprising Hex10HexNAc2, Hex11HexNAc2, Hex12HexNAc2, Hex13HexNAc2, Hex14HexNAc2, Hex15HexNAc2 and/or Hex16HexNAc2 at N83, wherein one or more of said glycosylations, alone or in combination, is present in 0.5-30% of said glycoproteins in the preparation, preferably 1-20%, more preferably 2-18%.

In embodiments, the protein preparation comprises a rhAAT glycoprotein with an amino acid sequence according to SEQ ID NO 4 and at least one N-linked glycosylation comprising Hex9HexNAc2, Hex10HexNAc2, Hex11HexNAc2, Hex12HexNAc2, Hex13HexNAc2, Hex14HexNAc2, Hex15HexNAc2 and/or Hex16HexNAc2 at N247, wherein one or more of said glycosylations, alone or in combination, is present in 0.1-15% of said glycoproteins in the preparation, preferably 0.5-10%, more preferably 1-5%.

### Further aspects and embodiments:

In embodiments, the rhAAT protein or fragment thereof is expressed in *Pichia pastoris.* As described in detail below, a novel production method has been developed that leads to effective expression, secretion and isolation of the desired protein or protein preparation. The production method may therefore be used to characterize the rhAAT glycoprotein itself or protein preparation. Without being bound by theory, the production method employed using P. pastoris, defined by any one or more features disclosed herein, may in embodiments convey to the glycoprotein its unique glycosylation pattern and/or associated beneficial properties, for example in expression, secretion, yield, isolation, enzymatic activity and/or therapeutic activity.

In embodiments, the one or more N-linked glycosylations are measured, determined and/or analyzed using peptide mapping. Peptide mapping refers to an established technique based on mass spectrometry, used to identify proteins and their modifications based on mass measures. In embodiments, the peptide mapping employs protease degradation of a sample comprising said glycoprotein or fragment thereof or protein preparation and subsequent mass spectrometric analysis of said sample. In embodiments, liquid chromatography coupled to mass spectrometry (LC-MS) may be employed.

### Further aspects and embodiments relating to a method of manufacture:

The invention further relates to a method for producing a recombinant human alpha1-antitrypsin (rhAAT) glycoprotein or fragment thereof in a *Pichia pastoris,* comprising:
i. Providing a genetically modified *Pichia pastoris* mut^{s} strain, comprising an exogenous hAAT-encoding nucleotide sequence under control of an AOX1 promoter (PAOX1),
ii. Cultivating said strain in yeast growth medium, said cultivating comprising at least:
   a) a batch phase comprising cultivation in the presence of glycerol, preferably for 10-24 hours,
   b) a transition phase comprising cultivation in the presence of glycerol and methanol, preferably for 10-24 hours, and
   c) an induction phase comprising cultivation in the presence of methanol, preferably for 40-100 hours, more preferably 60 to 90 hours, and
iii. obtaining secreted rhAAT protein or fragment thereof in a culture supernatant, and optionally isolating said rhAAT or fragment thereof from said supernatant.

In embodiments, the method is for producing the rhAAT glycoprotein or fragment thereof or protein preparation described in the aspects and embodiments above. Surprisingly, by developing the novel method described herein, a unique rhAAT glycoprotein has been obtained with beneficial properties. The method may therefore in embodiments be defined by and/or used to produce the inventive rhAAT described herein. The method of manufacturing the rhAAT as described herein leads to effective expression, secretion and/or isolation of the desired protein or protein preparation. The production method may therefore be used to characterize the rhAAT glycoprotein itself or protein preparation, and vice versa. Without being bound by theory, the production method employed using P. pastoris, defined by any one or more features disclosed herein, may in embodiments convey to the glycoprotein its unique glycosylation pattern and/or associated beneficial properties, for example in expression, secretion, yield, isolation, enzymatic activity and/or therapeutic activity.

The method as described herein is also characterized by high levels of rhAAT expression, rhAAT secretion and ultimately rhAAT yield from supernatants obtained after fermentation using the methods disclosed herein. The present method enables high yields, for example levels of rhAAT in the supernatant at concentrations of over 100 mg/L, or over 200 mg/L. Table 18 below, using a 10L fermentation scale, shows rhAAT in the supernatant at concentrations of over 200 mg/L for example after 60 hours, or 90 hours, of induction with methanol.

In embodiments of the method, said cultivating occurs in vessels comprising 500 mL-25,000 L of yeast growth medium in a bioreactor, and preferably wherein said yeast growth medium is a basal salt medium (BSM).

A skilled person is capable of selecting appropriate vessel size or production conditions depending on the intended use and/or required scale of production. To the knowledge of the inventors, such large-scale production has not previously been established for rhAAT in P. pastoris and represents a beneficial and effective method for producing large quantities of rhAAT intended for clinical use.

The cultivation may therefore occur in any given vessel, bioreactor or other suitable container at a size of 500 mL, 1, 2, 3, 4, 5, 10, 15, 20, 50 or 100 L. Larger bioreactors for industry scale manufacture may also be employed using 500L, 1000L, 2000L, 3000L, 5000L or even up to 10,000L, 20,000L or 25,000 L containers and/or bioreactors. The vessel, container and/or bioreactor size may be any one or more of the values disclosed herein, or defined by a range using any one or more of the values disclosed herein, for the vessel, container and/or bioreactor sizes.

In embodiments of the method, said supernatant comprising secreted rhAAT is (optionally processed by centrifugation and filtering) subsequently processed using affinity chromatography, anion exchange chromatography and/or size exclusion chromatography to obtain isolated an rhAAT glycoprotein or protein preparation.

Any suitable form of chromatography or purification scheme may be employed, as are established in the art and known to a skilled person. The preferred methods of affinity chromatography, ion exchange or size exclusion chromatography represent feasible approaches towards obtaining the desired rhAAT glycoprotein or fragment thereof or the preparation described herein.

In embodiments of the method, the rhAAT protein comprises a sequence according to SEQ ID NO 4 or is encoded by SEQ ID NO 1, 2 or 3, preferably by SEQ ID NO 3.

In embodiments of the method, the strain comprises the exogenous hAAT-encoding nucleotide sequence integrated in the P. pastoris genome.

In embodiments of the method, said cultivating comprises:
a. a batch phase comprising cultivation in the presence of glycerol for 10-24 hours,
b. a transition phase comprising cultivation in the presence of glycerol and methanol for 12-24 hours, and subsequently
c. an induction phase comprising cultivation in the presence of methanol for 40-100 hours, preferably 60 to 90 hours.

In embodiments of the method, in step a) a glycerol fed batch phase is initiated 8-16 hours after inoculation of the BSM medium, comprising a glycerol feed for a period of 6-10 hours after inoculation, wherein the glycerol fed batch phase comprises a glycerol feed to said medium of 5-20 grams of glycerol per hour per liter of medium (g/h/L).

In embodiments of the method, the glycerol feed is ramped from 5-8 g/h/L of glycerol to 12-20 g/h/L over a period of 6-10 hours after inoculation.

In embodiments of the method, in step b) the transition phase is initiated 16-24 hours after inoculation of the BSM medium, comprising a glycerol feed and a methanol feed for a period of 12-24 hours after inoculation, wherein the transition phase comprises a glycerol feed to said medium of 2 to 15 grams of glycerol per hour per liter of medium (g/h/L) and a methanol feed to said medium of 1 to 6 grams of methanol per hour per liter of medium (g/h/L).

In embodiments of the method, the glycerol feed is ramped from 2-8 g/h/L of glycerol to 8-15 g/h/L, and the methanol feed is ramped from 1-3 g/h/L of methanol to 4-6 g/h/L, over a period of 12-24 hours after inoculation.

In embodiments of the method, in step c) the induction phase is initiated 24-48 hours after inoculation of the BSM medium, comprising a methanol feed for a period of 40-100 hours after inoculation, wherein the induction phase comprises a methanol feed to said medium of 2 to 15 grams of methanol per hour per liter of medium (g/h/L).

In embodiments of the method, the methanol feed is ramped from 2-6 g/h/L of methanol to 6-15 g/h/L, over a period of 40-100 hours after inoculation.

In embodiments of the method, the strain comprises a nucleotide sequence encoding an α-mating factor pre-pro secretion leader sequence from S. cerevisiae. Without being bound by theory, such a factor appears in embodiments to enhance secretion of the rhAAT glycoprotein into the supernatant.

In embodiments of the method, said yeast growth medium is a basal salt medium (BSM). This medium is preferred but not essential to production of the inventive rhAAT.

In embodiments of the method, the oxygen saturation of the medium is 20-40%, preferably 25-35%, more preferably about 30%, for one or more of steps a)-c), preferably for two or more steps of a)-c), more preferably for the entire process.

In embodiments of the method, said supernatant comprising secreted rhAAT is subsequently processed by centrifugation and filtering, before being purified and/or isolated using the chromatography approaches described herein. Further options for protein isolation are also described in the detailed description below.

Embodiments and features of the invention described with respect to the method, the glycoprotein, the protein preparation or other aspects, are considered to be disclosed with respect to each and every other aspect of the disclosure, such that features characterizing the methods, may be employed to characterize the glycoproteins or protein preparations, and vice-versa.

The various aspects of the invention are unified by, benefit from, are based on and/or are linked by the common and surprising finding of the glycosylation of the rhAAT described herein, and optionally also by the beneficial characteristics evident in expression, secretion, yield, isolation, enzymatic activity and/or therapeutic activity of these aspects of the invention.

### Overview of sequences disclosed in the description:

| **SEQ ID NO** | **Sequence** | **Description** |
|---|---|---|
| 1 | ATGCCGTCTTCTGTCTCGTGGGGCATCCTCCTGCTGGCAG GCCTGTGCTGCCTGGTCCCTGTCTCCCTGGCTGAGGATCC CCAGGGAGATGCTGCCCAGAAGACAGATACATCCCACCAT GATCAGGATCACCCAACCTTCAACAAGATCACCCCCAACCT GGCTGAGTTCGCCTTCAGCCTATACCGCCAGCTGGCACAC CAGTCCAACAGCACCAATATCTTCTTCTCCCCAGTGAGCAT CGCTACAGCCTTTGCAATGCTCTCCCTGGGGACCAAGGCT GACACTCACGATGAAATCCTGGAGGGCCTGAATTTCAACCT CACGGAGATTCCGGAGGCTCAGATCCATGAAGGCTTCCAG GAACTCCTCCGTACCCTCAACCAGCCAGACAGCCAGCTCC AGCTGACCACCGGCAATGGCCTGTTCCTCAGCGAGGGCCT GAAGCTAGTGGATAAGTTTTTGGAGGATGTTAAAAAGTTGT ACCACTCAGAAGCCTTCACTGTCAACTTCGGGGACACCGAA GAGGCCAAGAAACAGATCAACGATTACGTGGAGAAGGGTA CTCAAGGGAAAATTGTGGATTTGGTCAAGGAGCTTGACAGA GACACAGTTTTTGCTCTGGTGAATTACATCTTCTTTAAAGGC AAATGGGAGAGACCCTTTGAAGTCAAGGACACCGAGGAAG AGGACTTCCACGTGGACCAGGTGACCACCGTGAAGGTGCC TATGATGAAGCGTTTAGGCATGTTTAACATCCAGCACTGTAA GAAGCTGTCCAGCTGGGTGCTGCTGATGAAATACCTGGGC AATGCCACCGCCATCTTCTTCCTGCCTGATGAGGGGAAACT ACAGCACCTGGAAAATGAACTCACCCACGATATCATCACCA AGTTCCTGGAAAATGAAGACAGAAGGTCTGCCAGCTTACAT TTACCCAAACTGTCCATTACTGGAACCTATGATCTGAAGAG CGTCCTGGGTCAACTGGGCATCACTAAGGTCTTCAGCAATG GGGCTGACCTCTCCGGGGTCACAGAGGAGGCACCCCTGAA GCTCTCCAAGGCCGTGCATAAGGCTGTGCTGACCATCGAC | AAT coding sequence |
| | GAGAAAGGGACTGAAGCTGCTGGGGCCATGTTTTTAGAGG CCATACCCATGTCTATCCCCCCCGAGGTCAAGTTCAACAAA CCCTTTGTCTTCTTAATGATTGAACAAAATACCAAGTCTCCC CTCTTCATGGGAAAAGTGGTGAATCCCACCCAAAAATAA | |
| 2 | GAAGATCCACAAGGTGATGCTGCTCAGAAAACAGACACCTC ACACCATGATCAAGATCATCCGACATTTAACAAGATCACAC CTAACCTTGCAGAGTTCGCCTTTTCCTTGTATCGTCAGCTTG CTCATCAAAGCAACTCGACGAACATTTTCTTTTCCCCAGTAA GTATTGCAACTGCATTTGCTATGCTATCGTTGGGTACCAAA GCTGACACTCATGACGAAATATTGGAGGGTCTAAACTTTAA CTTGACAGAAATCCCCGAAGCCCAAATTCATGAGGGATTTC AAGAGTTGTTGAGAACTCTAAACCAACCTGACTCTCAACTG CAGTTAACTACCGGTAATGGACTGTTCTTAAGCGAAGGTTT AAAATTGGTCGATAAGTTCCTTGAGGACGTTAAGAAGTTGT ATCACTCTGAGGCTTTTACGGTCAATTTCGGAGATACTGAG GAAGCCAAGAAACAAATCAATGACTACGTGGAAAAGGGAAC TCAAGGCAAGATCGTTGACTTGGTGAAAGAACTGGATAGAG ATACCGTATTTGCTTTAGTGAACTACATCTTCTTTAAAGGGA AATGGGAAAGACCATTCGAGGTCAAGGATACTGAGGAGGA AGATTTTCACGTCGACCAGGTAACCACTGTTAAGGTTCCGA TGATGAAACGATTGGGAATGTTCAACATTCAGCACTGTAAG AAGTTGTCAAGTTGGGTTCTGCTTATGAAGTACTTAGGGAA TGCAACTGCCATTTTCTTCTTGCCTGATGAAGGTAAACTGCA ACATTTGGAAAATGAACTTACACACGATATTATCACCAAATT TCTAGAGAACGAAGATAGGAGATCAGCCTCTTTGCATTTGC CAAAGCTGTCAATAACAGGTACTTATGACTTGAAATCCGTTC TTGGCCAATTGGGCATAACTAAGGTGTTTTCTAATGGTGCT GATTTGAGTGGTGTTACAGAGGAAGCTCCCTTAAAGCTATC TAAGGCTGTTCACAAAGCAGTTCTTACCATTGACGAGAAAG GTACTGAAGCTGCAGGAGCTATGTTTCTGGAAGCCATTCCT ATGTCCATTCCACCTGAAGTTAAATTCAATAAGCCTTTTGTC TTTCTTATGATTGAGCAGAATACGAAATCTCCATTATTCATG GGAAAGGTAGTGAATCCAACCCAGAAA | Codon optimized AAT coding sequence 1 |
| 3 | GAGGACCCTCAAGGTGACGCCGCTCAAAAGACTGATACCT CGCACCACGACCAAGACCACCCAACTTTTAACAAGATTACT CCTAATCTAGCTGAGTTCGCATTCTCTCTCTACAGACAACTC GCTCATCAGTCTAACTCTACGAACATTTTCTTCTCCCCAGTG TCCATTGCAACTGCTTTCGCCATGCTTTCTTTGGGTACTAAG GCTGACACACACGACGAGATTTTAGAAGGATTGAACTTTAA TCTTACCGAAATTCCTGAGGCCCAAATTCACGAAGGTTTTC AGGAGCTGCTTAGAACTCTTAACCAGCCAGATAGCCAGCTA CAACTTACTACTGGAAATGGACTATTCTTGAGTGAAGGTCT GAAGCTAGTTGACAAATTCCTCGAAGATGTGAAAAAGTTGT ACCATTCTGAGGCTTTCACCGTCAACTTCGGTGACACAGAG GAGGCTAAAAAGCAGATCAACGACTACGTTGAAAAGGGTAC GCAAGGTAAAATCGTTGACCTCGTTAAAGAGCTTGATAGAG ACACCGTATTTGCTCTTGTCAATTACATCTTCTTTAAAGGAA AGTGGGAGAGACCTTTCGAGGTCAAGGACACCGAGGAGGA AGATTTTCATGTAGATCAAGTCACCACTGTTAAGGTTCCAAT GATGAAGCGTCTCGGTATGTTTAACATCCAACACTGTAAAA AGCTATCTTCTTGGGTCCTTCTGATGAAGTACCTGGGTAAC GCTACTGCAATCTTCTTCCTACCAGATGAAGGTAAGCTCCA GCACCTAGAGAACGAACTTACCCACGACATCATTACTAAGT TCCTGGAAAACGAAGACCGTAGATCCGCTTCCCTGCACCTC CCAAAACTGTCTATCACTGGTACCTATGACCTCAAGTCTGTT CTGGGTCAACTAGGTATAACCAAAGTGTTTTCAAACGGCGC TGACCTCTCCGGTGTGACTGAAGAGGCACCTCTCAAGCTGT CCAAGGCCGTTCACAAGGCAGTCCTTACCATTGATGAAAAG GGAACTGAAGCTGCCGGCGCTATGTTCCTAGAGGCCATCC CAATGTCAATTCCTCCCGAGGTTAAGTTCAACAAGCCATTC | Codon optimized AAT coding sequence 2 (preferred) |
| | GTCTTCCTAATGATTGAACAAAACACAAAGTCTCCTCTGTTC ATGGGAAAGGTTGTTAACCCAACCCAAAAGTAATAG | |
| 4 | EDPQGDAAQKTDTSHHDQDHPTFNKITPNLAEFAFSLYRQLA HQSNSTNIFFSPVSIATAFAMLSLGTKADTHDEILEGLNFNLTEI PEAQIHEGFQELLRTLNQPDSQLQLTTGNGLFLSEGLKLVDKF LEDVKKLYHSEAFTVNFGDTEEAKKQINDYVEKGTQGKIVDLV KELDRDTVFALVNYIFFKGKWERPFEVKDTEEEDFHVDQVTTV KVPMMKRLGMFNIQHCKKLSSWVLLMKYLGNATAIFFLPDEG KLQHLENELTHDIITKFLENEDRRSASLHLPKLSITGTYDLKSVL GQLGITKVFSNGADLSGVTEEAPLKLSKAVHKAVLTIDEKGTEA AGAMFLEAIPMSIPPEVKFNKPFVFLMIEQNTKSPLFMGKVVNP TQK | Human AAT sequence |
| 5 | AGATCTAACATCCAAAGACGAAAGGTTGAATGAAACCTTTTT GCCATCCGACATCCACAGGTCCATTCTCACACATAAGTGCC AAACGCAACAGGAGGGGATACACTAGCAGCAGACCGTTGC AAACGCAGGACCTCCACTCCTCTTCTCCTCAACACCCACTT TTGCCATCGAAAAACCAGCCCAGTTATTGGGCTTGATTGGA GCTCGCTCATTCCAATTCCTTCTATTAGGCTACTAACACCAT GACTTTATTAGCCTGTCTATCCTGGCCCCCCTGGCGAGGTT CATGTTTGTTTATTTCCGAATGCAACAAGCTCCGCATTACAC CCGAACATCACTCCAGATGAGGGCTTTCTGAGTGTGGGGT CAAATAGTTTCATGTTCCCCAAATGGCCCAAAACTGACAGTT TAAACGCTGTCTTGGAACCTAATATGACAAAAGCGTGATCT CATCCAAGATGAACTAAGTTTGGTTCGTTGAAATGCTAACG GCCAGTTGGTCAAAAAGAAACTTCCAAAAGTCGGCATACCG TTTGTCTTGTTTGGTATTGATTGACGAATGCTCAAAAATAAT CTCATTAATGCTTAGCGCAGTCTCTCTATCGCTTCTGAACCC CGGTGCACCTGTGCCGAAACGCAAATGGGGAAACACCCGC TTTTTGGATGATTATGCATTGTCTCCACATTGTATGCTTCCA AGATTCTGGTGGGAATACTGCTGATAGCCTAACGTTCATGA TCAAAATTTAACTGTTCTAACCCCTACTTGACAGCAATATAT AAACAGAAGGAAGCTGCCCTGTCTTAAACCTTTTTTTTTATC ATCATTATTAGCTTACTTTCATAATTGCGACTGGTTCCAATT GACAAGCTTTTGATTTTAACGACTTTTAACGACAACTTGAGA AGATCAAAAAACAACTAATTATTCGAAA | AOX1 promoter from *pichia pastoris* |
| 6 | QLAHQSNSTNIFFSPVSIATAFAMLSLGTK | Trypsin digest of rhAAT comprising N46 N-Glycosylation site |
| 7 | QLAHQSNSTNIFFSPVSIATAFAMLSLGTK | Trypsin/GluC double digest of rhAAT comprising N46 N-Glycosylation site |
| 8 | ADTHDEILEGLNFNLTEIPEAQIHEGFQELLR | Trypsin digest of rhAAT comprising N83 N-Glycosylation site |
| 9 | GLNFNLTEIPE | Trypsin/GluC double digest of rhAAT comprising N83 N-Glycosylation site |
| 10 | YLGNATAIFFLPDEGK | Trypsin digest of rhAAT comprising N247 N-Glycosylation site |
| 11 | YLGNATAIFFLPDEGK | Trypsin/GluC double digest of rhAAT comprising N247 N-Glycosylation site |
| 12 | YLGNATAIFFLPDEGKLQHLE | Trypsin/GluC double digest of rhAAT comprising N247 N-Glycosylation site |
| 13 | YLGNATAIFFLPDEGKLQHLENELTHDIITK | Trypsin digest of rhAAT comprising N247 N-Glycosylation site |

| | | |
|---|---|---|
| Trypsin: arginine (R) or lysine (K); GluC: aspartic (D) or glutamic (E) | | |

### DETAILED DESCRIPTION OF THE INVENTION

All cited documents of the patent and non-patent literature are hereby incorporated by reference in their entirety.

### Alpha-1 antitrypsin (AAT):

Alpha-1 antitrypsin (also known as A1AT, AAT, PI, SERPINA1) is a ~52kDa glycoprotein that is one of the most abundant endogenous serine protease inhibitors (SERPIN superfamily). AAT is considered to be an acute phase protein, whereby AAT concentration can increase many fold upon acute inflammation.

The AAT encoding region is preferably any nucleic acid that encodes a naturally occurring or synthetic AAT protein sequence that exhibits AAT function, with reduced, the same, similar or increased activity compared to human AAT, or is functionally analogous to human AAT. The amino acid sequence of AAT is available under accession number 1313184B from the NCBI database. Corresponding nucleic acid sequences that encode AAT may be provided by one skilled in the art of molecular biology or genetics. The use of sequence variants of AAT that exhibit functional analogy or similarity to the unmodified human form of AAT is encompassed by the present invention.

One AAT coding sequence (CDS) is published at http://www.ncbi.nlm.nih.gov/nuccore/NM_000295.4 and is one preferred embodiment. This sequence comprises bases 262 to 1518 of the full sequence. SEQ ID NO 1 represent one exemplary AAT encoding sequence.

In some embodiments of the invention the CDS is codon optimized to increase protein production. The coding sequence after codon optimization preferably reads as in SEQ ID NO 2.

The nucleotide sequence according to SEQ ID NO 1 and/or 2 and/or 3 encodes a human AAT protein of the amino acid sequence according to SEQ ID NO 4.

In embodiments, a human AAT protein is employed according to SEQ ID NO 4, corresponding to a human AAT sequence as known in the art.

As used herein, a recombinant human AAT (rhAAT) glycoprotein is any protein comprising a segment or fragment of human rhAAT sequence that shows essentially equivalent or similar activity to the rhAAT assessed in the examples below. Fragments of SEQ ID NO 4 are therefore also considered human AAT, as are sequence variations that exhibit an equivalent or essentially similar function as rhAAT described herein.

The invention therefore encompasses the use of a genetically modified yeast, preferably *Pichia pastoris,* expressing recombinant AAT as described herein, wherein the yeast comprises a nucleic acid molecule selected from the group consisting of:
a) a nucleic acid molecule comprising a nucleotide sequence that encodes AAT protein, such as according to SEQ ID NO 4, preferably by a nucleotide sequence according to SEQ ID NO 1 or 2 or 3, more preferably SEQ ID NO 3.
b) a nucleic acid molecule which is complementary to a nucleotide sequence in accordance with a);
c) a nucleic acid molecule comprising a nucleotide sequence having sufficient sequence identity to be functionally analogous/equivalent to a nucleotide sequence according to a) or b), comprising preferably a sequence identity to a nucleotide sequence according to a) or b) of at least 70%, 75%, 80%, 85%, preferably 90%, more preferably 95%;
d) a nucleic acid molecule which, as a consequence of the genetic code, is degenerated into a nucleotide sequence according to a) through c); and/or
e) a nucleic acid molecule according to a nucleotide sequence of a) through d) which is modified by deletions, additions, substitutions, translocations, inversions and/or insertions and is functionally analogous/equivalent to a nucleotide sequence according to a) through d).

The invention therefore encompasses an AAT protein according to SEQ ID NO 4 or variants thereof, or the use of a genetically modified yeast, preferably *Pichia pastoris* as described herein, comprising a nucleotide sequence encoding an amino acid sequence, according to SEQ ID NO 4 5.

The invention encompasses further sequence variants of SEQ ID NO 3, in particular those of at least 70% sequence identity to SEQ ID NO 4, preferably at least 75%, 80%, 85%, 90%, or at least 95% sequence identity to SEQ ID NO 4. Such sequence variants are preferably functionally analogous or equivalent to the human AAT disclosed herein.

Variations in the length of the protein are also encompassed by the invention, in cases where the functional equivalence or similarity of human AAT of SEQ ID NO 4 is maintained. Truncations or extensions in the length of the protein of, for example, up 50 amino acids, 40, 30, 20, or 10 amino acids, may maintain AAT activity and are therefore encompassed in the present invention.

The term rhAAT glycoprotein fragment refers also to truncations (or fragments) of e.g., SEQ ID NO 4 that maintain in essence the observed activity for the inventive rhAAT glycoprotein. Sufficient details regarding enzyme and/or therapeutic activity are disclosed in the Examples below, with which as killed pesron may determine that a rhAAT fragment exhibits essentially the same, a similar or analogous activity.

A rhAAT glycoprotein fragment therefore preferably comprises all three rhAAT glycosylation sites described herein, and maintain the activity as described herein. Any fragment in which up to 20 amino acids are missing from the N- and/or C-terminal sequence of SEQ ID NO 4 may be considered a functional fragment.

In embodiments, the AAT may comprise a 0 to 10 amino acid addition or deletion at the N and/or C terminus of a relevant sequence. This means that the polypeptide may have a) 0, 1 , 2, 3, 4, 5, 6, 7, 8, 9 or 10 additional amino acids at its N terminus and 0, 1 , 2, 3, 4, 5, 6, 7, 8, 9 or 10 amino acids deleted at its C terminus or b) 0, 1 , 2, 3, 4, 5, 6, 7, 8, 9 or 10 additional amino acids at its C terminus and 0, 1 , 2, 3, 4, 5, 6, 7, 8, 9 or 10 nucleotides deleted at its N terminus, c) 0, 1 , 2, 3, 4, 5, 6, 7, 8, 9 or 10 additional amino acids at its N terminus and 0, 1 , 2, 3, 4, 5, 6, 7, 8, 9 or 10 additional amino acids at its N terminus or d) 0, 1 , 2, 3, 4, 5, 6, 7, 8, 9 or 10 amino acids deleted at its N terminus and 0, 1 , 2, 3, 4, 5, 6, 7, 8, 9 or 10 amino acids deleted at its C terminus.

Functionally analogous sequences refer to the ability to provide a functional AAT gene product and to enable the same or similar functional effect as human AAT. AAT function may be determined by its ability to inhibit a variety of proteases in vitro, such as trypsin, or by its ability to inhibit neutrophil elastase (as described below). Appropriate assays for determining protease activity or for determining neutrophil elastase activity are known to a skilled person.

Protein modifications to the AAT protein, which may occur through substitutions in amino acid sequence, and nucleic acid sequences encoding such molecules, are also included within the scope of the invention. Substitutions as defined herein are modifications made to the amino acid sequence of the protein, whereby one or more amino acids are replaced with the same number of (different) amino acids, producing a protein which contains a different amino acid sequence than the primary protein. In some embodiments this amendment will not significantly alter the function of the protein. Like additions, substitutions may be natural or artificial. It is well known in the art that amino acid substitutions may be made without significantly altering the protein's function. This is particularly true when the modification relates to a "conservative" amino acid substitution, which is the substitution of one amino acid for another of similar properties. Such "conserved" amino acids can be natural or synthetic amino acids which because of size, charge, polarity and conformation can be substituted without significantly affecting the structure and function of the protein. Frequently, many amino acids may be substituted by conservative amino acids without deleteriously affecting the protein's function. In general, the non-polar amino acids Gly, Ala, Val, Ile and Leu; the non-polar aromatic amino acids Phe, Trp and Tyr; the neutral polar amino acids Ser, Thr, Cys, Gln, Asn and Met; the positively charged amino acids Lys, Arg and His; the negatively charged amino acids Asp and Glu, represent groups of conservative amino acids. This list is not exhaustive. For example, it is well known that Ala, Gly, Ser and sometimes Cys can substitute for each other even though they belong to different groups.

The terminology "conservative amino acid substitutions" is well known in the art, which relates to substitution of a particular amino acid by one having a similar characteristic (e.g., similar charge or hydrophobicity, similar bulkiness). Examples include aspartic acid for glutamic acid, or isoleucine for leucine. A conservative substitution variant will 1) have only conservative amino acid substitutions relative to the parent sequence, 2) will have at least 90% sequence identity with respect to the parent sequence, preferably at least 95% identity, 96% identity, 97% identity, 98% identity or 99% or greater identity; and 3) will retain neuroprotective or neurorestorative activity. In this regard, any conservative substitution variant of the above-described polypeptide sequences is contemplated in accordance with this invention. Such variants are considered to be "an AAT protein".

As used herein, a "percent (%) sequence identity" with respect to a reference polypeptide or nucleic acid sequence is defined as the percentage of amino acid residues or nucleotides in a candidate sequence that are identical with the amino acid residues or nucleotides in the reference polypeptide or nucleic acid sequence, after aligning the sequences and introducing gaps, if necessary, to achieve the maximum percent sequence identity, and not considering any conservative substitutions as part of the sequence identity. Alignment for purposes of determining percent amino acid or nucleic acid sequence identity can be achieved in various ways that are within the skill in the art, for instance, using publicly available computer software programs. Software such as BLAST or Clustal enable such sequence alignments and calculation of percent identity. As used herein, the percent homology between two sequences is equivalent to the percent identity between the sequences. Determination of percent identity or homology between sequences can be done, for example, by using the GAP program (Genetics Computer Group, software; now available via Accelrys on http://www.accelrys.com), and alignments can be done using, for example, the ClustalW algorithm (VNTI software, InforMax Inc.). A sequence database can be searched using the nucleic acid sequence of interest. Algorithms for database searching are typically based on the BLAST software (Altschul et al., 1990). In some embodiments, the percent homology oridentity can be determined along the full-length of the nucleic acid.

Furthermore, in addition to the polypeptides described herein, peptidomimetics are also contemplated. Peptide analogs are commonly used in the pharmaceutical industry as nonpeptide drugs with properties analogous to those of the template peptide. These types of nonpeptide compound are termed "peptide mimetics" or "peptidomimetics" (Fauchere (1986) Adv. Drug Res. 15: 29; Veber and Freidinger (1985) TINS p. 392; and Evans et al. (1987) J. Med. Chem. 30: 1229) and are usually developed with the aid of computerized molecular modelling. Peptide mimetics that are structurally similar to therapeutically useful peptides may be used to produce an equivalent therapeutic or prophylactic effect. It may be preferred in some embodiments to use peptide mimetics in order to prolong the stability of the polypeptides, when administered to a subject. To this end peptide mimetics for the polypeptides may be preferred that are not cleaved by human proteasomes.

A nucleic acid molecule encoding an AAT of the invention can be codon-optimized according to methods standard in the art for expression in the cell containing the target DNA of interest. For example, if the intended target nucleic acid is in a yeast cell, a yeast codon-optimized polynucleotide encoding AAT is contemplated for use in the constructs described herein.

In other embodiments, the Alpha-1 antitrypsin (AAT) protein may be replaced by other serine protease inhibitors. Any feature with reference to AAT as disclosed herein may therefore also relate to other serine protease inhibitors. In one embodiment, the serine protease inhibitor is a trypsin-like serine protease inhibitor.

A trypsin-like serine protease inhibitor inhibits a serine protease with trypsin-like activity. Serine serves as the nucleophilic amino acid at the enzyme's active site of the serine protease enzyme. Numerous trypsin-like serine proteases have been under active pursuit as therapeutic targets for inhibition. Viral entry via endocytosis is dependent on target cell proteases, such as serine proteases. Viral infections are target cell serine protease dependent if the virus requires a target cell serine protease for viral entry and growth.

The target cell serine protease activates the virus Spike protein required for fusion of the viral and target cell membranes and the release of the viral genome into the cytosol of the target cell. Targeting the host cell serine protease can prevent viral growth of an endocytosis dependent virus, whereby inhibiting the target cell serine protease is an effective method for treating a subject infected with the endocytosis dependent virus. The serine protease inhibitor, preferably a trypsin-like serine protease inhibitor, inhibits viral infection and virus growth. As non-limiting examples, trypsin-like serine protease inhibitors include camostat, aprotinin, benzamidine, gabexate, leupeptin, nafamostat, pepstatin A, ribavirin, sepimostat, ulinastatin, and patamostat.

### Molecules:

As used herein, a "nucleic acid" or a "nucleic acid molecule" is meant to refer to a molecule composed of chains of monomeric nucleotides, such as, for example, DNA molecules (e.g., cDNA or genomic DNA). A nucleic acid may encode, for example, a promoter, an AAT gene or portion thereof, or regulatory elements. A nucleic acid molecule can be single-stranded or double-stranded.

An "AAT nucleic acid" refers to a nucleic acid that comprises the AAT gene or a portion thereof, or a functional variant of the AAT gene or a portion thereof. A functional variant of a gene includes a variant of the gene with minor variations such as, for example, silent mutations, single nucleotide polymorphisms, missense mutations, and other mutations or deletions that do not significantly alter gene function.

The term "nucleic acid construct" as used herein refers to a nucleic acid molecule, either single-or double-stranded, which is isolated from a naturally occurring gene or which is modified to contain segments of nucleic acids in a manner that would not otherwise exist in nature or which is synthetic. The term nucleic acid construct is synonymous with the term "expression cassette" when the nucleic acid construct contains the control sequences required for expression of a coding sequence of the present disclosure.

A DNA sequence that "encodes" a particular AAT protein (including fragments and portions thereof) is a nucleic acid sequence that is transcribed into the particular RNA and/or protein. A DNA polynucleotide may encode an RNA (mRNA) that is translated into protein, or a DNA polynucleotide may encode an RNA that is not translated into protein (e.g., tRNA, rRNA, or a DNA-targeting RNA; also called "non-coding" RNA or "ncRNA").

As used herein, the terms "gene" or "coding sequence," is meant to refer broadly to a DNA region (the transcribed region) which encodes a protein. A coding sequence is transcribed (DNA) and translated (RNA) into a polypeptide when placed under the control of an appropriate regulatory region, such as a promoter. A gene may comprise several operably linked fragments, such as a promoter, a 5'-leader sequence, a coding sequence and a 3'-non-translated sequence, comprising a polyadenylation site. The phrase "expression of a gene" refers to the process wherein a gene is transcribed into an RNA and/or translated into an active protein.

As used herein, "protein" or "polypeptide" shall mean both peptides and proteins. In this invention, polypeptides may be naturally occurring or recombinant (i.e., produced via recombinant DNA technology), and may contain mutations (e.g., point, insertion and deletion mutations) as well as other covalent modifications (e.g., glycosylation and labelling (via biotin, streptavidin, fluorescein, and radioisotopes)) or other molecular bonds to additional components. For example, PEGylated proteins are encompassed by the scope of the present invention. PEGylation has been widely used as a post-production modification methodology for improving biomedical efficacy and physicochemical properties of therapeutic proteins. Applicability and safety of this technology have been proven by use of various PEGylated pharmaceuticals for many years (refer Jevsevar et al, Biotechnol J. 2010 Jan;5(1):1 13-28). In some embodiments the polypeptides described herein are modified to exhibit longer in vivo half-lives and resist degradation when compared to unmodified polypeptides. Such modifications are known to a skilled person, such as cyclized polypeptides, polypeptides fused to Vitamin B12, stapled peptides, protein lipidization and the substitution of natural L-amino acids with D-amino acids (refer Bruno et al, Ther Deliv. 2013 Nov; 4(11):1443-1467).

### Producing recombinant AATin yeast:

The invention relates to a method of producing recombinant AAT or fragment(s) thereof from genetically modified yeast, preferably comprising the steps of culturing a genetically modified yeast comprising an exogenous nucleic acid molecule with an AAT-encoding region operably linked to a promoter or promoter/enhancer combination, expressing recombinant AAT in the cultured yeast, and isolating recombinant AAT from the culture.

The yeast expression platform that can be used in the context of the invention comprises any strain of yeast used to produce large amounts of proteins, here AAT, for research or industrial uses. While yeast is often more resource-intensive to maintain than for example bacteria, certain products can only be produced by eukaryotic cells like yeast, necessitating use of a yeast expression platform. Yeasts differ in productivity and with respect to their capabilities to secrete, process and modify proteins. As such, different types of yeast (i.e. different expression platforms) are better suited for different research and industrial applications. Importantly, protein expression and production in yeast is advantageous since yeast is able to grow rapidly in large containers, produce proteins in an efficient way, is safe and can produce and modify the protein products to be as ready for human consumption in the context of cosmetic compositions.

The manufacture of recombinant therapeutics is a fast-developing section of therapeutic pharmaceuticals. Yeasts are established eukaryotic host for heterologous protein production and offer distinctive benefits in synthesizing pharmaceutical recombinants. Yeasts are proficient of vigorous growth on inexpensive media, easy for gene manipulations, and are capable of adding post translational changes of eukaryotes. Numerous yeasts comprising *Saccharomyces cerevisiae, Pichia pastoris, Hansenula polymorpha, Yarrowia lipolytica, Arxula adeninivorans, Kluyveromyces lactis,* and *Schizosaccharomyces pombe* are exemplary yeast hosts for recombinant protein production, and may be employed accordingly.

Yeasts are common hosts for the production of proteins from recombinant DNA. They offer relatively easy genetic manipulation and rapid growth to high cell densities on inexpensive media. As eukaryotes, they are able to perform protein modifications like glycosylation which are common in eukaryotic cells, but relatively rare in bacteria. Due to this, yeast can produce complex proteins that are identical or very similar to native products from mammals and in particular humans. The most commonly used yeast expression platform is based on the baker's yeast *Saccharomyces cerevisiae.* However, further yeast expression platforms such as *Saccharomycetaceae Pichia,* in particular, *Pichia pastoris* have been studied and are widely used for various applications based on their different characteristics and capabilities. For instance, some of them grow on a wide range of carbon sources and are not restricted to glucose, as it is the case with baker's yeast. Several of them are also applied to genetic engineering and to the production of foreign proteins and can be used in the context of the present invention.

In embodiments, the yeast related to the present invention is of the family *Saccharomycetaceae,* preferably *Saccharomycetaceae Pichia,* more preferably *Pichia pastoris.*

*Pichia pastoris* is an excellent expression host for the production of heterologous proteins including industrial enzymes and biopharmaceuticals. So far, this methylotrophic expression system has been successfully utilized for the generation of numerous recombinant proteins including human erythropoietin, phospholipase C, phytase, human superoxide dismutase, trypsin, human serum albumin, collagen and human monoclonal antibody 3H6 Fab fragment. Compared to any other yeast species, *P. pastoris* is efficient in the secretory production of recombinant proteins.

The industrial interest to this host is also attributed to powerful methanol-regulated alcohol oxidase promoter (AOX1), highly efficient secretion mechanism, posttranslational modification capabilities, and high cell density growing on defined medium. Various recombinant proteins have been expressed using *P. pastoris.* There are several successful cases for production of therapeutic protein in *P. pastoris.* In a previous study (2016), it has been demonstrated the production of a nanobody (VHH) against Clostridium botulinum neurotoxin type E (BoNT/E) in P. *pastoris.* A product yield of 16 mg/l was achieved that was higher than the levels produced by E. coli. In addition, Xia et al. showed production of 30 mg/l of recombinant angiogenin in this yeast. Furthermore, the productivity of 111 mg/l for human adiponectin, 8.1 g/l for recombinant xylanase and 260 mg/l of anti-HIV antibody have been reported in *P. pastoris.*

The average concentration of AAT in plasma is 1.3mg/ml, with a half-life of 3 to 5 days. Protein size, glycosylation pattern, metastable inhibitory nature of AAT and production cost represent the challenges in the production of recombinant AAT. The methylotrophic yeast *P. pastoris* as an attractive host for production of human AAT. It owns the ability for introducing many of posttranslational modifications such as glycosylation and proteolytic processing which is obtained through moving in the secretory pathway. For glycoproteins such as AAT, these modifications are very important for appropriate function and/or structure.

In embodiments, the AAT is expressed in *P. pastoris* as a fusion protein to a histidine tag (Histag) which facilitates a purification step. Signal sequence from Saccharomyces cerevisiae α-factor secretion signal was included to direct secretion of the protein to extracellular medium. This powerful expression system made use of the highly inducible alcohol oxidase 1 (AOX1) promoter to express large quantities of glycosylated protein. The *P. pastoris* produced AAT activity and its features were evaluated using elastase inhibitory assay, respectively.

In embodiments, the genetically modified yeast is of the family *Saccharomycetaceae,* preferably *Saccharomycetaceae Saccharomyces,* more preferably *Saccharomyces cerevisiae.* In embodiments, S. *cerevisiae* may be a preferred host over bacteria. The Saccharomyces cerevisiae expression system is one of the most commonly used eukaryotic organisms that has been utilized as a model to study various biological phenomena and for the recombinant production of therapeutic proteins. Potential issues in protein glycosylation using Saccharomyces cerevisiae have been addressed, making S. *cerevisiae* a viable yeast for therapeutic protein production. For example, it has been shown that disruption of Mnn2p and Mnn11p genes that are related to glycosylation modification pathways, improved the production of recombinant cellulases. Furthermore, removal of the α-1, 6-mannosyltransferase Och1p enhanced the production of active form of human tissue-type plasminogen activator. These studies indicated that N-glycosylation modification also leads to increased protein secretion. Recombinant proteins can be expressed intracellularly or directed to the secretory apparatus using a secretory signal peptide. The frequently used signal sequence that is functional in all yeast expression systems, is prepro-sequence of mating factor α1 (MFα1).

*Saccharomyces cerevisiae* has also some important advantages from the safety point of view and this property encourages the use of this system in various industrial processes. It is generally regarded as safe (GRAS) because S. *cerevisiae* is nonpathogenic and historically have used in various nutritional industries and production of biopharmaceuticals. On the other hand, current knowledge on genetics, physiology, and fermentation of yeasts facilitate the use of this organism in the production of useful products. Hepatitis B surface antigen, hirudin, insulin, glucagon, urate oxidase, macrophage colony-stimulating factor and platelet-derived growth factor are examples of products on the market from *S*. *cerevisiae.* Alternative expression systems including methylotrophic yeasts *Pichia pastoris* and *Hansenula polymorpha* and non-methylotrophic yeast *Yarrowia lipolytica, Kluyveromyces lactis* and *Arxula adeninivorans* are also viable alternatives.

As used herein, the term "promoter" shall mean a sequence of DNA to which proteins bind to initiate transcription of an RNA transcript from the DNA downstream of the promoter.

As used herein, the term "AOX1 promoter", alcohol oxidase I promoter, shall mean a methanol inducible promoter for protein expression in pichia pastoris. An AOX1 promoter in the context of the present invention comprises a WT AOX1 promoter (native AOX1 promoter) or any AOX1 promoter with alternative and analogous functional sequences derived from a native AOX1 promoter. AOX1 promoter libraries have been developed with deletion or insertion methods according to the prior art. In one embodiment, the AOX1 promoter is according to SEQ ID NO:5.

In embodiments, the promoter used for rhAAT can be an inducible AOX1 promoter, constitutive GAP (glyceraldehyde 3-phosphate dehydrogenase) promoter, or ADH3 (alcohol dhydrogenase) promoter. Further suitable promoters from Pichia pastoris are e.g.,:
Inducible promoters: DAS (dihydroxyacetone phosphate), FLD1 (formaldehyde dehydrogenase), PEX8 (peroxisomal matrix protein), ICL1 (isocitrate lyase), LRA3 (L) (L-rhamnonate dehydratase), LRA4 (L-KDR aldolase), THI11 (thiamine biosynthesis protein), GTH1 (high affinity glucose transport) and PPLCC1 (Laccase);
Constitutive promoters: YPT1 (GTPase involved in secretion), TEF1 (translation elongation factor-1 alpha), GCW14 (Glycosylphosphatidylinositol) and PGK1 (phosphoglycerate kinase).

In embodiments, the rhAAT as described herein produced using a Muts strain of Pichia pastoris. recombinant proteins are typically expressed in the wild-type Mut+ host strain from the methanolinducible promoter PAOX1. However, additional host strains, such as Muts (AOX1-) and Mut-(AOX1- AOX2-), have been produced that consume less methanol and express recombinant protein at greater levels than Mut+. MutS Pichia pastoris strains are known and available to a skilled person.

### Post-translational modifications:

In one embodiment, the method as used herein comprises post-translational modification of the recombinant AAT protein.

In embodiments, the post-translational modification is N-glycosylation, O-glycosylation, N-terminal methionine removal, N-acetylation and/or phosphorylation or any combination thereof.

In embodiments, the recombinant AAT protein has one or more human-like glycoform patterns.

In embodiments, the method as used herein comprises modifying the recombinant AAT protein in vitro after isolation from the yeast.

In some embodiments, the modification is covalent attachment of recombinant AAT protein to a biocompatible polymer.

The term "Posttranslational modification" refers to any alteration of the translated polypeptide chain of AAT that occurs after or during translation. This includes modifications of amino acid side chains of the polypeptide or modifications of the terminal amino or carboxyl group. Posttranslational modification refers to attaching a biochemical group such as acetate, phosphate, carbohydrate moieties and lipids to amino acid side chains that alter the biochemical and physical properties of a protein after translation. Many proteins undergo post translational modifications shortly after their translation and some after protein folding and some after localization. Most common protein post translational modifications are Phosphorylation, Glycosylation, Methylation, Ubiquitination, S-Nitrosylation, N-Acetylation.

Such modifications can be covalent modifications carried out enzymatically by the expression system following protein biosynthesis. Posttranslational modifications further comprise enzymatic cleavage of peptide bonds and processing of the translated protein, covalent additions of particular chemical groups, polymer, lipids, carbohydrates, or even entire proteins to amino acid side chains. These chemical modifications of a polypeptide chain after its biosynthesis extends the range of amino acid structures and properties, and consequently, diversifies structures and functions of proteins. In a preferred embodiment, the recombinant AAT protein undergoes posttranslational modification, such as phosphorylation, glycosylation, ubiquitination, nitrosylation, methylation, acetylation, lipidation and proteolysis.

In one aspect of the invention, secreted or released AAT protein undergoes glycosylation encompassing a diverse selection of sugar-moiety additions to the proteins that ranges from simple monosaccharide modification of nuclear transcription factors to highly complex branched polysaccharide changes of the cell surface receptors in the yeast expression system, preferably O-glycosylation and/or N-glycosylation. N-glycosylation is binding of carbohydrates to asparagine of the polypeptide. O-glycosylation is binding of carbohydrates to serine/threonine.

In embodiments, a modification of recombinant AAT protein is the covalent attachment of recombinant AAT to a biocompatible polymer such as polyethylene glycol (PEG). The term "PEGylation" as used herein refers to a process of both covalent and non-covalent attachment of polyethylene glycol polymer chains to molecules and macrostructures, such as a drug, or a bioactive protein or vesicle. PEGylation is routinely performed through incubation of a reactive derivative of PEG with the target molecule. The covalent attachment of PEG to a protein can "mask" the agent from the host's immune system (reducing immunogenicity and antigenicity), and increase its hydrodynamic size (size in solution), which prolongs its circulatory time by reducing renal clearance. In one aspect, PEGylation can occur at N-terminus of the recombinant AAT protein.

As used herein, the term "biocompatible polymer" refers to polymer with the suitability to body and body fluids exposure. Biocompatible polymers are both synthetic and natural and aid in the close vicinity of a living system or work in intimacy with living cells. These are used to gauge, treat, boost, or substitute any tissue, organ or function of the body. A biocompatible polymer improves body functions without altering its normal functioning and triggering allergies or other side effects. It encompasses advances in tissue culture, tissue scaffolds, implantation, artificial grafts, wound fabrication, controlled drug delivery, bone filler material, etc. Biocompatible polymer are such as, Polystyrene (PS), Polypropylene (PP), Polyvinyl chloride (PVC), Polyethylene (PE), Polyurethane (PU), Polycarbonate (PC), Polyethylene terephthalate (PET), Polyetheretherketone (PEEK).

As used herein, "polyethylene glycol" is a polyether compound with many applications, from industrial manufacturing to medicine. PEG is also known as polyethylene oxide or polyoxyethylene, depending on its molecular weight.

In certain aspects, a recombinant AAT protein is conjugated to a water-soluble polymer. This can occur by any of a variety of chemical methods known in the art. For example, in one embodiment the recombinant AAT protein is modified by the conjugation of PEG to free amino groups of the protein using N-hydroxysuccinimide (NHS) esters. In another embodiment the water-soluble polymer, for example PEG, is coupled to free SH groups using maleimide chemistry or the coupling of PEG hydrazides or PEG amines to carbohydrate moieties of the recombinant AAT protein after prior oxidation.

According to one embodiment of the present invention, PEGylation is performed using PEG of 20 kDa or more. PEG is conjugated to AAT and provides an effect of wrapping around the protein, which leads to the defense against the loss after binding to a scavenger receptor or degradation by an inactivating protease.

PEG is a polymer in a chain form that is linear or branched, and PEG having small molecular weight cannot fully wrap the protein and thus cannot fully protect the protein. Thus, the molecular weight should be the same or above the critical level to sufficiently wrap the protein to be protected. PEGylation of the recombinant AAT protein of the present invention can be carried out by substituting the amino acid at the PEGylation position with cysteine and then conjugating to the PEG containing an acryloyl, sulfone or maleimide group specific to cysteine at one end.

### Recombinant human AAT isolation/purification:

According to the present invention, AAT is preferably purified or isolated, for example from a culture supernatant or other preparation obtained from yeast culture, using means known to a skilled person. The harvest of material obtained by fermentation of *Pichia pastoris* is possible using standard laboratory equipment, in order to process products from a supernatant.

For example, a suitable process comprises of a centrifugation step and/or depth filtration, followed optionally by a (sterile) membrane filtration. Also available is a low-speed centrifugation, sufficient to pellet down the yeast cells, leaving a clear and transparent supernatant, which also may serve as evidence for the absence of bacterial contamination. Such procedures yield supernatant ready for follow-on purification steps, such as chromatography.

For example, various methods involving liquid chromatography, suitable to separate a protein of interest, may be elected by a skilled person without undue effort. For example, solid phases used for chromatography, known as chromatography media or resins, are typically engineered porous inert supports functionalized with various chemical groups that determine the interactions with the molecules to be separated. Commonly used modes of separation, applicable for the rAAT described herein, are, without limitation, based on specific binding interactions (affinity chromatography), charge (ion exchange chromatography), size (size exclusion chromatography/gel filtration chromatography), hydrophobic surface area (hydrophobic interaction chromatography and reverse phase chromatography) and/or multiple properties (multimodal or mixed-mode chromatography) that distinguish based upon size and/or aggregation can be selected to provide a glycoprotein preparation with a desired glycan characteristic or characteristics.

For example, normal phase liquid chromatography can be used to separate proteins, glycans and/or glycoproteins based on polarity. Reverse-phase chromatography can be used, e.g., with derivatized sugars. Anion- exchange columns can be used to purify proteins or modified proteins, with sialylated, phosphorylated, and/or sulfated sugars. Other methods include high pH anion exchange chromatography and size exclusion chromatography, which can be employed in the present invention and are based on size separation.

Affinity based methods can be selected that preferentially bind certain proteins and/or glycan structures. Matrices such as m-aminophenylboronic acid, immobilized lectins and antibodies can bind particular proteins and/or glycan structures. M-aminophenylboronic acid matrices can form a temporary covalent bond with any molecule (such as a carbohydrate) that contains a 1,2-cis-diol group. The covalent bond can be subsequently disrupted to elute the protein of interest. Lectins are a family of carbohydrate-recognizing proteins that exhibit affinities for various monosaccharides. Lectins bind carbohydrates specifically and reversibly. Primary monosaccharides recognized by lectins include mannose/glucose, galactose/N-acetylgalactosamine, N-acetylglucosamine, fucose, and sialic acid (QProteome Glycoarray Handbook, Qiagen, September 2005, available at:
http://wolfson.huji.ac.il/purification/PDF/Lectins/QIAGEN_GlycoArrayHandbook.pdf ) or similar references.

According to the present invention, anion-exchange chromatography (AEX) is one option to purify the rhAAT produced from Pichia pastoris. AEX is a process that separates substances based on their charges using an ion-exchange resin containing positively charged groups, such as diethylaminoethyl groups (DEAE). In solution, the resin is coated with positively charged counter-ions (cations). Anion exchange resins will bind to negatively charged molecules, displacing the counter-ion. Anion exchange chromatography is commonly used to purify proteins, amino acids, sugars/carbohydrates and other acidic substances with a negative charge at higher pH levels. The tightness of the binding between the substance and the resin is based on the strength of the negative charge of the substance.

In embodiments, rhAAT produced from Pichia pastoris can be purified by size-exclusion chromatography (SEC). SEC is a chromatographic method in which molecules in solution are separated by their size, and in some cases molecular weight. It is usually applied to large molecules or macromolecular complexes such as proteins and industrial polymers. Typically, when an aqueous solution is used to transport the sample through the column, the technique is known as gel-filtration chromatography, versus the name gel permeation chromatography, which is used when an organic solvent is used as a mobile phase. The chromatography column is packed with fine, porous beads which are commonly composed of dextran, agarose, or polyacrylamide polymers. The pore sizes of these beads are used to estimate the dimensions of macromolecules. SEC is a widely used polymer characterization method because of its ability to provide good molar mass distribution (Mw) results for polymers.

In embodiments, rhAAT produced from Pichia pastoris can be purified by affinity chromatography. Affinity chromatography typically employs an antibody or other binding reagent specific for the target protein, rhAAT. Affinity chromatography is typically considered as any separation method based on a specific binding interaction between an immobilized ligand and its binding partner. Examples include antibody/antigen, enzyme/substrate, and enzyme/inhibitor interactions. Affinity chromatography has the advantage of specific binding interactions between the analyte of interest (normally dissolved in the mobile phase), and a binding partner or ligand (immobilized on the stationary phase). In a typical affinity chromatography experiment, the ligand is attached to a solid, insoluble matrix, usually a polymer such as agarose or polyacrylamide, chemically modified to introduce reactive functional groups with which the ligand can react, forming stable covalent bonds. The stationary phase is typically first loaded into a column to which the mobile phase is introduced. Molecules that bind to the ligand will remain associated with the stationary phase. A wash buffer is then applied to remove non-target biomolecules, while the biomolecules of interest will remain bound. Target biomolecules may then be removed by applying a so-called elution buffer. The target molecule is thus recovered in the eluting solution. Affinity chromatography resin with high selectivity for efficient industrial purification of alpha-1 antitrypsin (AAT) is known, and is for example available from Cytiva (Alpha-1 Antitrypsin Select). Alpha-1 Antitrypsin Select is a packed-bed affinity chromatography resin with high selectivity for AAT. It can be used for purifying human AAT from plasma, as well as from recombinant or transgenic sources.

The approach of purifying rhAAT produced according to the present invention can be carried out by any suitable approach in the art in terms of the cost and demand of purification degree.

### Medical Indications:

In one aspect of the present invention there is provided a pharmaceutical composition or combination as herein described for use in the treatment and/or prevention of a non-viral disease of the lung associated with inflammation and/or a pathological immune response. Preferred treatments relate to treating a non-viral disease of the lung associated with inflammation and/or a pathological immune response or associated diseases are those described herein.

Typically, the term "inflammation" as used in its art-recognized sense relates to a localized or systemic protective response elicited by injury, infection or destruction of tissues which serves to protect the subject from an injurious agent and the injured tissue. Inflammation is preferably characterized by fenestration of the microvasculature, leakage of the elements of blood into the interstitial spaces, and migration of leukocytes into the inflamed tissue, which may lead to an uncontrolled sequence of pain, heat, redness, swelling, and loss of function.

Inflammation can be classified as either acute or chronic. Acute inflammation is the initial response of the body to harmful stimuli and is achieved by the increased movement of plasma and leukocytes (especially granulocytes) from the blood into the injured tissues. A cascade of biochemical events propagates and matures the inflammatory response, involving the local vascular system, the immune system, and various cells within the injured tissue. Prolonged inflammation, known as chronic inflammation, leads to a progressive shift in the type of cells present at the site of inflammation and is characterized by simultaneous destruction and healing of the tissue from the inflammatory process.

The term "unwanted inflammation" or "pathological inflammation" preferably refers to an inflammation in a subject that exceeds the level of a physiological beneficial inflammatory reaction and leads to damages of the cells, tissues and/or organs at the site of the inflammation.

The term "unwanted immune response" or "pathological immune response" preferably refers to an alteration of the reactivity of the immune system in a subject that has deleterious effect on its health and may involve the stimulation and/or production of cytokines as well as the recruitment of immune cells. Unwanted immune responses occur for instance in autoimmune diseases, transplant rejections, allergies or inflammatory diseases. Cytokines involved in "unwanted inflammation" and/or "pathological immune response" in non-viral diseases of the lung, which contribute to further progression of the inflammation are termed "inflammatory cytokines" or and include the proinflammatory cytokines IL-1AB (also termed IL-1Aβ), IL-4, IL-5, IL-6, IL-8, CXCL8, IL-33, IFN-γ, TNF-α and the anti-inflammatory cytokines IL-1 receptor antagonist (IL-1R antagonist, IL1-Ra, IL-1 RA) and IL-10.

Examples of inflammatory diseases of the lung include but are not limited to lung injury, chronic obstructive pulmonary disease (COPD) including chronic bronchitis, emphysema, bronchiectasis and bronchiolitis, acute respiratory distress syndrome (ARDS), asthma, sarcoidosis, hypersensitivity pneumonitis and/or pulmonary fibrosis and bronchiolitis obliterans syndrome (BOS) such as BOS associated with lung transplantation and/or allogenic hematopoietic stem cell transplantation (chronic graft-versus host disease).

Asthma also termed "bronchial asthma" is a chronic inflammatory disease of the respiratory tract, which is characterized by bronchial inflammation and hyperreactivity and variable airway obstruction. Due to persistent inflammation of the bronchial mucosa further a remodeling of the bronchial walls can occur including hypertrophy of the smooth muscle, thickening of the subepithelial basement membrane, hyperplasia of the glandular cells and a subepithelial fibrosis. Asthma can be classified into allergic (extrinsic) asthma and non-allergic (intrinsic) asthma, whereby however mixed forms are observed in most patients. Allergic asthma is caused by external stimuli (allergenic substances in the environment, so-called allergens) is often associated with genetic predisposition to atopy. Type E immunoglobulins (IgE) are formed which, in interaction with the external stimuli such as specific allergens, cause the release of messenger substances such as histamine, leukotrienes and bradykinins from mast cells. These substances trigger airway constriction. In addition to this immediate type I reaction after contact with the allergen, a delayed reaction can occur after 6 to 12 hours; which is triggered by type G immunoglobulins (IgG). Often both reactions occur (dual reaction). Non-allergic asthma can be caused by other stimuli including Infections, usually of the respiratory tract, drug intolerances - so-called analgesic asthma (a pseudoallergic reaction to analgesics, usually nonsteroidal anti-inflammatory drugs such as acetylsalicylic acid), drug side effects (e.g. of beta-receptor blockers and cholinesterase inhibitors), exposure to toxic (toxic) or irritating substances (solvents, cold air, additives, and others), special physical exertion (exercise-induced asthma), and reflux disease (reflux of stomach acid). Asthma can further be classified by the severity of the symptoms and the corresponding lung function (usually assessed by spirometry, wherein the one second capacity (FEV1), the Peak Expiratory Flow (PEF), the forced (expiratory) vital capacity (FVC), the mid or mean expiratory flow (MEF) can be determined). These classes include intermittent, low degree persistent, medium persistent and severely persistent asthma. Asthma can further be classified by the therapy control and response into controlled asthma, partially controlled asthma and uncontrolled asthma. Treatment of asthma to date includes administration of the administration of beta-2-agonists such as salbutamol, bambuterol and indacaterol, glucocorticoids such as beclomethasone, budesonide and fluticasone, theophylline, leukotriene antagonist such as montelukast, cromoglicic acid, nedocromil and lodoxamid usually by inhalation.

Bronchiolitis obliterans (BO) is a rare, chronic, pulmonary disease characterized by inflammation and fibrous wall thickening of the bronchioles, narrowing their lumens and restricting air circulation. BO mostly emerges after pulmonary infection, or in combination with associated conditions, such as lung or allogeneic hematopoietic stem cell transplantations (chronic graft-versus-host disease). BO emerging after lung or allogeneic hematopoietic stem cell transplantations (chronic graft-versus-host disease) is termed Bronchiolitis obliterans syndrome (BOS). Further several autoimmune diseases such as polymyositis, dermatomyositis, sklerodermia, systemic Lupus erythematodes (SLE), chronic inflammatory bowel diseases, paraneoplastic Pemphigus, Non-Hodgkin-Lymphoma, Paraneoplastic Autoimmune Multiorgan Syndrome (PAMS), CML, Morbus Castleman, thymoma and Morbus Waldenström can be associated with BO. Post-infectious BO (PiBO) also termed "Swyer-James-Syndrome" can occur subsequent to an infection with bacteria such as mycoplasma pneumoniae or a virus such as an adenovirus, RSV, an influenza virus or a parainfluenza virus. Bronchiolitis obliterans can lead to dyspnoea and a dry cough. These symptoms are often progressive over weeks to months. The lung function test shows an obstructive ventilation disorder: reduction in one second capacity (FEV1), increase in residual volume (RV) and RV/ TLC and normal carbon monoxide diffusion capacity (DLCO). Treatment of BO to date usually includes administration of anzithromycinm, cyclophosphamide, ciclosporin, azathioprine, anti-CD3-antibodies, tacrolimus, mycophenolate or statins.

Acute respiratory distress syndrome (ARDS) is a life-threatening condition. It is characterized by a massive disturbance of gas exchange due to impaired permeability of the pulmonary capillaries and tissue oedema due to an inflammatory reaction but neutrophil elastase. The severity of the disease varies depending on the degree of hyperinflammation. ARDS is often associated with a multi-organ failure in the context of a systemic inflammatory response syndrome (SIRS) and has a high mortality rate. The most common cause for ARDS is pneumonia. Further pulmonary causes include pulmonary contusion, aspiration of stomach contents or water, inhalation trauma (smoke inhalation, burns), ventilation with high oxygen concentrations (so-called respirator lung), fat embolism, amniotic fluid embolism, E-cigarette-induced lung injury (EVALI), probably due to additions of vitamin E to the liquid. Systemic causes of ARDS include SIRS, sepsis, shock, disseminated intravascular coagulation, polytrauma, pre-eclampsia, eclampsia, burns, acute pancreatitis, medication and toxins (e.g., salicylic acic, tricyclic antidepressants, bleomycin, organophosphates, paraquas, narcotics), metabolic derailments (e.g., uraemia, liver failure, diabetic ketoacidosis), lung transplant, craniocerebral trauma, mass transfusion (TRALI) and radiation.

In this context the term "non-viral disease of the lung associated with inflammation and/or a pathological immune response" refers to a disease which can be initiated by a variety of factors, as disclosed above, which subsequently lead to an ongoing, often self-reinforcing inflammation and pathological immune response independent of the initial cause. For example, the inflammation occurring in Bronchiolitis obliterans (BO) leading fibrous wall thickening of the bronchioles, narrowing their lumens and restricting air circulation occurs in both Post-infectious BO (PiBO) and Bronchiolitis obliterans syndrome (BOS) and is thus independent of the initial cause of the disease. In this context the use of rhAAT in the treatment and/or prevention of such non-viral diseases of the lung characterized by an ongoing, often self-reinforcing inflammation and pathological immune response refers to the anti-inflammatory function of rhAAT acting on the inflammatory process and not on the initial cause of the disease, such as a viral infection, e.g., by preventing viral entry into the cell.

Any one or more of the above-mentioned a non-viral disease of the lung associated with inflammation and/or a pathological immune response may be susceptible to the inventive treatment described herein and the treatment of each disease as such represents an embodiment of the present invention.

The term "serum half-life" refers to the period of time that elapses after intravenous administration between the maximum concentration of a drug in the blood plasma until it drops to half this value. The term pulmonary half-life refers to the period of time that elapses after administration to the lung for example by inhalation between the maximum concentration of a drug in the lung tissue, for example in the bronchial or alveolar tissue until it drops to half this value. The term "bronchial half-life" refers the period of time that elapses after administration to the lung for example by inhalation between the maximum concentration of a drug in the bronchial tissue until it drops to half this value. The term "alveolar half-life" refers the period of time that elapses after administration to the lung for example by inhalation between the maximum concentration of a drug in the alveolar tissue until it drops to half this value.

As used herein, the "patient" or "subject" may be a vertebrate. In the context of the present invention, the term "subject" includes both humans and animals, particularly mammals, and other organisms.

As used herein, "a subject in need thereof," refers to a subject afflicted with, or at risk of developing, a disease, specifically, coronavirus disease. The subject in need thereof may be a subject being hospitalized with symptoms of coronavirus disease, a subject having symptoms of coronavirus diseases and is under ambulatory setting or at home, or a subject having asymptomatic coronavirus disease and is under ambulatory setting or at home.

In the present invention "treatment" or "therapy" generally means to obtain a desired pharmacological effect and/or physiological effect. In one embodiment "treatment" or "therapy" means slowing, stopping or reversing the disorder's progression. The effect may be prophylactic in view of completely or partially preventing a disease and/or a symptom, for example by reducing the risk of a subject having a disease or symptom or may be therapeutic in view of partially or completely curing a disease and/or adverse effect of the disease.

In the present invention, "therapy" includes arbitrary treatments of diseases or conditions in mammals, in particular, humans, for example, the following treatments (a) to (c): (a) Prevention of onset of a disease, condition or symptom in a patient; (b) Inhibition of a symptom of a condition, that is, prevention of progression of the symptom; (c) Amelioration of a symptom of a condition, that is, induction of regression of the disease or symptom.

### Compositions and administration:

The AAT protein or composition comprising said protein as described herein may comprise different types of carriers depending on whether they are to be administered in solid, liquid or aerosol form, and whether they need to be sterile for such routes of administration as injection.

The active agent AAT can be administered intravenously, intradermally, intraarterially, intraperitoneally, intralesionally, intracranially, intraarticularly, intraprostaticaly, intrapleurally, intratracheally, intranasally, intravitreally, intravaginally, intrarectally, topically, intratumorally, intramuscularly, intraperitoneally, subcutaneously, subconjunctival, intravesicularlly, mucosally, intrapericardially, intraumbilically, intraocularally, orally, topically, locally, inhalation (e.g., aerosol inhalation), injection, infusion, continuous infusion, directly, via a catheter, via a lavage, in cremes, in lipid compositions (e.g., liposomes), locally applied by sponges or by other method or any combination of the forgoing as would be known to one of ordinary skill in the art (see, for example, Remington's Pharmaceutical Sciences, 18th Ed. Mack Printing Company, 1990, incorporated herein by reference).

The present invention encompasses treatment of a patient by introducing a therapeutically effective number polypeptides into a subject, or a subject's bloodstream. As used herein, "introducing" polypeptides into the subject's bloodstream shall include, without limitation, introducing such polypeptides into one of the subject's veins or arteries via injection. Such administering can also be performed, for example, once, a plurality of times, and/or over one or more extended periods. A single injection is preferred, but repeated injections over time (e.g., quarterly, half-yearly or yearly) may be necessary in some instances. Such administering is also preferably performed using an admixture of polypeptides and a pharmaceutically acceptable carrier. Pharmaceutically acceptable carriers are well known to those skilled in the art and include, but are not limited to, 0.01-0.1 M and preferably 0.05 M phosphate buffer or 0.8% saline.

As used herein, the term "therapeutically effective amount" is exchangeable with any one of "therapeutically effective dose" or "sufficient/effective amount or dose," and refers to a dose that produces the required therapeutic effects. Specifically, an effective dose generally refers to the amount of the composition disclosed herein sufficient to induce immunity, to prevent and/or ameliorate coronavirus infection, or to reduce at least one symptom associated with the coronavirus infection and/or to enhance the efficacy of another therapeutic composition. An effective dose may refer to the amount of the composition sufficient to delay or minimize the onset of an infection. An effective dose may refer to the amount of the composition sufficient to prevent infection by a virus or reduce risk of infection by a virus. An effective dose may also refer to the amount of the composition that provides a therapeutic benefit in the treatment or management of infection. In addition, an effective dose may be the amount with respect to the composition alone, or in combination with other therapies, that provides a therapeutic benefit in the treatment or management of a viral infection. An effective dose may also be the amount sufficient to enhance a subject's (in particular, human's) own immune response against a subsequent exposure to coronavirus. The exact effective dose depends on the purpose of the treatment, and is ascertainable by one skilled in the art using known techniques.

As used herein, "pharmaceutically acceptable carrier" includes any and all solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions.

Additionally, such pharmaceutically acceptable carriers can be aqueous or non-aqueous solutions, suspensions, and emulsions, most preferably aqueous solutions. Aqueous carriers include water, alcoholic/aqueous solutions, emulsions and suspensions, including saline and buffered media. Parenteral vehicles include sodium chloride solution, Ringer's dextrose, dextrose and sodium chloride, lactated Ringer's and fixed oils. Intravenous vehicles include fluid and nutrient replenishers, electrolyte replenishers such as Ringer's dextrose, those based on Ringer's dextrose, and the like. Fluids used commonly for i.v. administration are found, for example, in Remington: The Science and Practice of Pharmacy, 20th Ed., p. 808, Lippincott Williams S-Wilkins (2000). Preservatives and other additives may also be present, such as, for example, antimicrobials, antioxidants, chelating agents, inert gases, and the like.

The phrase "pharmaceutically acceptable" refers to molecular entities and compositions that do not produce an allergic or similar untoward reaction when administered to a human. The preparation of an aqueous composition that contains a protein as an active ingredient is well understood in the art. Typically, such compositions are prepared as injectables, either as liquid solutions or suspensions; solid forms suitable for solution in, or suspension in, liquid prior to injection can also be prepared. The preparation can also be emulsified.

Examples of parenteral dosage forms include aqueous solutions of the active agent, in an isotonic saline, 5% glucose or other well-known pharmaceutically acceptable liquid carriers such as liquid alcohols, glycols, esters, and amides. The parenteral dosage form in accordance with this invention can be in the form of a reconstitutable lyophilizate comprising a dose of a composition comprising granulin. In one aspect of the present embodiment, any of a number of prolonged or sustained release dosage forms known in the art can be administered such as, for example, the biodegradable carbohydrate matrices described in U.S. Patent Nos. 4,713,249; 5,266,333; and 5,417,982, the disclosures of which are incorporated herein by reference.

In an illustrative embodiment pharmaceutical formulations for general use with AAT for parenteral administration comprising: a) a pharmaceutically active amount of the granulin; b) a pharmaceutically acceptable pH buffering agent to provide a pH in the range of about pH 4.5 to about pH 9; c) an ionic strength modifying agent in the concentration range of about 0 to about 250 millimolar; and d) water soluble viscosity modifying agent in the concentration range of about 0.5% to about 7% total formula weight are described or any combinations of a), b), c) and d).

In various illustrative embodiments, the pH buffering agents for use in the compositions and methods herein described are those agents known to the skilled artisan and include, for example, acetate, borate, carbonate, citrate, and phosphate buffers, as well as hydrochloric acid, sodium hydroxide, magnesium oxide, monopotassium phosphate, bicarbonate, ammonia, carbonic acid, hydrochloric acid, sodium citrate, citric acid, acetic acid, disodium hydrogen phosphate, borax, boric acid, sodium hydroxide, diethyl barbituric acid, and proteins, as well as various biological buffers, for example, TAPS, Bicine, Tris, Tricine, HEPES, TES, MOPS, PIPES, Cacodylate, MES.

In another illustrative embodiment, the ionic strength modulating agents include those agents known in the art, for example, glycerin, propylene glycol, mannitol, glucose, dextrose, sorbitol, sodium chloride, potassium chloride, and other electrolytes.

Compositions of the present invention are preferably formulated for parenteral administration, e.g., intravascular (intravenous or intraarterial), intraperitoneal or intramuscular administration. The term "parenteral administration" refers to a mode of administration, such as by injection or infusion, other than through the alimentary canal and further refers to subcutaneous, intramuscular, or intravenous injection, intraperitoneal injection, which is also contemplated herein. Intramuscular administration includes intravenous or intraarterial administration. The liquid pharmaceutical compositions, whether they be solutions, suspensions or other like form, may include one or more of the following suitable excipients: sterile diluents such as water for injection, saline solution, preferably physiological saline, Ringer's solution, isotonic sodium chloride, fixed oils such as synthetic mono or diglycerides which may serve as the solvent or suspending medium, polyethylene glycols, glycerin, propylene glycol or other solvents; antibacterial agents such as benzyl alcohol or methyl paraben; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parenteral preparation can be enclosed in ampoules, disposable syringes or multiple dose vials made of glass or plastic. An injectable pharmaceutical composition is preferably sterile. In addition, the pharmaceutical compositions to be administered may also contain, if desired, small amounts of nontoxic auxiliary substances such as wetting or emulsifying agents, pH buffering agents, stabilizers, dissolution enhancers and other such agents, for example, sodium acetate, sorbitan monolaurate, triethanolamine oleate and cyclodextrins.

The unitary daily dosage of the composition comprising the AAT protein can vary significantly depending on the patient condition, the disease state being treated, the route of administration of AAT and tissue distribution, and the possibility of co-usage of other therapeutic treatments. The effective amount of AAT to be administered to the patient is based on body surface area, patient weight, physician assessment of patient condition, and the like.

In one illustrative embodiment, an effective dose of AAT can range from about 1 mg/kg of patient body weight to about 500 mg/kg of patient body weight, more preferably from about 10 mg/kg of patient body weight to about 300 mg/kg of patient body weight, and more preferably from about 50 mg/kg of patient body weight to about 200 mg/kg of patient body weight, for example about 100 mg/kg or about 180 mg/kg body weight.

According to the invention, administration of the composition may preferably occur through inhalation to a mucosal surface of the respiratory tract a subject. "Inhalation administration" refers to both mouth inhalation and nasal inhalation. "Mouth inhalation" relates to drugs administered by inhalation through the mouth and must be atomized into smaller droplets than those administered by the nasal route, so that the drugs can pass through the windpipe (trachea) and into the lungs. How deeply into the lungs they go depends on the size of the droplets. Smaller droplets go deeper, which increases the amount of drug absorbed. Inside the lungs, they are absorbed into the bloodstream.

The drug administered by this route can be delivered by a metered-dosed container (inhaler) in form of dry powder.

The drug can also be nebulized from a solution comprising said drug into mist or into suitable-sized droplets or aerosol by a nebulizer, such as Pari Boy Pro for inhalation.

The inhaled medications can be absorbed quickly and act both locally and systemically. An inhaler device can achieve the correct dose. In general, only 20-50% of the pulmonary-delivered dose rendered in powdery particles will be deposited in the lung upon mouth inhalation. The remainder of 50-70% undeposited aerosolized particles are cleared out of lung as soon as exhalation. In embodiments an inhaled powdery particle that is >8 µm is structurally predisposed to depositing in the central and conducting airway by inertial impaction. In embodiments, an inhaled powdery particle that is between 3 and 8 µm in diameter tend to largely deposit in the transitional zones of the lung by sedimentation. In embodiments, an inhaled powdery particle that is <3 µm in diameter is structurally predisposed to depositing primarily in the respiratory regions of the peripheral lung via diffusion.

The term "nasal inhalation" refers to a drug product or preparation, including the delivery device if applicable, whose intended site of deposition is the respiratory tract or the nasal or pharyngeal region. Nasal inhalation does not include a topical nasal spray or irrigation that is deposited primarily in the nasal passages. The drug can also be nebulized from a solution comprising said drug into mist or into suitable-sized droplets or aerosol by a nebulizer such as Pari Boy Pro for inhalation.

The simultaneous administration and/or subsequential administration of the pharmaceutical composition by different administration routes are also contemplated in the context of the invention. In embodiments, the administration route of the composition comprises inhalation administration. In a preferred embodiment, parenteral administration of the pharmaceutical composition according to the present invention or a pharmaceutical composition comprising a human plasma derived AAT protein is carried out simultaneously or subsequentially. In another preferred embodiment, nasal and/or oral administration of pharmaceutical composition according to the present invention or a pharmaceutical composition comprising a human plasma derived AAT protein is carried out simultaneously or subsequentially.

As used herein, the "respiratory tract" is commonly considered to comprise the nose, pharynx, larynx, trachea, and the lung with its different compartments. The respiratory tract is involved with the process of respiration in mammals, and is a part of the respiratory system, and is lined with respiratory mucosa or respiratory epithelium.

As used herein, a "mucosal surface" is characterised by the presence of an overlying mucosal fluid, e.g. saliva, tears, nasal, gastric, cervical and bronchial mucus, the functions of which include to supply and deliver an array of immunoregulatory and pro-healing species including growth factors, antimicrobial proteins and immunoglobulins.

As used herein, a "pharmaceutical excipient" is essentially anything other than the active pharmaceutical ingredient in a composition.

In the formulation of pharmaceutical compositions, excipients are generally added along with the active pharmaceutical ingredients in order to 1) protect, support or enhance the stability of the formulation; 2) bulk up the formulation in case of potent drug for assisting in the formulation of an accurate dosage form; 3) improve patient acceptance; 4) help improve bioavailability of active drugs; 5) enhance overall safety and effectiveness of the formulation during its storage and use.

Excipients used in the formulation of pharmaceutical compositions are sub-divided into various functional classifications, depending on the role they intend to play in the resultant formulation. Some excipients can have different functional roles in different formulation types and in addition, individual excipients can have different grades, types, and sources depending on those different functional roles. The possible types of excipients commonly used in the formulation of pharmaceutical suspensions include solvents/vehicle, co-solvent, buffering agents, preservatives, antioxidants, wetting agents/surfactants, anti-foaming agents, flocculation modifiers, suspending agents/viscosity-modifiers, flavouring agents, sweetening agents, colorants, humectants, and chelating agents.

As used herein, "cryopreservation" is a process where organelles, cells, tissues, extracellular matrix, organs, or any other biological constructs susceptible to damage caused by unregulated chemical kinetics are preserved by cooling to very low temperatures by freezing (typically -20 to - 80 °C using solid carbon dioxide or in some cases to -196 °C using liquid nitrogen). At low enough temperatures, any enzymatic or chemical activity which might cause damage to the biological material in question is effectively stopped. Cryopreservation methods seek to reach low temperatures without causing additional damage caused by the formation of ice crystals during freezing.

As used herein, an "aerosol" is a suspension of liquid and solid particles produced for example by an aerosol generator, such as a small-volume nebulizer (SVN), a pressurized metered-dose inhaler (pMDI), or a dry-powder inhaler (DPI). Aerosol deposition is a process of aerosol particles depositing on absorbing surfaces.

Aerosol devices in clinical use produce heterodisperse (also termed polydisperse) particle sizes, meaning that there is a mix of sizes in the aerosol. Specific sizes and/or size ranges are presented in the Summary of the invention above. Monodisperse aerosols, which consist of a single particle size, are rare. Polydisperse aerosols can be defined by the mass median diameter (MMD). This measure determines the particle size (in µm) above and below which 50% of the mass of the particles is contained. This is the particle size that evenly divides the mass, or amount of the drug in the particle size distribution. This is usually given as the mass median aerodynamic diameter, or MMAD, due to the way sizes are measured. The higher the MMAD, the more particle sizes are of larger diameters.

Dry powder formulations can offer advantages, including greater stability than liquid formulations and potential that preservatives may not be required. Powders tend to stick to the moist surface of the nasal mucosa before being dissolved and cleared. The use of bio-adhesive excipients or agents that slow ciliary action may decrease clearance rates and improve absorption. A number of factors like moisture sensitivity, solubility, particle size, particle shape, and flow characteristics will impact deposition and absorption.

According to some embodiments, the pharmaceutical composition administered via inhalation is administered once daily for at least two days. According to some embodiments, the pharmaceutical composition administered via inhalation is administered once daily for at least three days. According to some embodiments, the pharmaceutical composition administered via inhalation is administered once daily for at least four days. According to some embodiments, the pharmaceutical composition administered via inhalation is administered once daily for at least five days. According to some embodiments, the pharmaceutical composition administered via inhalation is administered once daily for at least six days. According to some embodiments, the pharmaceutical composition administered via inhalation is administered once daily for at least a week. According to some embodiments, the pharmaceutical composition administered via inhalation is administered once daily for at least eight days. According to some embodiments, the pharmaceutical composition administered via inhalation is administered once daily for at least nine days. According to some embodiments, the pharmaceutical composition administered via inhalation is administered once daily for at least ten days. According to some embodiments, the pharmaceutical composition administered via inhalation is administered once daily for ten days.

### Examples of dry powder nasal sprays:

SBNL Pharma (www.snbl.com) recently reported data on a Phase 1 study with a zolmitriptan powder cyclodextrin formulation (µcoTM System) for enhanced absorption, described previously in an in vitro study. The zolmitriptan absorption was rapid, and the relative bioavailability was higher than the marketed tablet and nasal spray. The company has a capsule-based, single-dose powder device (Fit-lizer), suitable for administration of a powder formulation according to the present invention. When inserted into a chamber, the top and bottom of the capsule is cut off by sharp blades. A plastic chamber is compressed by hand, compressed air passes through a oneway valve and the capsule during actuation, and the powder is emitted.

Bespak (www.bespak.com), the principle for Unidose-DPTM, is similar to the Fit-lizer device. An air-filled compartment is compressed until a pin ruptures a membrane to release the pressure to emit the plume of powder. Delivery of powder formulations of a model antibody (human IgG) has been tested in a nasal cast model based on human MRI images. Approximately 95 % of the dose was delivered to the nasal cavity, but the majority of it was deposited no further than the nasal vestibule with only about 30 % deposited into deeper compartments of the nasal cavity.

### Examples of dry powder inhalers:

Astra Zeneca markets budesonide powder delivered with the Turbuhaler multi-dose inhaler device modified for nasal inhalation (Rhinocort Turbuhaler^{®}; www.az.com). It is marketed for allergic rhinitis and nasal polyps in some markets as an alternative to the liquid spray.

The Aptar group (www.aptar.com) offers a simple blister-based powder inhaler. The blister is pierced before use and the device nosepiece placed into one nostril. The subject closes the other nostril with the finger and inhales the powder into the nose. A powder formulation of apomorphine for Parkinson's using this blister-based powder inhaler (BiDoseTM/ProhalerTM) from Pfeiffer/ Aptar was in clinical development by Britannia, a UK company recently acquired by Stada Pharmaceutical (www.stada.de). Such administration devices are contemplated for administering the composition as described herein.

### Examples of inhalers with liquid formulations:

Inhalation solution and suspension drug products are typically aqueous-based formulations that contain therapeutically active ingredients and can also contain additional excipients. Aqueous-based oral inhalation solutions and suspension must be sterile. Inhalation solutions and suspensions are intended for delivery to the lungs by oral inhalation for local and/or systemic effects and are to be used with a specified nebulizer, for example Pari Boy Pro which can generate flexible and with variable droplet spectrum for the therapy of severe, chronic respiratory diseases. Unit-dose presentation is recommended for these drug products to prevent microbial contamination during use. The container closure system for these drug products consists of the container and closure and can include protective packaging such as foil overwrap.

For example, amikacin liposome inhalation suspension (ALIS; Arikayce^{®}) is a liposomal formulation of the aminoglycoside antibacterial drug amikacin. The ALIS formulation, administered via inhalation following nebulization, is designed to facilitate targeted and localized drug delivery to the lungs while minimizing systemic exposure. Such administration devices are contemplated for administering the composition as described herein.

### Examples of nasal sprays with liquid formulation:

Nasal spray dispensers are typically non-pressurized dispensers that deliver a spray containing a metered dose of the active ingredient. The dose can be metered by the spray pump or could have been pre-metered during manufacture. A nasal spray unit can be designed for unit dosing or can discharge up to several hundred metered sprays of formulation containing the drug substance. Nasal sprays are applied to the nasal cavity for local and/or systemic effects.

The liquid nasal formulations are mainly aqueous solutions, but suspensions and emulsions can also be delivered. Liquid formulations are considered convenient particularly for topical indications where humidification counteracts the dryness and crusting often accompanying chronic nasal diseases.

For example, the dispensers available for example from Nemera (La Verpilliere, France) or Aptar Pharma (Illinois, USA) are preferred. For example, Nemera provides Advancia^{®} nasal spray dispensers, which are high-performing pumps with good dose consistency and prime retention, anti-clogging actuators, no metal contacting the formulation and hygienic anti-actuation snap-on overcaps. As a further example, Aptar Pharma's nasal pump technology removes the need for drug manufacturers to add preservatives to nasal spray formulations. The Advanced Preservative Free (APF) systems use a tip-seal and filter technology to prevent contamination of the formulation. A spring-loaded tip seal mechanism is employed with a filter membrane in the ventilation channel.

Metering and spray producing (e.g., orifice, nozzle, jet) pump mechanisms and components are used for reproducible delivery of drug formulation, and these can be constructed of many parts of different design that are precisely controlled in terms of dimensions and composition. Energy is required for dispersion of the formulation as a spray. This is typically accomplished by forcing the formulation through the nasal actuator and its orifice. The formulation and the container closure system (container, closure, pump, and any protective packaging) collectively constitute the drug product. The design of the container closure system affects the dosing performance of the drug product.

In some embodiments, the multi-use dispenser employs a membrane (preferably of silicone) that prevents bacteria or viruses from entering the reservoir or pump device. In some embodiments, the multi-use dispenser employs a metal-free fluid path, thereby preventing the oxidization of the formulation. In some embodiments, the multi-use dispenser employs a spring-loaded tip seal mechanism, thereby preventing microbes from entering the device between spray events.

In one embodiment, the dispenser is configured for multiple individual spray events of 5 to 1000 µL, preferably 5 to 500 µL, more preferably 10 to 300 µL, more preferably 20 to 200 µL.

In one embodiment, the dispenser comprises a total volume of composition of 0.1 to 500 mL, preferably 1 to 100 mL, more preferably 2 to 50 mL, such as for example about 5, 10 or 15 mL.

### Examples of throat sprays for liquid formulations:

Bona, ShenZhen China, is a supplier of medical packaging and complements. The firm offers a line of long-neck sprays that offer excellent dispensing and dosing, designed for throat sprays and other treatments that require throat targeting. The arm of the dispenser swings 360 degrees for consumer ease of use. The sprayers are suited for medicated throat treatments as well as other treatments that local treatment, for example to protect the throat from viral infections. The dispensers are preferably prepared from pharma-grade PP and PE, and the dispensers can be paired with any bottle size. Currently, the sprayers come in a variety of popular sizes (18/410,18/415, 20/410, 24/410) and can be set to dispense from 220 microliters to 50 microliters.

### Simultaneous and sequential administration

Simultaneous administration of one or more active agents shall mean administration of one or more agents at the same time. For example, in one embodiment, AAT can be administrated together other active agent useful for treating a non-viral disease of the lung associated with inflammation and/or a pathological immune response. In other embodiment, AAT can be administrated by inhalation together by other administration route including oral, nasal, parenteral including intravascular, intraperitoneal, or intramuscular administration. In some embodiments, at least one route of administration to be the recombinant AAT according to the present invention is required, preferably inhalation administration. The other routes of administration can alternatively be a composition comprising human plasma derived AAT or any other recombinantly produced AAT. In embodiments, the administration route applied in a simultaneous manner can also be in a sequential manner.

Sequential administration of one or more active agents shall mean administration of the therapeutic agents in a sequential manner. In one embodiment, each therapeutic agent is administered at a different time. In other embodiments, by administration of two or more therapeutic agents wherein at least two of the therapeutic agents are administered in a sequential manner which is a substantially simultaneous manner. Substantially simultaneous administration can be accomplished, for example, by administering to the subject a single dose having a fixed ratio of each therapeutic agent or in multiple, single doses for each of the therapeutic agents. The one or more therapeutic agents as described herein comprises a least the pharmaceutical composition comprising recombinant AAT protein according to the present invention. In an embodiment, the therapeutic agents comprise further pharmaceutical composition comprising human plasma derived AAT and/or other recombinantly produced AAT protein. In a preferred embodiment, the therapeutic agents comprise further pharmaceutical composition comprising Xanthohumol.

As used herein, the term "simultaneously" refers to administration of one or more agents at the same time. For example, in certain embodiments, administration of AAT in combination with other active agents useful for treating a viral infection or medical condition associated with lung inflammation are administered at simultaneously. Simultaneously includes administration contemporaneously, that is during the same period of time. In certain embodiments, the one or more agents are administered simultaneously in the same hour, or simultaneously in the same day. Sequential or substantially simultaneous administration of each therapeutic agent can be affected by any appropriate route including, but not limited to, oral routes, intravenous routes, subcutaneous routes, intramuscular routes, direct absorption through mucous membrane tissues (e.g., nasal, mouth, vaginal, and rectal), and ocular routes (e.g., intravitreal, intraocular, etc.). The therapeutic agents can be administered by the same route or by different routes. For example, one component of a particular combination may be administered by inhalation while the other component(s) of the combination may be administered intravenously. The components may be administered in any therapeutically effective sequence.

The decision for simultaneous and sequential administration can be taken depending on the severity of the disease. For example, for the patient with severe symptoms of BO, Asthma or ARDS receiving the inhalation of AAT, a simultaneous and/or sequential intravenous administration of AAT protein can be decided.

### Culture Media and Processing:

The production methods described herein can include determining and/or selecting media components or culture conditions which result in the production of a desired glycan characteristic or characteristics of rhAAT. In embodiments, culture parameters that can be determined include media components, pH, feeding conditions, osmolarity, carbon dioxide levels, agitation rate, temperature, cell density, seeding density, timing and sparge rate.

Changes in production parameters such the speed of agitation of a cell culture, the temperature at which cells are cultures, the components in the culture medium, the times at which cultures are started and stopped, variation in the timing of nutrient supply can be selected appropriately. Thus, methods described herein can include one or more of: increasing or decreasing the speed at which cells are agitated, increasing or decreasing the temperature at which cells are cultures, adding or removing media components, and altering the times at which cultures are started and/or stopped. Sequentially selecting production parameters or a combination thereof, as used herein, means a first parameter (or combination) is selected, and then a second parameter (or combination) is selected, e.g., based on a constraint imposed by the choice of the first production parameter.

The methods described herein can include determining and/or selecting a media component and/or the concentration of a media component that has a positive correlation to a desired glycan characteristic. A media component can be added in or administered over the course of glycoprotein production or when there is a change in media, depending on culture conditions. Media components include components added directly to culture as well as components that are a byproduct of cell culture.

Media components include, e.g., buffer, amino acid content, vitamin content, salt content, mineral content, serum content, carbon source content, lipid content, nucleic acid content, hormone content, trace element content, ammonia content, co-factor content, indicator content, small molecule content, hydrolysate content and enzyme modulator content.

Various media components can be selected, comprising amino acids, vitamins, carbon source (natural and unnatural), salts, sugars, sera, plant derived hydrolysates, sodium pyruvate, surfactants, ammonia, lipids, hormones or growth factors, buffers, non-natural amino acids, sugar precursors, indicators, nucleosides or nucleotides, butyrate or organics, DMSO, animal derived products, gene inducers, non-natural sugars, regulators of intracellular pH, Betaine or osmoprotectant, trace elements, minerals, Non-natural amino acids, non-natural vitamins. Exemplary amino acid that can be included or eliminated from the media include alanine, arginine, asparagine, aspartic acid, cysteine, glutamic acid, glutamine, glycine, histidine, proline, isoleucine, leucine, lysine, methionine, phenylalanine, proline, serine, threonine, tryptophan, tyrosine and valine.

Examples of vitamins that can be present in the media or eliminated from the media include vitamin A retinoid vitamin B1 (thiamine), vitamin B2 (riboflavin) vitamin B3 (niacin), vitamin B5 (pantothenic acid), vitamin BO (pyroxidone), vitamin 131 (biotin),vitamin B9 (folic acid), vitamin B12 (cyanocobalamin), vitamin C (ascorbic acid), vitamin D, vitamin E, and vitamin K.

Minerals that can be present in the media or eliminated from the media include bismuth, boron, calcium, chlorine, chromium, cobalt, copper, fluorine, iodine, iron, magnesium, manganese, molybdenum, nickel, phosphorus, potassium, rubidium, selenium, silicon, sodium, strontium, sulfur, tellurium, titanium, tungsten, vanadium, and zinc. Exemplary salts and minerals include CaC12 (anhydrous), CuSO4 5H20, Fe(NO3) ·9H20, ICI, KNO3, KH2PO4, MgSO4 (anhydrous), NaC1, NaH2PO4H2O, NaHCO3, Na2SE3 (anhydrous), ZnSO4.7H20; linoleic acid, lipoic acid, D-glucose, hypoxanthine 2Na, phenol red, putrescine 2HC1, sodium pyruvate, thymidine, pyruvic acid, sodium succinate, succinic acid, succinic acid, glutathione (reduced), para-aminobenzoic acid (PAHA), methyl linoleate, bactopeptone G, adenosine, cytidine, guanosine, 2'-deoxyadenosine HC1, 2'-deoxycytidine HCl, 2'-deoxyguanosine and uridine.

In some embodiments, ammonia content can be selected as a production parameter to produce a desired glycan characteristic or characteristics. For example, ammonia can be present in the media in a range from 0.001 to 50 mM. Ammonia can be directly added to the culture and/or can be produced as a by product of glutamine or glucosamine. Another production parameter is butyrate content. The presence of butyrate in culture media can result in increased galactose levels in the resulting glycoprotein preparation. Butyrate provides increased sialic acid content in the resulting glycoprotein preparation.

In some embodiments, a component such as an enzyme, sugar and/or sugar precursors can be added to media or batch fed to cells to affect glycosynthesis. For example, enzymes and substrates such as sugar precursors can be added to the media or batch fed to cells to produce a desired glycan characteristic or characteristics. These methods can make use of monosaccharide substrates that are taken up by a cell, converted to "activated" monosaccharide substrates in vivo and incorporated into the expressed protein by the cell. The methods are amenable to any cell which can be manipulated to produce a desired glycoprotein. The cell can use, e.g., endogenous biochemical processing pathways or can be genetically engineered to convert, or process, the exogenously added monosaccharide into an activated form that serves as a substrate for conjugation to a target glycoprotein in vivo or in vitro.

Monosaccharides added to a polysaccharide chain can be incorporated in activated form. Activated monosaccharides, which can be added, include UDP-galactose, UDP-glucose, UDP-N-acetylglucosamine, UDP-N-acetylgactosamine, UDP-xylose, GDP-mannose, GDP-fucose, CMP-N-acetylneuraminic acid and CMP-N- acetylglycolylneuraminic acid. Other monosaccharide precursors that can be added to media or batch fed to cells include: N-acetylglucosamine, glucosamine, glucose, galactose, N-acetylgalactosamine, fructose, fucose, glucose-6-phosphate, mannose-6- phosphate, mannose- 1 -phosphate, fructose-6-phosphate, glucosamine-6-phosphate, N- acetylglucosamine-6-phosphate, N-acetylmannosamine, N-acetylneuraminic acid-6- phosphate, fucose-I-phosphate, ATP, GTP, GDP, GMP, CTP, CDP, CMP, UTP, UDP, UMP, uridine, adenosine, guanosine, cytodine, lactose, maltose, sucrose, fructose 1,6 biphosphate, 2 phosphoenol pyruvate, 2-oxaloacetate and pyruvate.

Some aspects include having glucosamine present in the media. Glucosamine can be added to the media or batch fed to the cell or the appropriate enzymes and/or substrates can be added to the media or batch fed to cells such that glucosamine is produced. In some embodiments, when increased levels of high mannose or hybrid glycan structures are desired, a cell (e.g., a Pichia pastoris) can be cultured in the presence of glucosamine. Glucosamine can be present, e.g., at a concentration of about 0.001 to 40 mM. The methods can further include having uridine added to the media or batch fed to a cell, e.g., to reduce the level of high mannose structures associated with a protein produced by the cell. The addition of cytidine, UTP, OMP and/or aspartate to media or batch fed to cells can also result in the production of uridine during culture. Preferably, uridine is present at a concentration of about 0.001 to 10 mM.

Other aspects include selecting a media component or components that do not affect a glycosylation characteristic or characteristics. For example, the presence of glucosamine and uridine in culture does not significantly alter galactosylation, fucosylation, high mannose production, hybrid production or sialylation of glycoproteins produced by a cell (e.g., a Pichia pastoris) cultured in the presence of this combination. When the presence of mannose is a selected production parameter, mannose can be added to the media, batch fed to the cells or can be produced by a cell exposed to the appropriate substrates such as fructose or mannan. Preferably, mannose is present at a concentration of about 0.001 to 50 mM.

### Fermentation parameters and bioreactors:

Methods described herein can include selecting culture conditions that are correlated with a desired glycan characteristic or characteristics. Such conditions can include temperature, pH, osmolality, shear force or agitation rate, oxidation, spurge rate, growth vessel, tangential flow, DO, CO2, nitrogen, fed batch, redox, cell density and feed strategy.

Examples of physiochemical parameters that can be selected from temperature, pH, osmolality, shear force (or agitation rate), oxidation, spurge rate, growth vessel, tangential flow, batch, DO, CO2, nitrogen, fed batch, Redox, cell density, perfusion culture, feed strategy.

In other embodiments, carbon dioxide levels can be selected which results in a desired glycan characteristic or characteristics. CO2 levels can be, e.g., about 5%, 6%, 7%, 8%, 9%, I.0%, 11%, 13%, 15%, 17%, 20%, 23% and 25% (and ranges in between).

A wide array of flasks, bottles, reactors, and controllers allow the production and scale up of cell culture systems. The system can be chosen based, at least in part, upon its correlation with a desired glycan characteristic or characteristics. Cells can be grown, for example, as batch, fed-batch, perfusion, or continuous cultures depending on the correlation with a particular glycan characteristic or characteristics.

Various culture media or growth media may be employed. The most commonly used medium for the high cell density fermentation of P. pastoris is a basal salt medium (BSM), as mentioned in Pichia fermentation process guidelines proposed by Invitrogen (USA). A non-limiting example of medium components for a BSM are described in the Examples below.

In other embodiments, a YPD medium may be employed, which is available in liquid (broth) form for the growth and propagation of yeast cultures. It primarily contains of bacteriological peptone, yeast extract, and glucose. This medium is non-selective for Candida, Pichia, Saccharomyces, and Zygosaccharomyces.

In other embodiments, a yeast nitrogen base (YNB with amino acids) media can be used, which is a commonly used growth medium used for the cultivation of yeast. This nutrient-rich microbial medium contains nitrogen, vitamins, trace elements, and salts. It is suitable for use in classifying yeasts based on amino acid and carbon requirements.

Batch culture is typically carried out by placing the cells to be cultured into a fixed volume of culture medium and allowing the cells to grow. Cell numbers increase, usually exponentially, until a maximum is reached, after which growth becomes arrested and the cells die. Batch culture is characterized in that it proceeds in a fixed volume (since nothing is added after placing the cells in the medium), for a fixed duration (dependent on the length of time the cells survive) with a single harvest.

Fed-batch culture is a variation on batch culture and involves the addition of a feed to the batch. Cells are cultured in a medium in a fixed volume. Before the maximum cell concentration is reached, specific supplementary nutrients are added to the culture. The volume of the feed is minimal compared to the volume of the culture. A fed-batch culture involves a batch cell culture to which substrate, in either solid or concentrated liquid form, is added either periodically or continuously during the period of growth. Fed-batch cultures are described, e.g., in U.S. Pat. Nos. 5,672,502.

In a perfusion culture, medium is perfused through the reactor at a high rate while cells are retained or recycled back into the reactor by sedimentation, centrifugation or filtration. The major function of perfusing such a large volume of medium is primarily to remove the metabolites, mainly lactate, from the culture fluid. Perfusion cultures are described, e.g., in U.S. Pat. No. 6,544,788.

In continuous culture, the cells are initially grown in a fixed volume of medium. To avoid the onset of the decline phase, a pumped feed of fresh medium is initiated before maximum cell concentration is reached. Culture, containing a proportion of the cells, is continuously removed from the vessel to maintain a constant volume. Continuous cultures and bioreactors are described, e.g., in U.S. Pat. Nos. 4,764,471; 5,135,853; 6,156,570.

A bioreactor is a device or system that supports a biologically-active environment, e.g., a device or system meant to grow cells or tissues in the context of cell culture (e.g., mammalian, plant, yeast, bacterial cells). This process can either be aerobic or anaerobic. Bioreactors are commonly cylindrical, ranging in size from some liters to cubic meters, and are often made of stainless steel. On the basis of mode of operation, a bioreactor may be classified as batch, fed batch or continuous batch (e.g. continuous stirred-tank reactor model).

By way of example, the Heraeus miniPERM bioreactor combines an autoclavable outer nutrient container and a disposable inner bioreactor chamber. New Brunswick Scientific's CELLIGEN PLUS^{®} is a highly flexible system for culture of virtually all eukaryotic cell lines. The Wave Bioreactor^{™} (from Wave Biotech, LLC) employs an adjustable-speed rocking platform and electric air pump to aerate the culture. Quark Enterprises provides a full range of bioreactors including Spingro^{®} flasks for high-density culture.

Stir tanks (and flasks) can provide cell cultures with increased density. Examples include isposable Stirred Tank Bioreactors (such as Xcellerex), or scalable, disposable stir tank bioreactors (XDR^{™}). Applikon offers a full line of stir tanks, from 2.3 I bench systems to 10,000 I production units. Batch bioreactors are also available, e.g., from Rockland Immunochemicals, Inc.

### General cultivation of Pichia Pastoris in a stirred-Tank Bioreactor (Non-limiting example):

Pichia pastoris is a methylotrophic yeast which provides a unique expression system for producing high levels of recombinant proteins, including enzymes, protease inhibitors, singlechain antibodies, and regulatory proteins at various levels. The following workflow is provided as a non-limiting example of a Pichia pastoris fermentation, which may be used as an alternative or in combination with the method described in the examples below. Details are provided in an "Introduction to Pichia pastoris culture in a stirred-tank bioreactor" (Application note, 253, October 2011, Eppendorf).

The medium is composed of both temperature sensitive and non-temperature-sensitive materials. Therefore, the inoculum can be prepared in two steps. Start by mixing and autoclaving the following:

| **Initial medium composition** | **Concentration** |
|---|---|
| Calcium sulfate dihydrate | 0.93 g/L |
| Potassium sulfate | 18.2 g/L |
| Magnesium sulfate heptahydrate | 14.9 g/L |
| Potassium hydroxide | 4.13 g/L |
| Phosphoric acid | 26.7 mL/L |
| Glyerol | 40.0 g/L |
| Antifoam (Breox^{®} Foam Control Agent FMT 30) | 1.0 mL/L |

After autoclaving and allowing time for the vessel to cool, addition of temperature-sensitive material can be made:

| **Medium composition** | **Quantity** |
|---|---|
| Trace metals solution, PTM1* | 4.6 mL/L |
| Base, to adjust the initial pH | 25 mL/L |
| Inoculum | 200 mL |
| Glycerol** | 400 mL |
| Methanol** | < 2 L |
| Base to maintain pH at setpoint | < 250 mL |

* 6 g/L cupric sulfate pentahydrate, 0.08 g/L sodium iodide, 3 g/L manganese sulfate monohydrate, 0.5 cobalt chloride (anhydrous), 20 g/L zinc chloride (anhydrous), 0.02 g/L boric acid, 0.2 g/L sodium molybdate dihydrate, 65 g/L ferrous sulfate heptahydrate, 0.2 g/l biotin, 30 mL/L 6N sulfuric acid. ** Added as required.

The inoculum is prepared using Pichia shake-flask growth medium. The inoculum is cultivated for 40 hours at 28° C in a shaker (New Brunswick model G25) running at 240 rpm.

Control setpoints for P. pastoris should be entered and achieved prior to inoculation and, except for dissolved oxygen (DO) which often remains high, the medium should be allowed to equilibrate prior to inoculation. An initial DO value of approximately 100 % is acceptable; it will decrease as the culture metabolizes it. Agitation limits may differ depending on the system and can be set to respond automatically to oxygen demand.

Preferred cultivation conditions are as follows:

| **Parameter** | **Setpoint** |
|---|---|
| Temperature | 30° C |
| pH | 5.0 |
| Dissolved oxygen | 30 % |
| Agitation | 300-1200 rpm* |

Control dissolved oxygen (DO) often includes a cascade, either to agitation, airflow or oxygen, individually or in combination. Cascades allow the controller to maintain setpoints by automatically adjusting other process loops. The inclusion of oxygen in a cascade may help to increase the overall culture density. pH control often uses the addition of liquid acid and liquid base solution to maintain pH at setpoint, but often it relies on the acid/base-producing properties of the culture or medium for a natural drift up or down. P. pastoris pH control is usually done through the addition of base (30 % NaOH).

### Glycosylated AAT produced from yeast

The Pichia pastoris expression system has been used extensively over the past decade for generation of recombinant proteins, and the nature of the processing that occurs in yeast has become a topic of interest. Yeast can carry out glycosylation of the amide nitrogen of asparagine residues in a protein when found in the consensus sequence Asn-Xaa-Thr/Ser, providing N-linked glycosylation. Also, glycosylation of hydroxy groups of threonine and/or serine residues in proteins occurs in yeast cells, yielding O-linked type of glycosylation. For a review, refer to Bretthauer, Biotechnol. Appl. Biochem. (1999) 30, 193-200.

As delineated mainly in Saccharomyces cerevisiae, protein glycosylation involves the following summarized events. N-linked oligosaccharides originate from an oligosaccharide (Glc3Man9GlcNAc2) that is assembled on dolichol (pyrophosphate) in the endoplasmic reticulum (ER), and that is transferred to the appropriate Asn of the nascent protein in a co-translational event. This is a common eukaryotic-cell-type glycosylation pathway for N-linked glycoproteins. However, differences in subsequent processing of the newly glycosylated protein occur in yeast as compared with higher eukaryotic cells (plants, insects and higher animals).

In most yeasts that have been studied, the three glucose residues and one specific a-1,2-linked mannose residue are removed by specific glycosidases in the ER, giving a protein N-linked Man8GlcNAc2 core structure that is further processed in the Golgi complex. This involves addition of a mannose residue that is linked a-1,6 to the a-1,3-linked mannose residue in the Mana-1,3Manb- 1,4GlcNAc inner-core sequence. The 1,6-linked residue can then be elongated to an a-1,6-linked backbone (50-100 residues) that can be branched with a-1 ,2-linked di- or trisaccharides of mannose that in turn can be capped with a- 1,3-linked mannose units. An additional processing event that occurs in most yeasts is the addition of phosphate, in the form of mannose 1-phosphate, to specific a-1,2- or a-1,6-linked mannose residues of the core or the side chain of N-linked oligosaccharides, forming the acidic phosphodiester component found in many yeast glycoproteins.

Complex-type oligosaccharides containing sialic acid, galactose, fucose and N-acetylgalactosamine are not found on S. cerevisiae-derived, and most other yeast-derived, glycoproteins. Therefore, the assembly and processing of only the high-mannose type of N-linked oligosaccharides on the expressed recombinant protein must be taken into account when considering the structure and the subsequent use (function) of the glycoprotein.

Whereas cell-wall mannans from some strains of P. pastoris have been found to contain b-1,2-linked mannose residues, such as present in the structure Man(b-1,2)Man(b-1,2)Man(a-1,2)Man, b-linked mannose residues have not been reported in recombinant glycoproteins from P. pastoris. In addition to the N-linked oligosaccharides, the presence of O-linked oligosaccharides of mannose is common in most yeasts. The biosynthetic process is unique compared with animals, in that the mannose residue that is a-glycosidically linked to the hydroxy group of a serine or threonine residue of the protein is derived in the ER from a dolichol phosphate carrier, dolichyl phosphate mannose, rather than from a sugar nucleotide in the Golgi complex. These short oligosaccharides can also be phosphorylated and thus contribute to the acidity and perhaps other functions of the glycoprotein.

Compared to the known glycosylation of Pichia pastoris-derived proteins, the rhAAT as produced herein contains the unique HexNAc1 glycosylation, preferably comprising 50-100% of total N-glycans, preferably 60-90%, more preferably 70-80%. It was unexpectedly discovered that the rhAAT protein described herein have increased biological and cellular activities as compared to the activity of plasma purified human AAT (Prolastin).

In embodiments, the post-translational modification is one or more of N-glycosylation, O-glycosylation, N-terminal methionine removal, N-acetylation and/or phosphorylation or any combination thereof. In embodiments, the recombinant AAT protein has one or more human-like glycosylations.

In embodiments, yeast for producing AAT as used herein allows the AAT to be modified into human-like structures. The term "human-like" structure or "human-like" glycoform patterns are related to the glycosylation pattern, which e.g. extends the serum half-life of the recombinant AAT and/or stability comparable to human plasma derived AAT. "Human-like" glycoform patterns are preferably different from but similar to human glycosylation patterns, to an extent of extending the serum half-life of the recombinant AAT compared to non-modified AAT protein. "Human-like" glycoform patterns may, by way of example, comprise one or more glycans selected from the group consisting of mannose, N-acetylglucosamine (GlcNAc), galactose, N-Acetylneuraminic acid (Neu5Ac), N-Glycolylneuraminic acid and Xylose.

The glycosylation pattern of recombinant AAT produced in yeast as described herein renders a new form of recombinant AAT. This can be verified by an SDS-page analysis by skilled person. A shift up or down of the band from recombinant AAT produced from yeast, such as Pichia pastoris compared to the band of plasma derived AAT can be an evident of new type of recombinant AAT. Alternatively, different peak distribution exhibited in a comparison analysis by size exclusion HPLC or Anion exchange HPLC between reference AAT (e.g. Prolastin) and AAT produced according to the invention highlights a novel type of rhAAT.

Human AAT typically includes up to 3 N-linked glycans attached to asparagine residues 46, 83, and 247. Naturally occurring AAT includes significant heterogeneity in glycosylation pattern and N-glycan structure. As such, several isoforms of AAT exist, also known as glycoforms. A glycoform is an isoform of a protein that differs only with respect to the number or type of attached glycan. Glycoproteins often consist of a number of different glycoforms, with alterations in the attached saccharide or oligosaccharide.

N-linked glycosylation refers to the attachment of an oligosaccharide, a carbohydrate consisting of several sugar molecules, sometimes also referred to as glycan, to a nitrogen atom (the amide nitrogen of an asparagine (Asn) residue of a protein), in a process called N-glycosylation. The resulting protein may be referred to as an N-linked glycan, or simply an N-glycan.

In one embodiment, the rhAAT glycoprotein includes 1, 2, 3, 4, 5, 6, 7, 8, 9, 10, 11, 12, 13, 14 or 15 predominant N-glycan structures. The term "predominant N-glycan structure" or "predominant glycoform" shall mean a specific N-glycan structure or glycoform of (i.e ., attached to) a protein constitutes the greatest percentage of all N-glycan structures or glycoforms of the protein. In embodiments, a predominant glycoform(s) accounts for at least about 40%, at least about 50%, at least about 60%, at least about 70%, at least about 80%, at least about 90%, or at least about 95% or greater of the population of all glycoforms on the protein.

In embodiments, a predominant N-glycan can be HexNAc1 glycosylation, wherein HexNAc1 glycosylation comprises 50-100% of total N-glycans, preferably 60-90%, more preferably 70-80%. In embodiments, a predominant N-glycan can be Hex9HexNAc2, Hex10HexNAc2, Hex11HexNAc2, Hex12HexNAc2, Hex13HexNAc2, Hex14HexNAc2 and Hex15HexNAc2 glycosylation.

As used herein, "HexNAc1" shall mean an amide derivative of a hexose, and may also be referred to as N-acetylhexosamine (HexNAc), which is an acetylated derivative of hexosamine (HexN). An example includes N-acetylglucosamine (GlcNAc), which is an acetylated derivative of glucosamine (GlcN).

In the context of the present invention, HexNAc1 refers to a single monosaccharide, not a component of an oligosaccharide. This applies in particular to the occupancy of any given glycosylation site. For example, the occupancy data for HexNAc1 solely relates the occupancy by a single HexNAc1 and does not relate to an HexNAc1 which may be included as a component of an oligosaccharide, e.g., Hex9HexNAc2.

As used herein, the term "hexose" refers to a monosaccharide (simple sugar) with six carbon atoms. The chemical formula for hexose is C6H12O6. A hexose can be glucose, mannose, galactose, allose, altrose, gulose, idose, talose and fructose. A hexose refers also to a unit derived from a hexose chosen from glucose (of form L or D), maltose (of form L or D), sucrose, mannose (L or D form), fructose (L or D form), lactose (L or D form), or a mixture thereof; more preferably said compound is glucose (of L or D form).

The oligosaccharides according to the present invention in the form of Hex(n)HexNAc(m), "n" can be 0, 1, 2, 3, 4, 5, 6, 7, 8, 9, 10, 11, 12, 13, 14, 15, 16, 17, 18, 19 or 20. "m" can be 1, 2, 3, 4, 5, 6, 7, 8, 9, or 10. The oligosaccharides can be branched or linear.

In embodiments, the oligosaccharides according to Hex(n)HexNAc(m) can be Hex9HexNAc2, Hex10HexNAc2, Hex11HexNAc2, Hex12HexNAc2, Hex13HexNAc2, Hex14HexNAc2, Hex15HexNAc2 and/or Hex16HexNAc2. In one embodiment, Hex(n)HexNAc(m) is Man(n)HexNAc(m). In embodiments, Hex(n) comprises mannose and other hexose, such galactose, glucose, GIcNac, or Neu5Ac. In embodiments, Hex(n) comprises other hexoses, such galactose, glucose, GIcNac, or Neu5Ac.

In embodiments, HexNAc or HexNAc(m) can be N-Acetylglucosamine (GlcNAc). GlcNAc is an amide derivative of the monosaccharide glucose. In embodiments, HexNAc or HexNAc(m) can be an amide derivative of other hexoses. Non-limiting examples of Hex9HexNAc2 are Man9GlcNAc2, or Glc3Man6GlcNAc2.

### Determination of glycosylation sites and site-specific heterogeneity in glycoproteins

Mass Spectrometry (MS) has emerged as the premier tool for oligosaccharide or glycosylated protein analysis. It provides structural information with high sensitivity. Glycan or glycosylated protein analysis is typically performed with matrix-assisted laser desorption/ionization (MALDI) or electrospray ionization (ESI) resulting in ions that are either sodium coordinated or protonated, respectively, in the positive MS mode and the deprotonated in the negative MS mode. The fragmentation behavior of the two types of ions in tandem MS varies slightly but glycans commonly yield fragment ions resulting from glycosidic bond cleavages.

Peptide mapping is the standard approach to determine site-specific glycosylation and employs a combination of specific enzymatic proteolysis (usually with trypsin), fractionation of glycopeptides (most often by liquid chromatography or affinity chromatography) and glycopeptide analysis by MS. Peptide mapping is a bottom-up approach to protein characterization which enables the profiling of a protein's primary structure as well as its post-translational modifications (PTMs), such as glycosylation.

A common workflow to confirm protein sequence and PTMs is the peptide mapping approach, which combines proteolytic digests with RP-LC-MS/MS analysis in which, e.g., tryptic peptides are separated by reversed phase chromatography and further analyzed using high-resolution tandem mass spectrometry. N-linked glycans are subsequently analyzed in a second experiment comprising enzymatic glycan release, chemical labeling, LC separation on graphitized carbon or HILIC columns and MS analysis. Glycopeptide analysis is typically applied in glycoproteomic experiments to identify the protein as well as the glycan using a specific fragmentation involving a collision energy stepping method. The resulting glycopeptide MS/MS spectra provide both glycan and peptide fragments and allow to assign the peptide sequence as well as the corresponding glycan structure in one spectrum.

An alternative method to overcome several limitations of trypsin digestion was developed using nonspecific proteases. In this approach, pronase is used, which is a mixture of exo- and endo-proteinases capable of cleaving essentially any peptide bond. The glycoprotein is digested save for small peptide 'footprints' around glycosylation sites that are protected from digestion by steric hindrance due to the glycan moiety. The products of the digestion are glycopeptides with short peptides, small (unglycosylated) dipeptides, and amino acids. Purification with solid phase extraction and subsequent MS analysis yields mass spectra that contain only glycopeptides. This method is now used routinely to obtain site heterogeneity for numerous glycoproteins.

Tandem MS can provide peptide and glycan sequence as well as the site of glycosylation. Studies of glycopeptide fragmentation reactions have focused almost exclusively on protonated (either singly via MALDI or multiply via ESI) tryptic glycopeptides. The typical fragments correspond to the loss of the glycan moiety, while the information on peptide sequence and glycan attachment sites is often minimal. Tandem MS is complicated by the sizes of tryptic peptides, which tend to be larger than the useful mass range of collision-induced dissociation (CID), and the labile nature of the glycan-peptide bond. A common strategy, therefore, is to determine the overall mass of the glycopeptide and perform tandem MS to yield the peptide mass.

A skilled person could choose any one of the approaches in the art or described above according to his need in determining the presence, identity and/or position of glycosylations of the rhAAT as described herein.

### FIGURES

The following figures are presented to describe particular embodiments of the invention, without being limiting in scope.

### Brief description of the figures:

Figure 1: An example expression plasmid carrying a PAOX1 promoter
Figure 2: Detailed view of electropherogram overlay of mock strain supernatant with (blue) and without EndoH (red) treatment, against example supernatant with (brown) and without EndoH (green) treatment.
Figure 3: Detailed view of electropherogram overlay of EndoH-treated screening supernatants of muts-strains 1B3 (blue) and 1 F8 (red) secreting rAAT against BSA diluted to 125 mg/L in mock strain matrix (brown).
Figure 4: Electropherogram overlay of supernatant pools from rescreening.
Figure 5: Example chromatogram of the elution part of the affinity chromatography for mut^{s} clone ID 1B6 (reactor S2).
Figure 6: Chromatograms of the SEC for mut^{s} clone ID 1B6 (reactor S2).
Figure 7: SDS-PAGE (left panels) and Western Blot (right panels) analysis of samples from affinity chromatography and SEC of crude bioreactor material from strain mut^{s} clone ID 1B6.
Figure 8: SDS-PAGE (left panel) and Western Blot (right panel) analysis of SEC eluate fractions.
Figure 9: Example chromatogram of the elution part of the affinity chromatography for mut^{s} clone ID 1B6 (reactor B1, B2 and S2).
Figure 10: Chromatogram of the SEC run for mut^{s} clone ID 1B6 (reactor B1, B2 and S2). UV-signal in blue, conductivity in brown. Fractions indicated in red along the x-axis.
Figure 11: SDS-PAGE (left panels) and Western Blot (right panels) analysis of samples from affinity chromatography and SEC of crude bioreactor material from strain mut^{s} clone ID 1B6.
Figure 12: Course of wet cell weight over process time.
Figure 13: Electropherogram overlay of supernatants from selected sampling points and filtrate for cultivation of strain 1B6 in reactor S2.
Figure 14: SDS-PAGE (left panel) and Western Blot (right panel) analysis of cultivation using strain mut^{s} 1B6 in 10 L bioreactor S2.
Figure 15: History plot of cultivation of strain mut^{s} AAT 1B6 in 10L reactor S2 (pH 6.0, 28°C, partial co-feed process slower).
Figure 16: Size exclusion (SE)-HPLC of DS (I).
Figure 17: Size exclusion (SE)-HPLC of DS (II).
Figure 18: Quantification of monomer related peak area of DS autosampler stability at 5±1 °C.
Figure 19: Size exclusion (SE)-HPLC linearity check of DS.
Figure 20: Size exclusion (SE)-HPLC elution profile of monomer related area of DS (III).
Figure 21: Anion exchange-HPLC of DS (I).
Figure 22: Anion exchange-HPLC of DS (II).
Figure 23: Quantification of monomer related peak area of DS autosampler stability at 5±1 °C.
Figure 24: Anion exchange (AEX)-HPLC linearity check of DS.
Figure 25: Anion exchange (AEX)-HPLC elution profile of monomer related area of DS (III).
Figure 26: BSA standard of the Pierce^{™} Rapid Gold BCA Protein Assay Kit.
Figure 27: RecA1AT inhibits neutrophil elastase activity similar to Prolastin^{®}.
Figure 28: RecA1AT inhibits TMPRSS2 activity similar to Prolastin^{®}.
Figure 29: Investigation of the inflammatory parameters in the sputum of post infectious Bronchiolitis obliterans (PiBO) patients.
Figure 30: miRNA expression of PiBO patients compared to controls.
Figure 31: Inflammation and miRNA-expression of human epithelial lung cells (A549).
Figure 32: Experimental design and set up of cell culture systems for human lung epithelial cell (A549)-stimulation.
Figure 33: Analysis of the effects of rhAAT on cellular inflammation and the level of IL-8 in A549 cells stimulated with a cytokine mixture.
Figure 34: Schematic representation of a balanced inflammatory and immune response.
Figure 35: Schematic representation of inflammation and pathologic immune response.

### Detailed description of the figures:

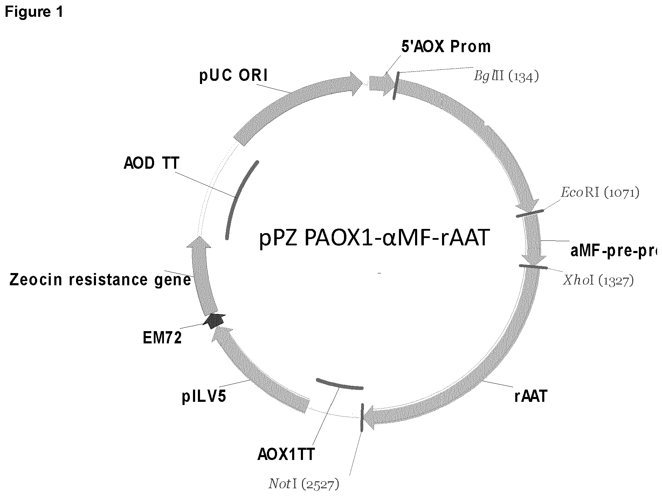

**Figure 1****:** Example plasmid map for rAAT-containing pPZ-PAOX1-aMF-plasmids. 5'AOX Prom represents remainder from original cloning, chromosomal sequence 5' of native PAOX1-sequence; aMF-pre-pro represents alpha-mating-factor (S. cerevisiae), pre-pro-version without EAEA; rAAT is gene sequence of target gene; AOX1TT represents transcription terminator of PpAOX1; pILV5 represents eukaryotic promoter controlling transcription of resistance gene; EM72 represents prokaryotic promoter controlling transcription of resistance gene; Zeocin resistance gene; AOD TT represents transcription terminator of PpAOD; pUC ORI represents origin of replication for E coli, from pUC-plasmid series.

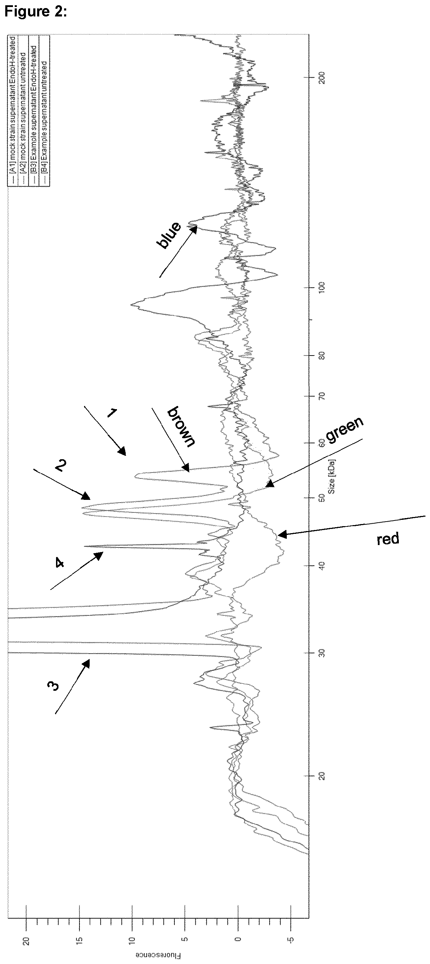

**Figure 2****:** Detailed view of electropherogram overlay of mock strain supernatant with (blue) and without EndoH (red) treatment, against example supernatant with (brown) and without EndoH (green) treatment; Arrow 1 marks putative rAAT-peak generated by de-glycosylation, Arrow 2 indicates rAAT-peak present under both conditions, Arrow 3 shows signal for EndoH (only blue and brown lines) and Arrow 4 depicts analytical artefact caused by air-bubbles in the microfluidic system.

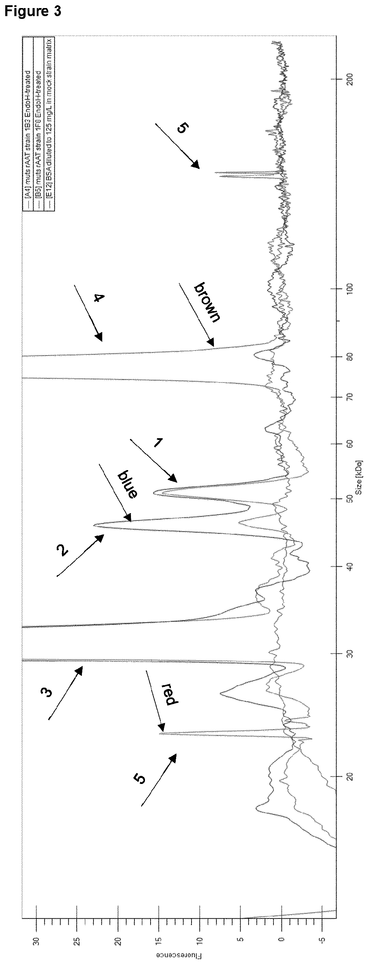

**Figure 3****:** Detailed view of electropherogram overlay of EndoH-treated screening supernatants of mut^{s}-strains 1B3 (blue) and 1F8 (red) secreting rAAT against BSA diluted to 125 mg/L in mock strain matrix (brown); Arrow 1 indicates putative target protein peak for de-glycosylated rAAT, Arrow 2 marks putative signal of non-glycosylated rAAT, Arrow 3 shows peak for EndoH, Arrow 4 highlights signal for BSA and Arrow 5 point at analytical artefacts (caused by air-bubbled in the microfluidic system).

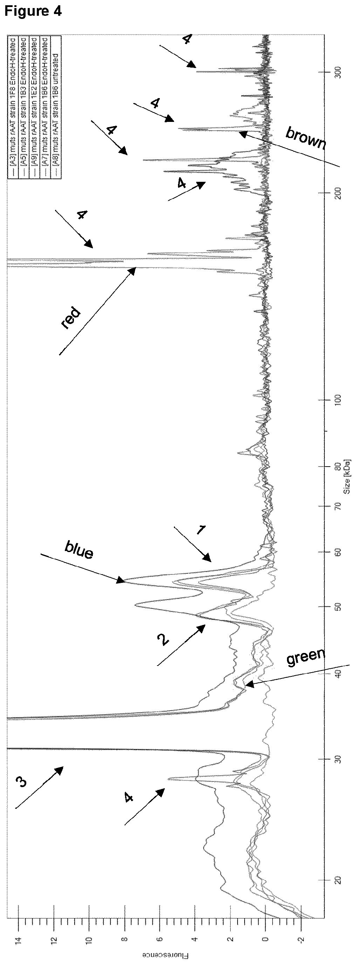

**Figure 4****:** Detailed view of electropherogram overlay of EndoH-treated rescreening supernatant pools of mut^{s} -strains 1F8 (blue), 1B3 (red) and 1E2 (brown) against 1B6 in EndoH-treated (green) versus untreated fashion (pink), all secreting rAAT; Arrow 1 indicates putative target protein peak for de-glycosylated rAAT, Arrow 2 marks putative signal of non-glycosylated rAAT, Arrow 3 shows peak for EndoH and Arrow 4 point at analytical artefacts (caused by air-bubbled in the microfluidic system).

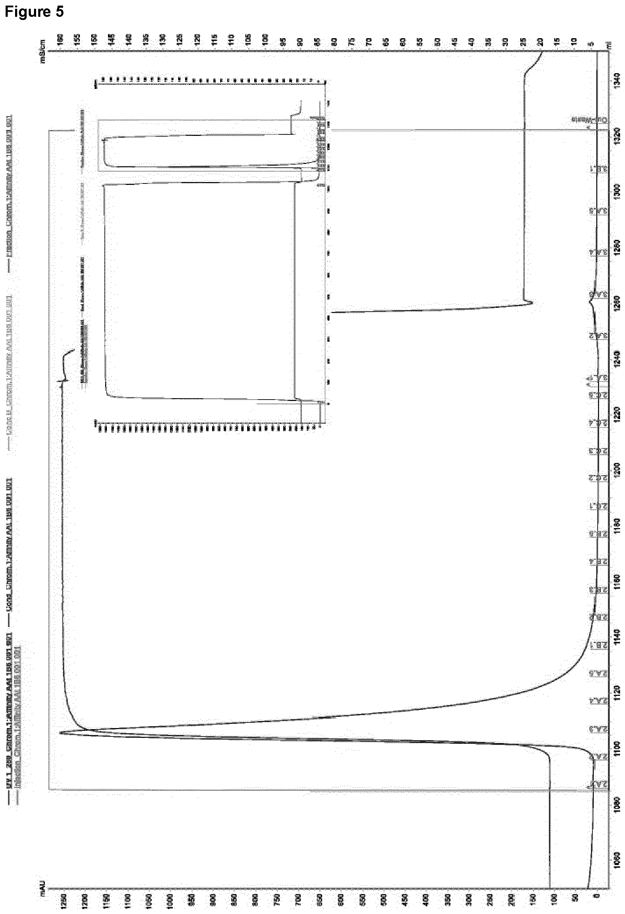

**Figure 5****:** Example chromatogram of the elution part of the affinity chromatography for mut^{s} clone ID 1B6 (reactor S2). UV-signal in blue, conductivity in brown, elution gradient (% of buffer B) in green. Fractions indicated in red along the x-axis. Inset in the upper right corner shows chromatogram of the whole run.

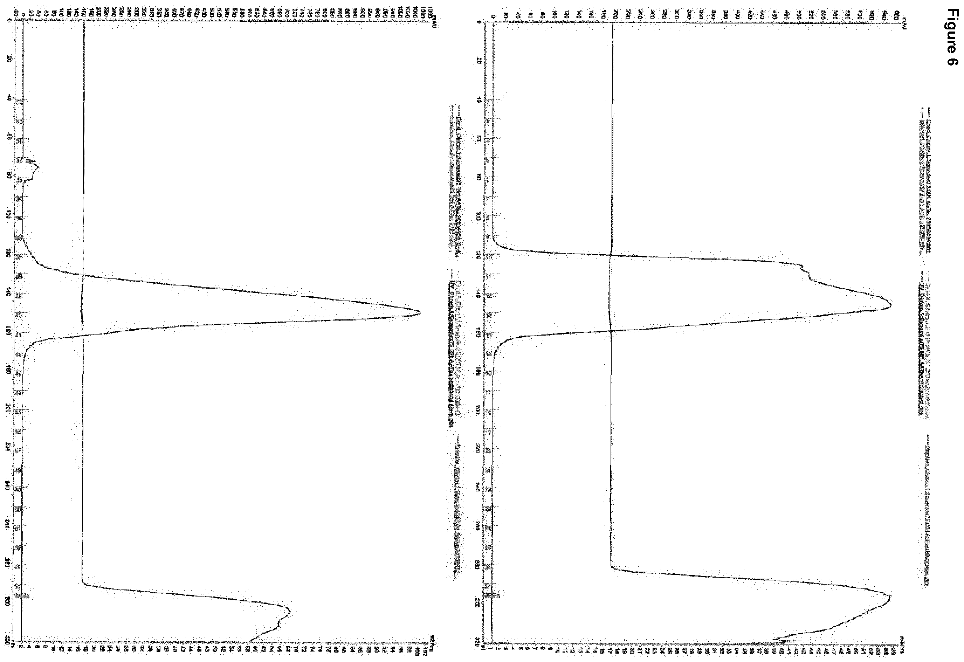

**Figure 6****:** Chromatograms of the SEC for mut^{s} clone ID 1B6 (reactor S2). Upper panel shows first SEC run (eluate pool of affinity run 1 + 2), lower panel shows second SEC run (eluate pool of affinity run 3 + 4). UV-signal in blue, conductivity in brown. Fractions indicated in red along the x-axis.

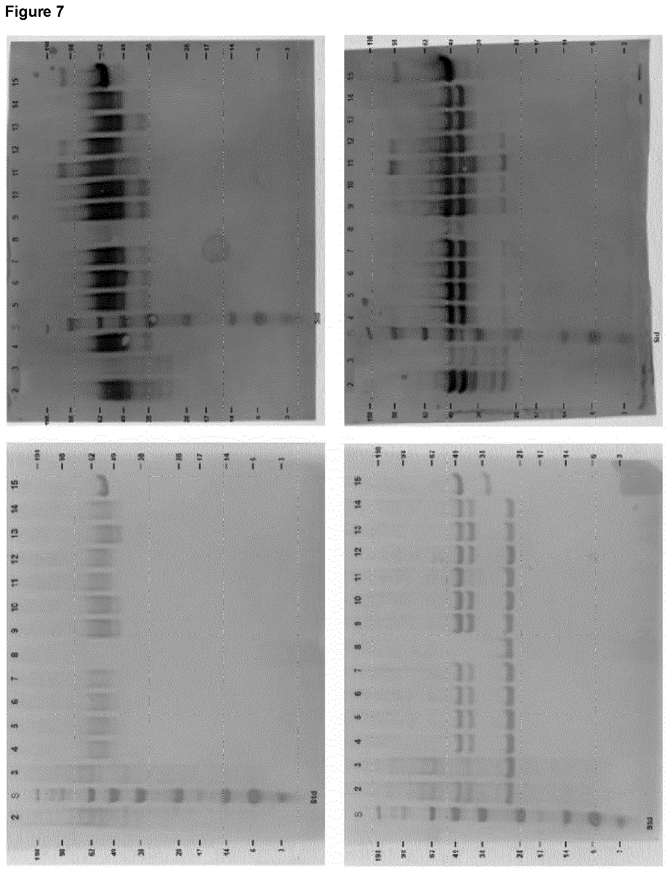

**Figure 7****:** SDS-PAGE (left panels) and Western Blot (right panels) analysis of samples from affinity chromatography and SEC of crude bioreactor material from strain mut^{s} clone ID 1B6. All samples were analyzed without (upper panels) and with (lower panels) addition of EndoH glycosidase. 10 µL sample loaded per lane; MES running buffer.
Lane S MW-Marker
Lane 2 Start sample (3-fold diluted for SDS-PAGE, 30-fold diluted for Western Blot)
Lane 3 Flow Through of affinity chromatography (3-fold diluted for SDS-PAGE, 30-fold diluted for Western Blot)
Lane 4 Capture Step Pool run 1 (40-fold diluted for SDS-PAGE, 400-fold diluted for Western Blot)
Lane 5 Capture Step Pool run 2 (40-fold diluted for SDS-PAGE, 400-fold diluted for Western Blot)
Lane 6 Capture Step Pool run 3 (40-fold diluted for SDS-PAGE, 400-fold diluted for Western Blot)
Lane 7 Capture Step Pool run 4 (40-fold diluted for SDS-PAGE, 400-fold diluted for Western Blot)
Lane 8 Flow Through of spin device (3-fold diluted for SDS-PAGE, 30-fold diluted for Western Blot)
Lane 9 SEC start sample run 1 (150-fold diluted for SDS-PAGE, 1500-fold diluted for Western Blot)
Lane 10 SEC start sample run 2 (150-fold diluted for SDS-PAGE, 1500-fold diluted for Western Blot)
Lane 11 SEC Pool run 1 #9-10 (20-fold diluted for SDS-PAGE, 200-fold diluted for Western Blot)
Lane 12 SEC Pool run 1 #11 (50-fold diluted for SDS-PAGE, 500-fold diluted for Western Blot)
Lane 13 SEC Pool run 1 #12-14 (25-fold diluted for SDS-PAGE, 250-fold diluted for Western Blot)
Lane 14 SEC Pool run 2 (50-fold diluted for SDS-PAGE, 500-fold diluted for Western Blot)
Lane 15 Reference material (1µg for SDS-PAGE, 0.2µg for Western Blot)

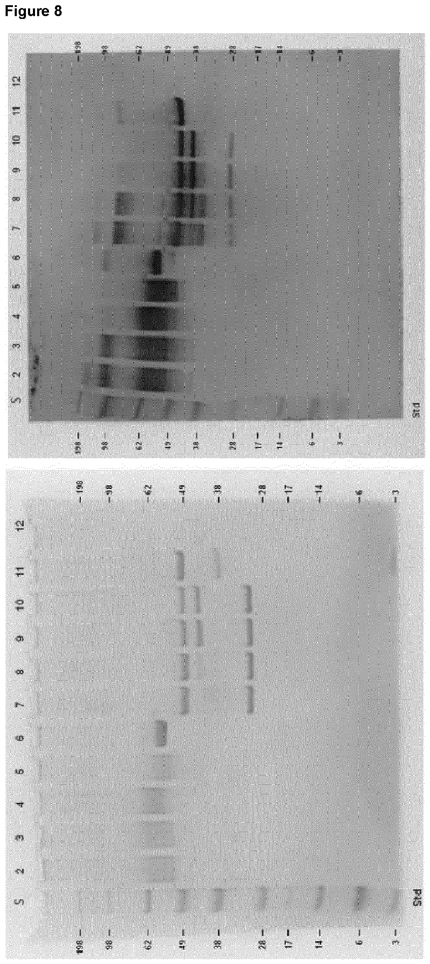

**Figure 8****:** SDS-PAGE (left panel) and Western Blot (right panel) analysis of SEC eluate fractions. All samples analyzed without (lane 2-6) or with (lane 7-12) addition of EndoH glycosidase. 10 µL sample loaded per lane; MES running buffer.
Lane S MW-Marker
Lane 2/7 SEC fraction #9 (15-fold diluted for SDS-PAGE, 150-fold diluted for Western Blot)
Lane 3/8 SEC fraction #10 (25-fold diluted for SDS-PAGE, 250-fold diluted for Western Blot)
Lane 4/9 SEC fraction #11 (20-fold diluted for SDS-PAGE, 200-fold diluted for Western Blot)
Lane 5/10 SEC fraction #12 (10-fold diluted for SDS-PAGE, 100-fold diluted for Western Blot)
Lane 6/11 Reference material (1µg for SDS-PAGE, 0.1µg for Western Blot)

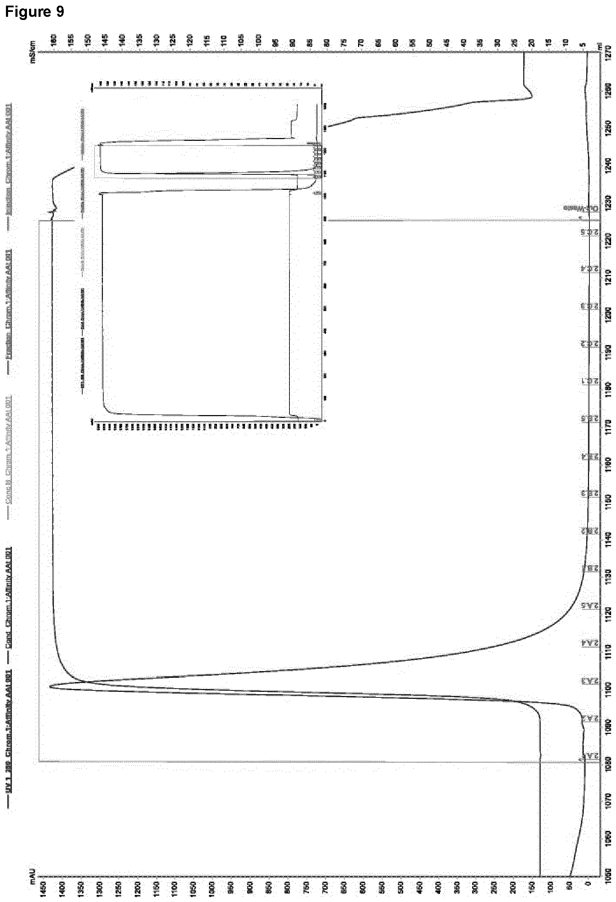

**Figure 9****:** Example chromatogram of the elution part of the affinity chromatography for mut^{s} clone ID 1B6 (reactor B1, B2 and S2). UV-signal in blue, conductivity in brown, elution gradient (% of buffer B) in green. Fractions indicated in red along the x-axis. Inset in the upper right corner shows chromatogram of the whole run.

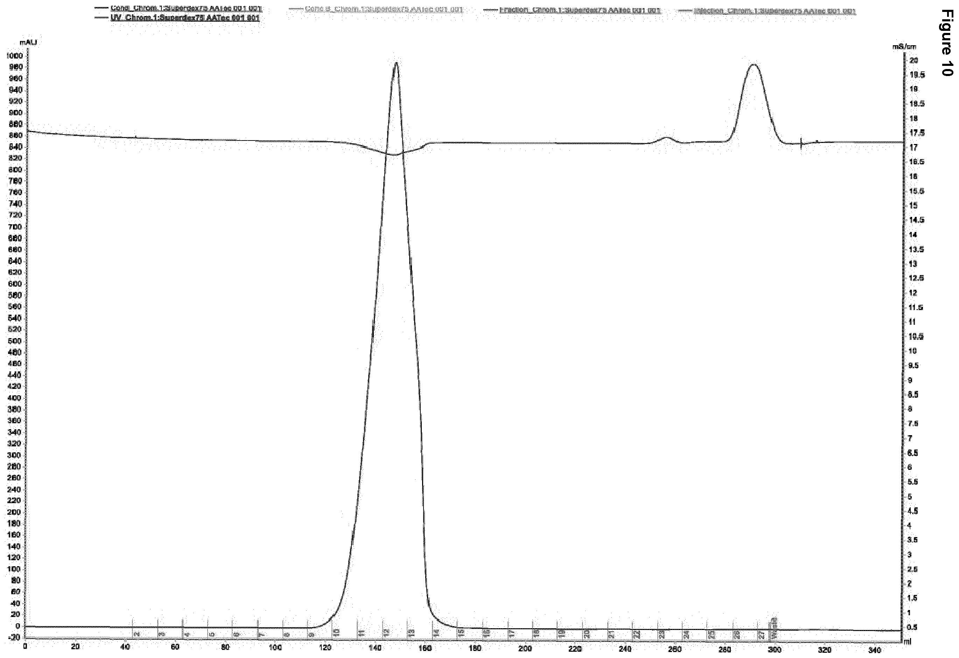

**Figure 10****:** Chromatogram of the SEC run for mut^{s} clone ID 1B6 (reactor B1, B2 and S2). UV-signal in blue, conductivity in brown. Fractions indicated in red along the x-axis.

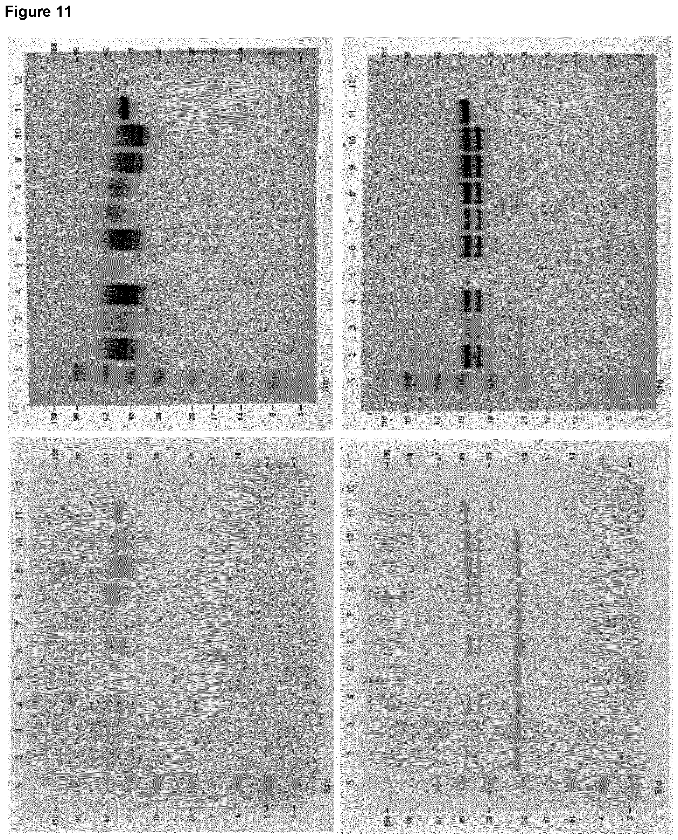

**Figure 11****:** SDS-PAGE (left panels) and Western Blot (right panels) analysis of samples from affinity chromatography and SEC of crude bioreactor material from strain mut^{s} clone ID 1B6. All samples analyzed without (upper panels) or with (lower panels) addition of EndoH glycosidase. 10 µL sample loaded per lane; MES running buffer.

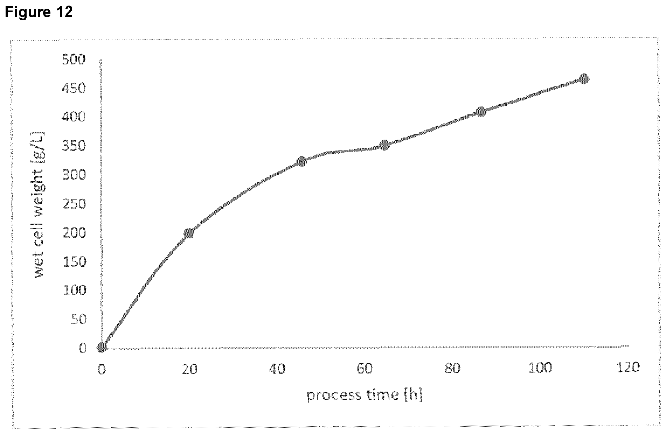

**Figure 12****:** Course of wet cell weight over process time during fermentation.

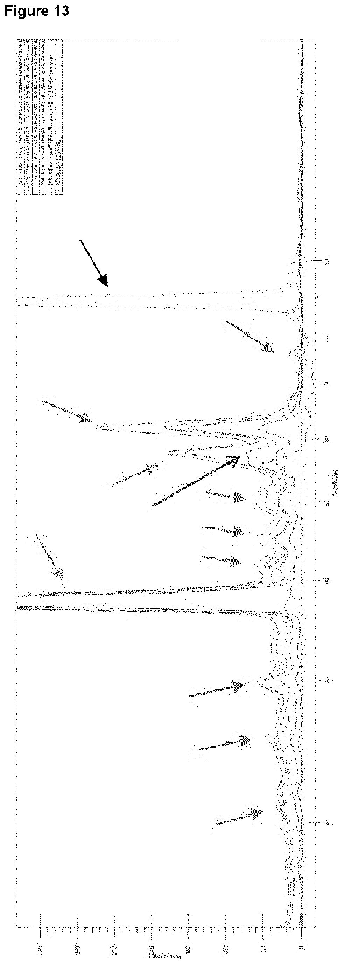

**Figure 13****:** Detailed view of electropherogram overlay of 2-fold diluted, EndoH-treated supernatants from individual sampling points (45 h induced in blue; 67 h induced in red and 90 h induced in brown) against 2-fold diluted filtrate (EndoH-treated in green, untreated in pink) and BSA reference (turquoise); green arrows mark putative signals of de-glycosylated target protein, red arrows indicate main by-products, blue arrow shows signal of EndoH and black arrow points at BSA signal.

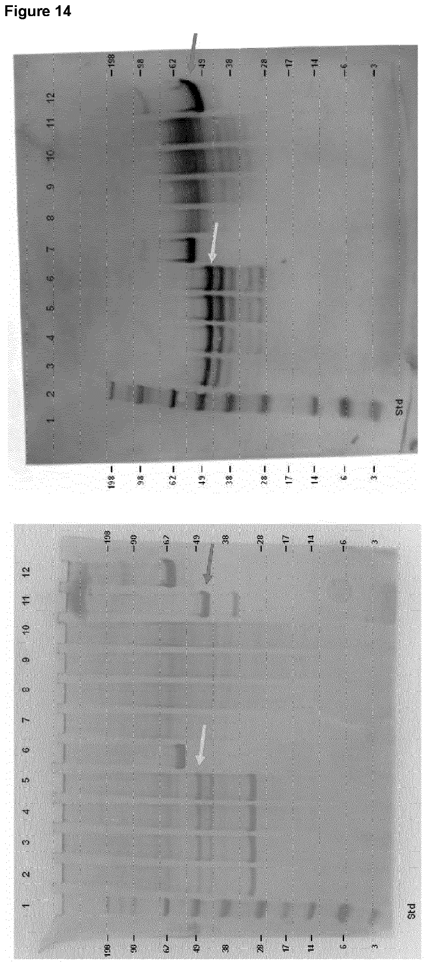

**Figure 14****:** SDS-PAGE (left panel) and Western Blot (right panel) analysis of cultivation using strain mut^{s} 1B6 in 10 L bioreactor S2. Orange arrow marks the band for putative full-length target protein; red arrow indicates the band for de-glycosylated AAT reference material.
Lane 1 (PAGE) 12 (WB) Protein Marker (SeeBlue Plus 2; MW of marker proteins indicated)
Lanes 2-5 (PAGE) / 3-6 (WB) Samples TP3-5 (45h , 67h and 90h induced) and filtrate; EndoH-treated (dil. 8-fold for PAGE, 40-fold for Western Blot)
Lane 6 (PAGE) 17 (WB) AAT Reference, untreated (1 µg loaded)
Lanes 7-10 (PAGE) / 8-11 (WB) Samples TP3-5 (48 , 68h and 91.5h induced) and filtrate; w/o EndoH addition (dil. 8-fold for PAGE, 40-fold for Western Blot)
Lane 11 (PAGE) / 12 (WB) AAT Reference, de-glycosylated (using PNGase F; 1 µg loaded), marked by red arrow
Lane 12 (PAGE only) BSA (1µg)

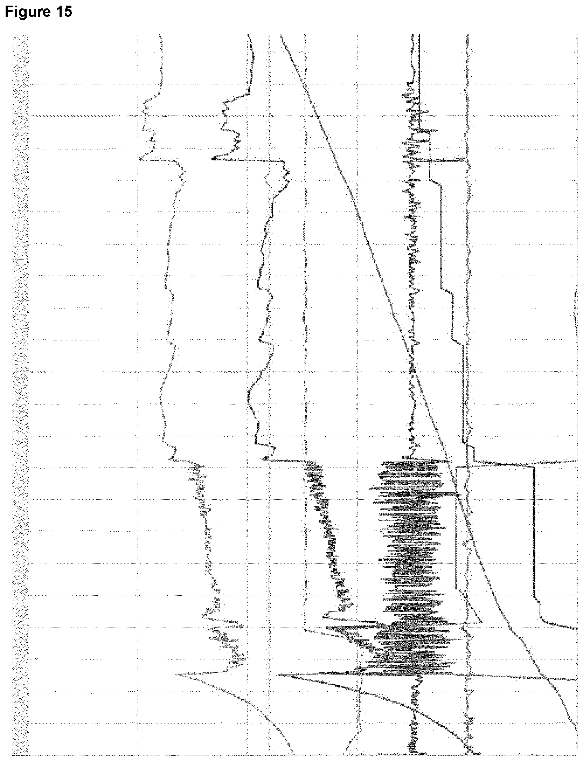

**Figure 15****:** History plot of cultivation of strain mut^{s} AAT 1B6 in 10L reactor S2 (pH 6.0, 28 °C, partial co-feed process slower); Stirrer (red); Pressure (light blue); Dissolved oxygen (dark blue); pH (light green); Base addition (magenta) ; Aeration (orange); Glycerol feed (dark green); Methanol feed (purple); Temperature (yellow); Due to software issue during induction phase, the methanol feed rate was manually adapted to suit the profile defined by the feeding strategy.

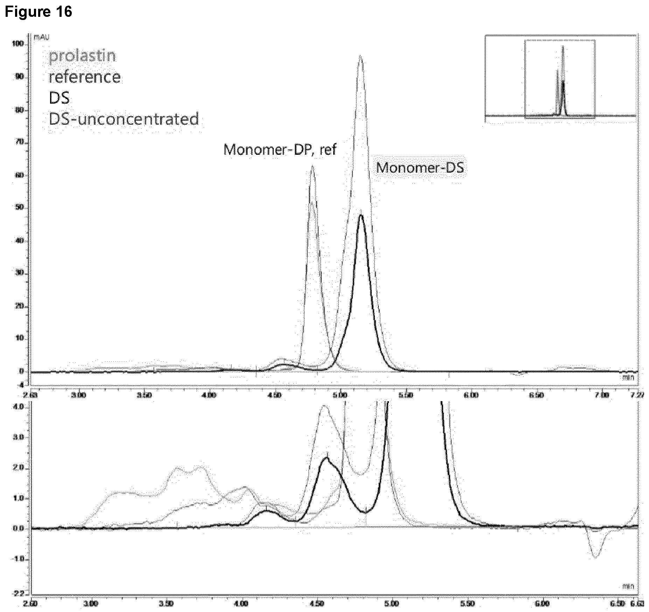

**Figure 16****:** Size exclusion (SE)-HPLC diagram of DS (I). A 10 µL injection volume with 1 mg/mL DS (AATec) was diluted in mobile phase (= 10 µg load). A prominent shift in elution is observed compared to reference and Prolastin.

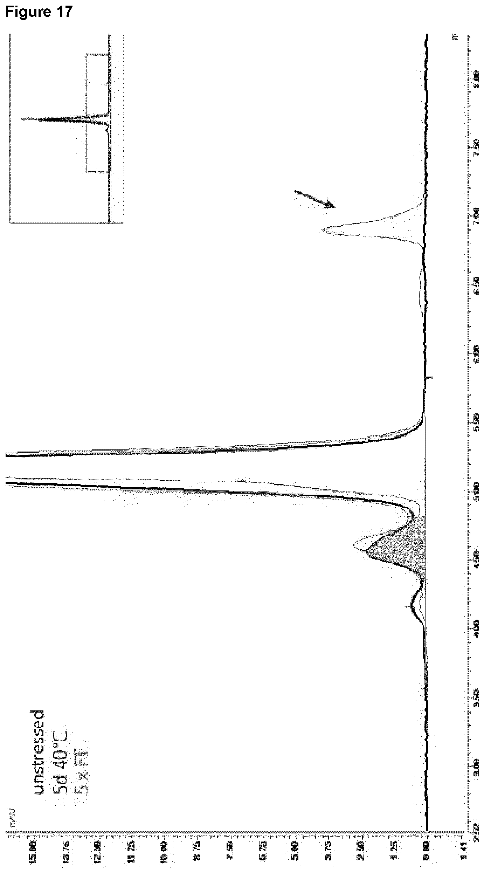

**Figure 17****:** Size exclusion (SE)-HPLC diagram of stressed and unstressed DS samples. The samples were stressed by storage at 40 °C for 5 days and undergoing 5 freeze-thaw cycles, respectively.

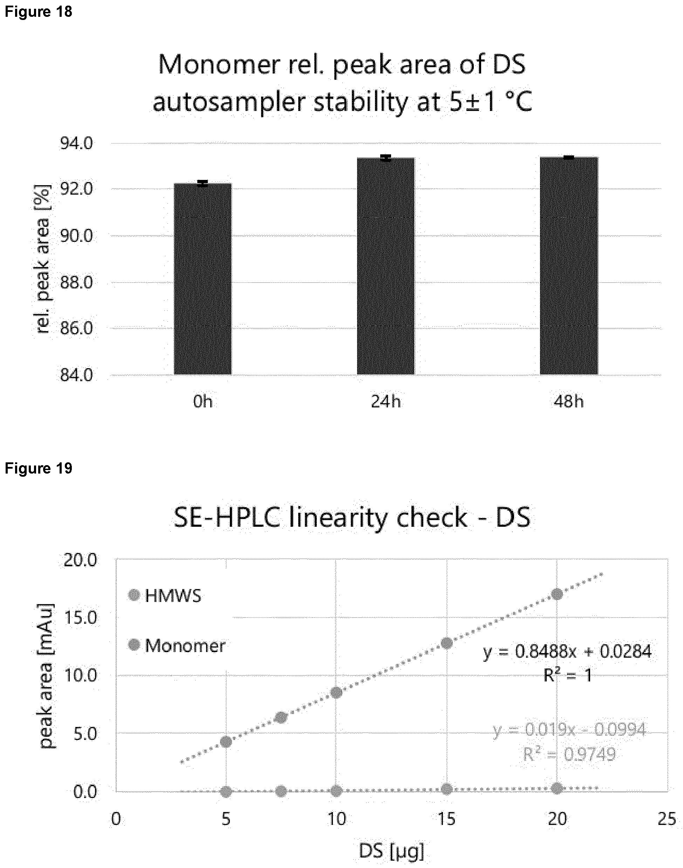

**Figure 18****:** Stability of DS monomer related peak area in autosampler at 5±1 °C. The time points of the HPLC autosampler are 0h, 24h and 48h. The percentage of relative peak areas remains stable at indicated time points.

**Figure 19****:** Linearity check of DS size exclusion (SE)-HPLC linearity check. The linearity of monomer absolute peak area is observed in the range of 5 to 20 µg.

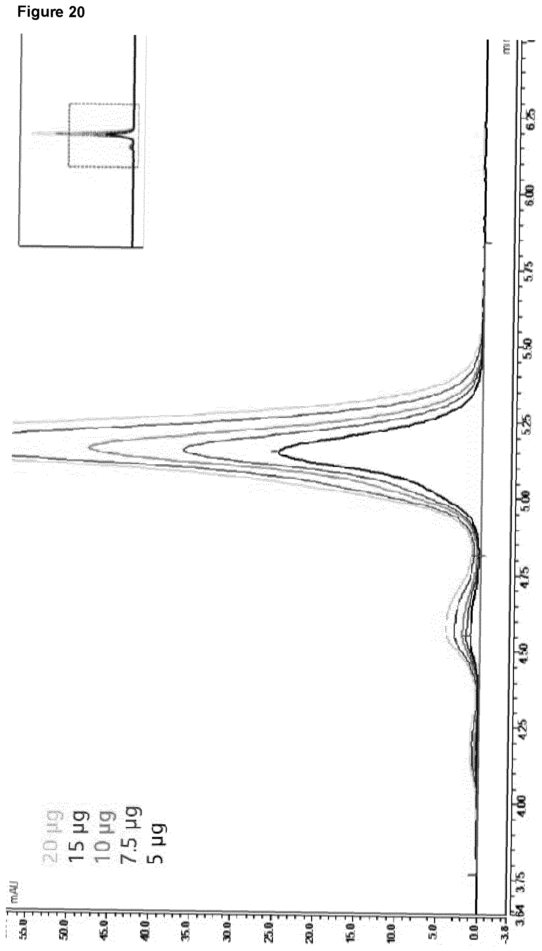

**Figure 20****:** Size exclusion (SE)-HPLC diagram of DS (III) shows monomer absolute peak area of 5, 7.5, 10, 15 and 20 µg, respectively.

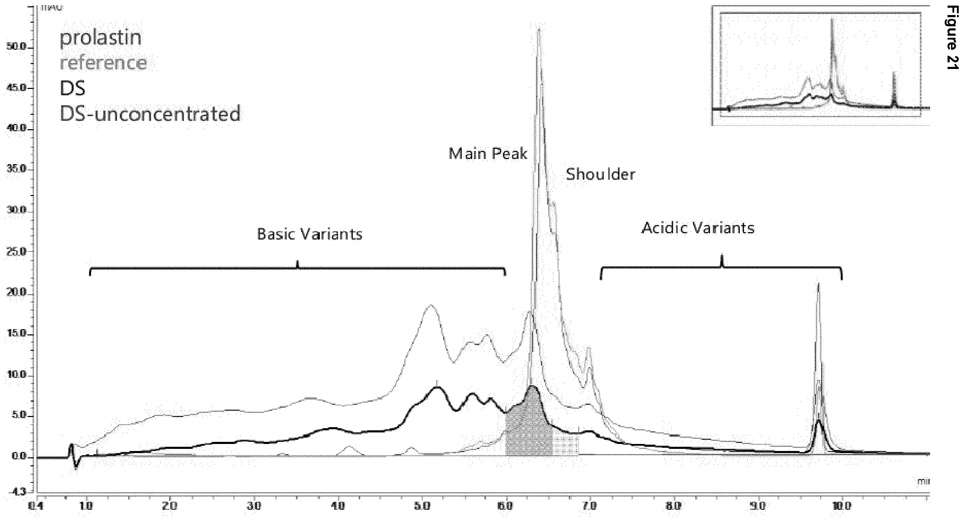

**Figure 21****:** Anion exchange-HPLC diagram of DS (I) shows elution profiles of Prolastin (DP), commercial reference (Ref) and Drug substance of AATec (DS). The elution profiles of DP and Ref exhibit a main peak and shoulder while the elution profile of DS shows a non-homogenous profile.

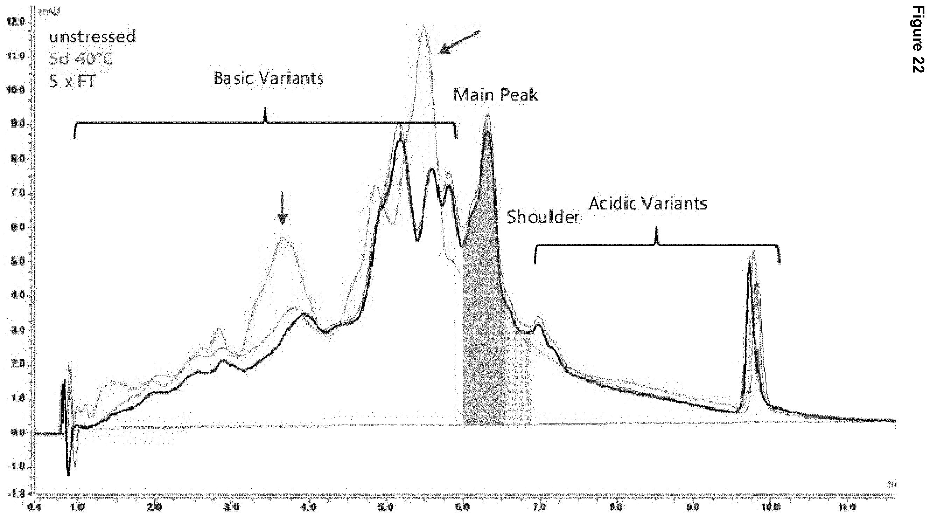

**Figure 22****:** Anion exchange-HPLC of DS (II). The samples were stressed by storage at 40°C for 5 days and 5 freeze-thaw cycles, respectively.

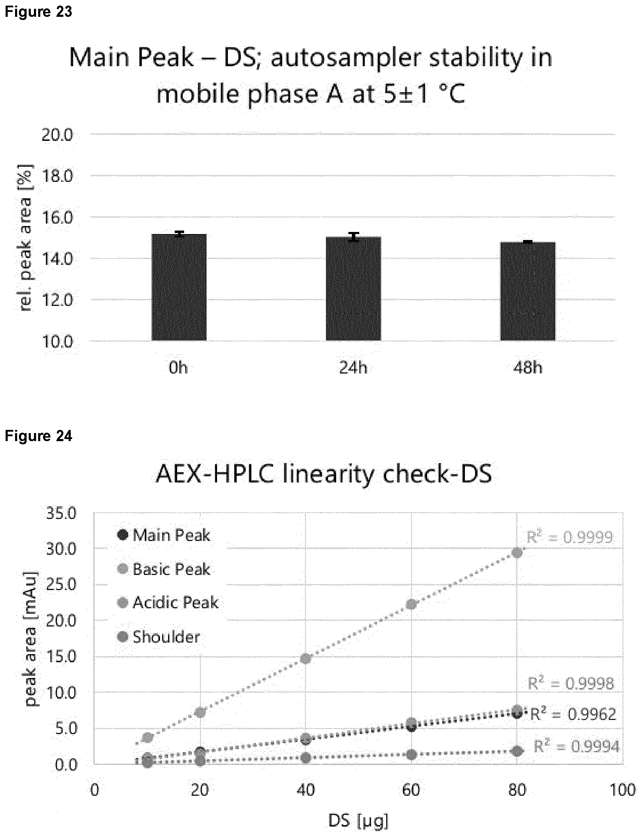

**Figure 23****:** Quantification of monomer related peak area of DS autosampler stability at 5±1 °C. The time points of the HPLC autosampler are 0h, 24h and 48h. The percentage of relative peak areas of the indicated time points is stable.

**Figure 24****:** Linearity check of DS using anion exchange (AEX)-HPLC of DS. The linearity of monomer's absolute peak area is observed in the range of 10 to 80 µg.

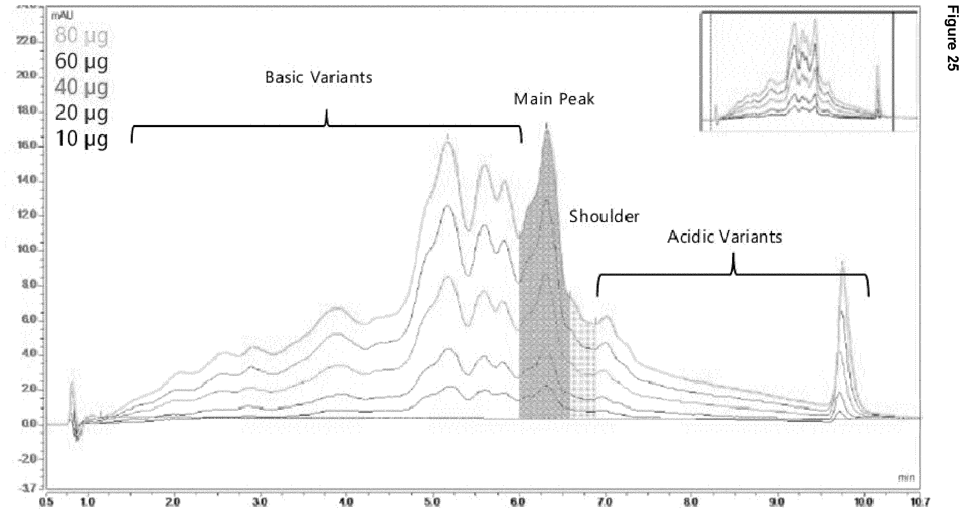

**Figure 25****:** Anion exchange (AEX)-HPLC diagram of DS (III) displays monomer absolute peak area of 10, 20, 40, 60 and 80 µg, respectively.

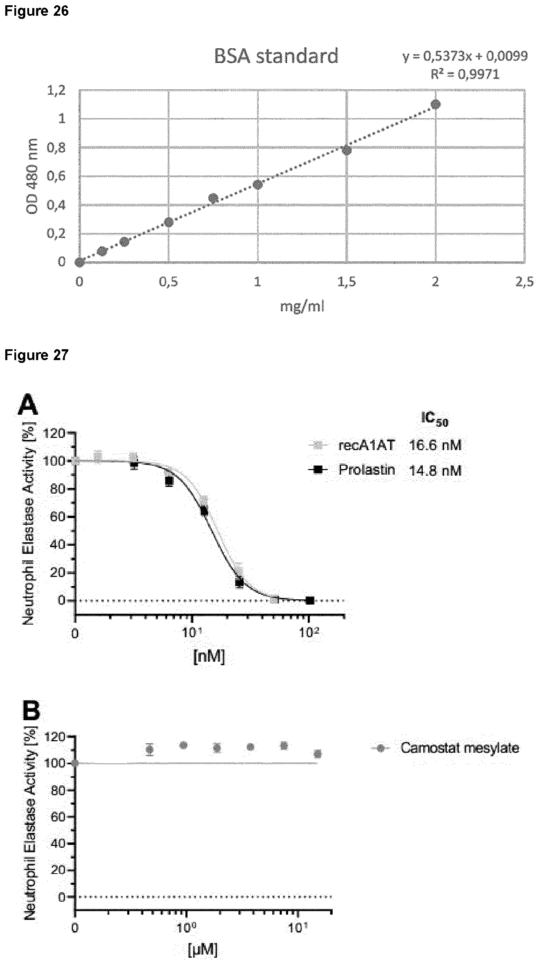

**Figure 26****:** BSA standard of the Pierce^{™} Rapid Gold BCA Protein Assay Kit. Defined dilutions of BSA in TM buffer were prepared according to the Pierce^{™} Rapid Gold BCA Protein Assay Kit to obtain a standard curve.

**Figure 27****:** RecA1AT inhibits neutrophil elastase activity similar to Prolastin^{®}. Serial dilutions of recA1AT, Prolastin^{®} and Camostat mesylate were mixed with human neutrophil elastase before addition of a fluorogenic reporter substrate. Fluorescence intensity was measured at an excitation wavelength of 380 nm and emission wavelength of 460 nm. Shown are mean values ± SEM of three independent experiments, each performed in triplicates. Half maximal inhibition concentrations (IC50) were calculated using a non-linear regression model.

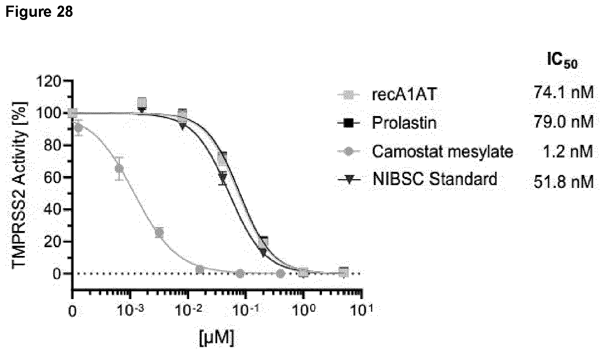

**Figure 28****:** RecA1AT inhibits TMPRSS2 activity similarto Prolastin^{®}. Serial dilutions of recA1AT, Prolastin^{®}, NIBSC standard and Camostat mesylate were mixed with recombinant TMPRSS2 before addition of a fluorogenic reporter substrate. Fluorescence intensity was measured at an excitation wavelength of 380 nm and emission wavelength of 460 nm. Shown are mean values ± SEM of three independent experiments, each performed in triplicates. Half maximal inhibition concentrations (IC50) were calculated using a non-linear regression model.

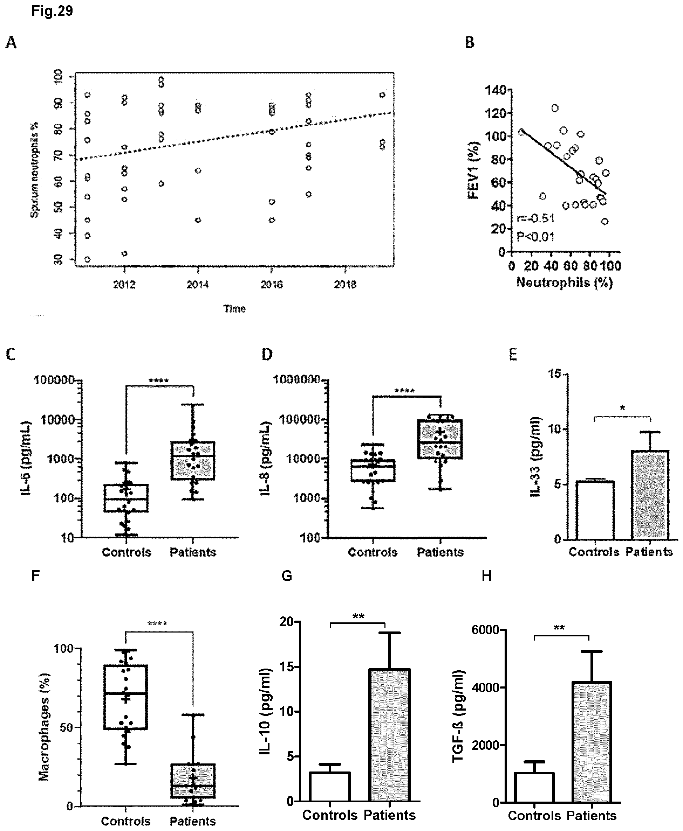

**Figure 29****:** Investigation of the inflammatory parameters in the sputum of post infectious Bronchiolitis obliterans (PiBO) patients. (A) Ongoing neutrophil inflammation correlates with (B) Lung function decline. Increased levels of neutrophil driven (C) IL-6, (D) IL-8 and lung epithelial cell driven (E) IL-33, (G) IL-10 and (H) TGF-β analyzed by Cytometric bead Array (CBA). (F) Analysis of the percentage of macrophages in lung tissue of PiBO patients and healthy controls.

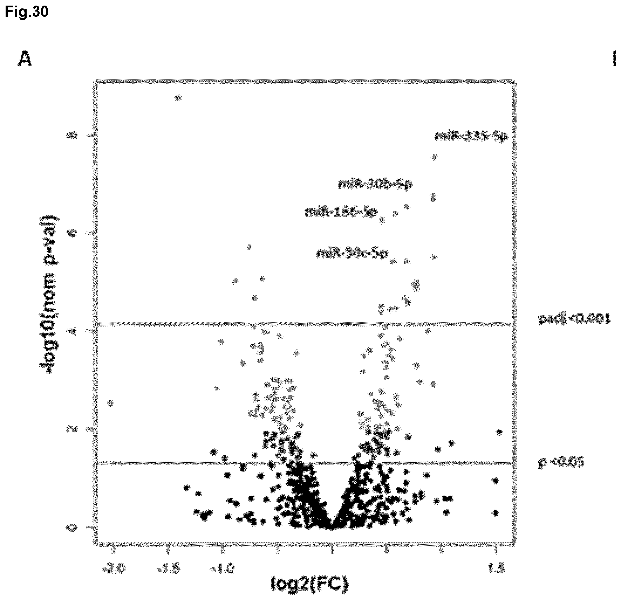

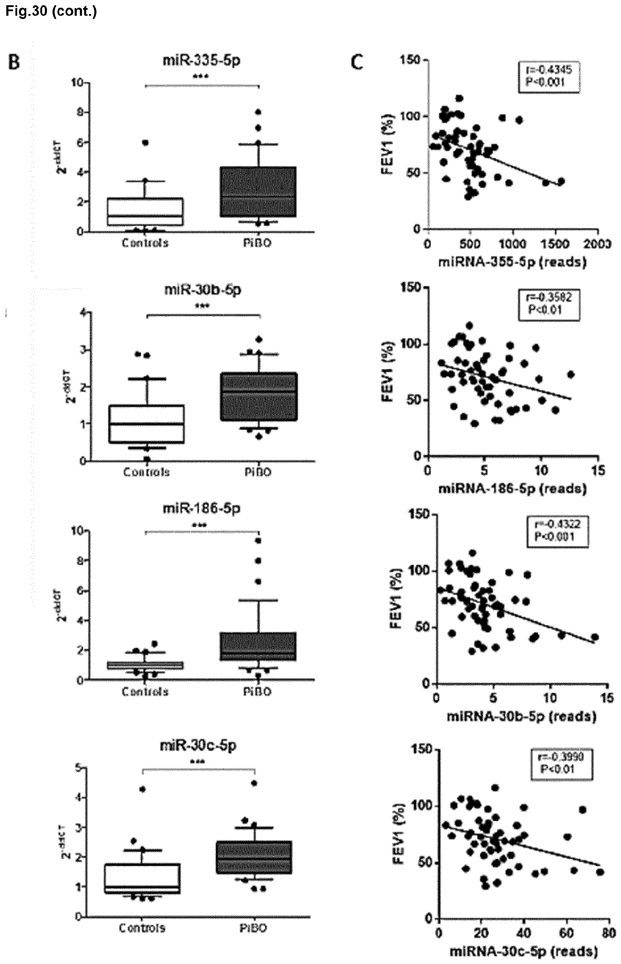

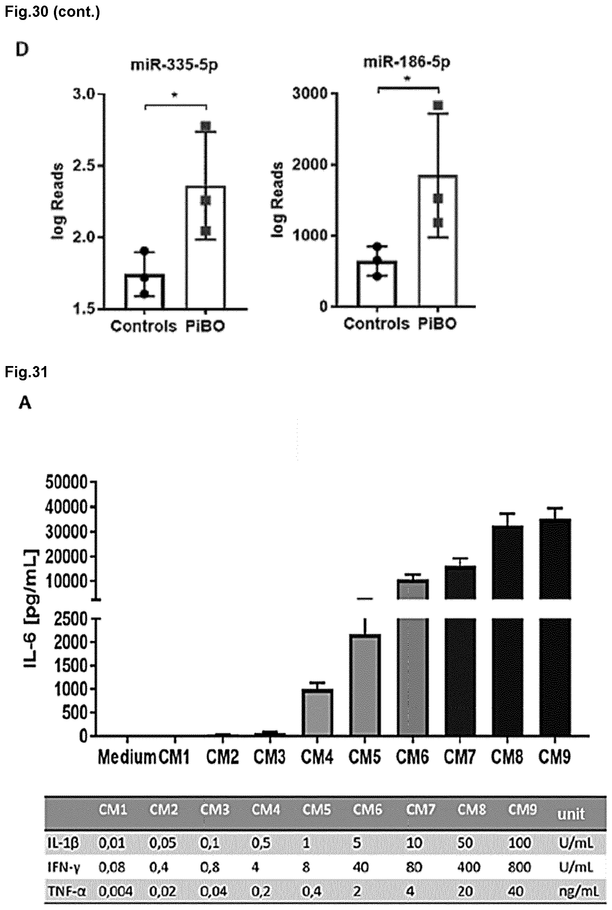

**Figure 30****:** miRNA expression of PiBO patients compared to controls. (A) Volcano plot showing miRNAs which were significant dysregulated at nominal level P < 0.05 and Padj < 0.001 , respectively. (B) Expression levels of miR-335-5p, miR-30b-5p, miR-186-5p and miR-30c-5p confirmed by TaqMan qPCR analysis. (C) Correlations between expression (reads) of verified miRNAs and the lung function parameter FEV1 analyzed in peripheral blood of PiBO patients and healthy controls. (D) NGS of induced sputum samples from PiBO patients compared to control samples (n = 3) showing the reads for miR-335-5p and miR-186-5p.

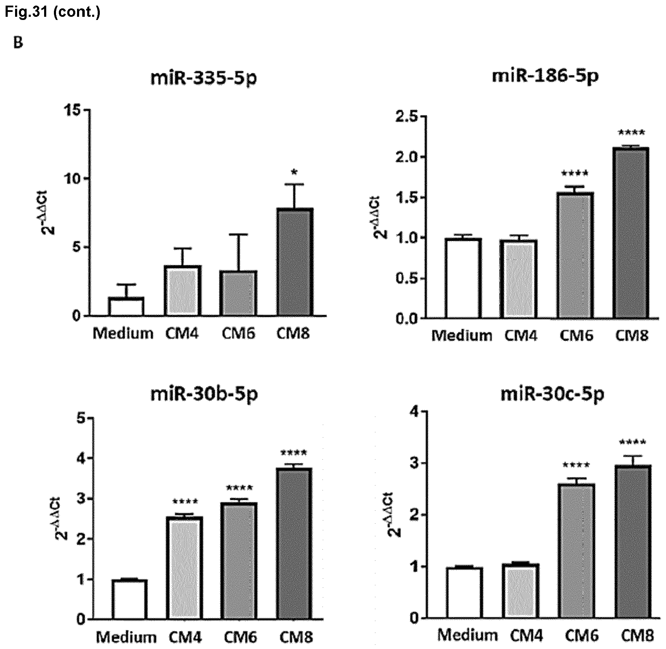

**Figure 31****:** Inflammation and miRNA-expression of human epithelial lung cells (A549). (A) IL-6 Production of A549 cells after stimulation with increasing concentration of the cytokine Mix (CM) containing IL-1β, IFN-βand TNF-α analyzed by Cytometric Bead Array. (B) Expression levels of miR- 335-5p, miR-30b-5p, miR-186-5p and miR-30c-5p after A549 stimulation as described above measured by qPCR.

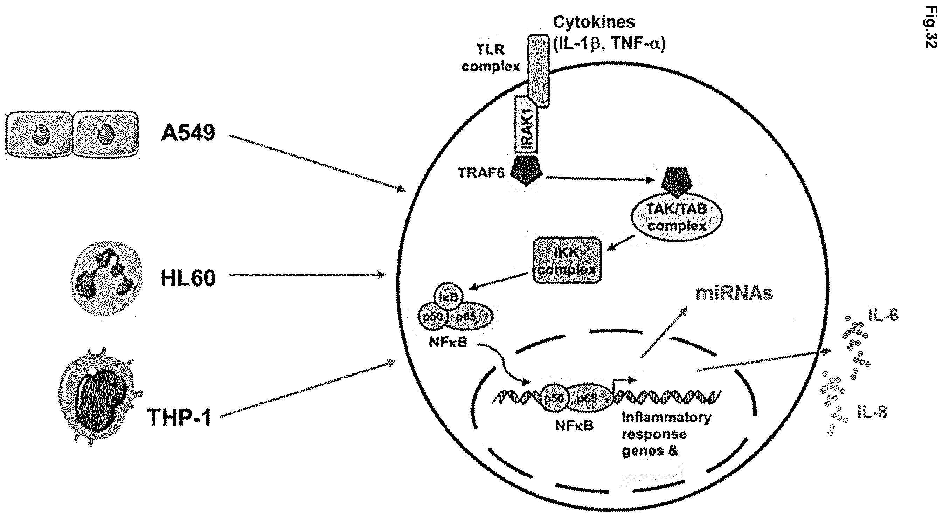

**Figure 32****:** Experimental design and set up of cell culture systems for human lung epithelial cell (A549)-stimulation and optional HL60 and THP-1cells using a cytokine mixture. Cell culture system(s) stimulating A549, (HL60 and THP-1 cells, optional) via a cytokine mixture (CM, IL-1β, IFN-y and TNF-α) to activate the NFKB pathway. Read out of inflammation are IL-6 and IL-8.

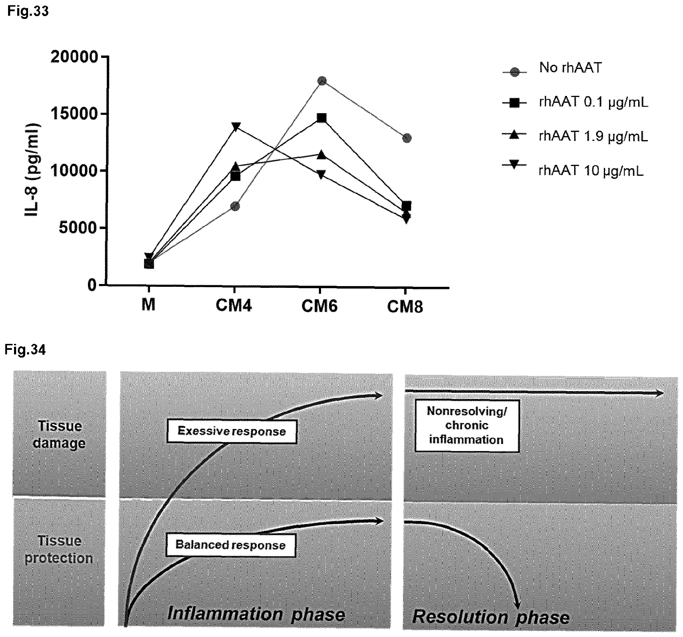

**Figure 33****:** Analysis of the effects of rhAAT on cellular inflammation and the level of IL-8 in A549 cells stimulated with a cytokine mixture. IL-8 Production of A549 cells after stimulation with increasing concentration of the cytokine Mix (CM) containing IL-1β, IFN-βand TNF-α and incubation with different concentration of rhAAT analyzed by Cytometric Bead Array.

**Figure 34****:** Schematic representation of a balanced inflammatory and immune response in comparison to an excessive response leading to non-resolving and/or chronic inflammation. A balanced response to inflammation starts with the inflammation phase followed by a resolution phase in which the inflammation subsides resulting in protection of the inflamed tissue. In contrast within excessive response to inflammation the inflammation phase is not followed by a resolution phase but a non-resolving chronic inflammation.

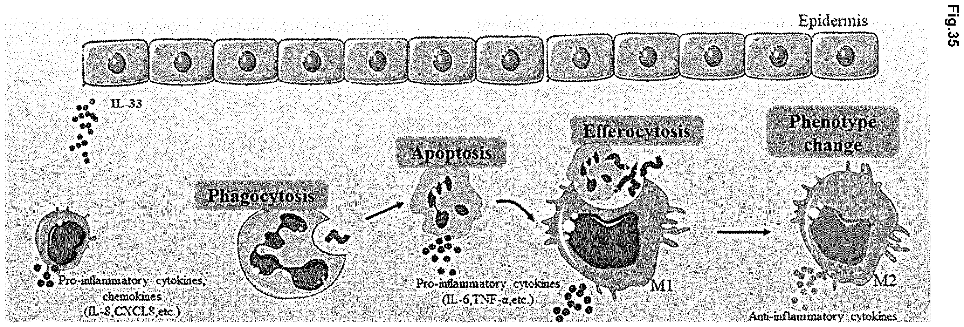

**Figure 35****:** Schematic representation of inflammation and pathologic immune response in lung tissue of BOS patients on the cell level. Proinflammatory mediators involved within PiBOS include IL-33, IL-6, IL-8, CXCL8 and TNF-α.

### EXAMPLES

A Pichia pastoris expression strain for the recombinant production of human alpha-antitrypsin (rhAAT) was developed and validated using the below approaches.

Examples 1-13 show a detailed example workflow for generation of human recombinant AAT. The workflow is composed of transformation of a Pichia pastoris muts strain with a recombinant AAT expression plasmid (Examples 1-4), microscale cultivation (Example 5), Analysis of target protein expression (Example 6), clone rescreening (Examples 7 and 8), purification of AAT from 2L fermentation scale from selected clones (Examples 9 and 10), bioreactor AAT production (up to 10L fermentation scale; Example 11) and analysis of target protein expression (Examples 12 and 13).

Examples 14-19 are directed to a comparative analysis of Prolastin (DP), a commercial reference (Ref) and the present rhAAT preparation (DS), using various chromatography techniques to identify differences in protein preparation from yeast compared to commercial products.

Further functional and structural characterization of the present rhAAT is shown, including measurement of anti-proteolytic activity of recombinant rhAAT (Example 20), and analysis of the rhAAT glycosylation pattern (Example 21).

Examples 22-24 provide experimental assessment of rhAAT in the treatment of acute respiratory distress syndrome (ARDS), Bronchiolitis obliterans (BO) and Asthma.

### Workflow examples for recombinant AAT generation:

### Example 1: Recombinant human alpha-1-antitrypsin:

This Example comprises cloning and *Pichia pastoris* expression strain development for the recombinant production of human alpha-1-antitrypsin (rAAT).

The amino acid sequence of the target protein (SEQ ID NO 4):

EDPQGDAAQKTDTSHHDQDHPTFNKITPNLAEFAFSLYRQLAHQSNSTNIFFSPVSIATAFAMLS
LGTKADTHDEILEGLNFNLTEIPEAQIHEGFQELLRTLNQPDSQLQLTTGNGLFLSEGLKLVDKFL
EDVKKLYHSEAFTVNFGDTEEAKKQINDYVEKGTQGKIVDLVKELDRDTVFALVNYIFFKGKWER
PFEVKDTEEEDFHVDQVTTVKVPMMKRLGMFNIQHCKKLSSWVLLMKYLGNATAIFFLPDEGKL
QHLENELTHDIITKFLENEDRRSASLHLPKLSITGTYDLKSVLGQLGITKVFSNGADLSGVTEEAPL
KLSKAVHKAVLTIDEKGTEAAGAMFLEAIPMSIPPEVKFNKPFVFLMIEQNTKSPLFMGKVVNPTQ
K

Amino acid sequence of target protein rAAT; potentially occupied N-glycosylation sites are underlined and shown in bold.

**Table 1 compiles theoretical molecular weight (without contribution of N-glycans) of the target protein and features.**

| Target protein | Molecular weight [Da] | Features |
|---|---|---|
| rAAT | 44,324.55 | 3x N-glycosylation sites, pl 5.37 |

### Example 2: Workflow in vitro and E. coli:

Upon development and production of an optimized synthetic gene sequence, the target gene was cloned via Xhol/Notl restriction into the expression plasmid pPZ-alpha (Xhol/Notl digested), carrying the alpha-mating factor pre-pro (w/o EAEA) secretion leader (S. cerevisiae), as well as in PAOX1 for methanol-induced production.

**Table 2: Workflow from generating synthetic gene to final expression plasmids:**

| **Step** | **Workflow** | **Result** |
|---|---|---|
| Receipt of synthetic gene in supplier's plasmid | Solubilization of dried plasmid, transformation into *E. coli* | E. coli clone carrying the supplier's plasmid harboring the synthetic gene |
| Restreak of transformant | Restreak of transformant on agarplates for plasmid preparation and VDG-internal culture collection | |
| Plasmid preparation | Gain of plasmid preparation | |
| Restriction digest, agarose gel-electrophoresis | *Xho*I/*Not*I double-digest to separate the synthetic gene from backbone of supplier's plasmid by agarose-gel-electrophoresis | |
| Cut band, elute | Band for synthetic gene was cut from agarose gel, purified and eluted | Double-digested synthetic gene, ready for ligation |
| Ligation into VDG-expression plasmids | Ligation of double-digested synthetic gene into double-digested expression plasmids carrying PAOX1-promoter, transformation into *E. coli* | |
| Restreak of transformants | Restreak of transformants per on agarplates for plasmid preparation | |
| Plasmid preparation | Gain of plasmid preparation | |
| Restriction digest, agarose gel-electrophoresis, analysis, send to sequencing | *Xho*l/*Not*l double-digest to separate the synthetic gene from backbone of expression plasmids by agarose-gel-electrophoresis, check sizes of bands | Agarose gel-electrophoresis to decide which preparation shows correct band sizes is selected for sequencing |
| Sequencing (external) | Sanger sequencing of selected plasmid using primers binding in a way to cover | Verification of in-frame expression cassette "PAOX1-secretion-signal_gene-of-interest_transcription-terminator" |
| Restreak of positive plasmids | Restreak of positive clones on agarplates for larger plasmid preparation (for transformation into *Pichia*) and VDG-internal culture collection | |
| Plasmid preparation and linearization | Gain of plasmid preparation, linearization using *Bgl*II | |
| Desalting, determination of concentration | Desalting over membranes,. determination of DNA-concentration | Ready-to-transform linearized plasmid preparations |

The nucleotide sequence of the inserted target gene (without flanking regions used for cloning purposes) with stop codons (SEQ ID NO 3).

GAGGACCCTCAAGGTGACGCCGCTCAAAAGACTGATACCTCGCACCACGACCAAGACCACC
CAACTTTTAACAAGATTACTCCTAATCTAGCTGAGTTCGCATTCTCTCTCTACAGACAACTCG
CTCATCAGTCTAACTCTACGAACATTTTCTTCTCCCCAGTGTCCATTGCAACTGCTTTCGCCA
TGCTTTCTTTGGGTACTAAGGCTGACACACACGACGAGATTTTAGAAGGATTGAACTTTAATC
TTACCGAAATTCCTGAGGCCCAAATTCACGAAGGTTTTCAGGAGCTGCTTAGAACTCTTAAC
CAGCCAGATAGCCAGCTACAACTTACTACTGGAAATGGACTATTCTTGAGTGAAGGTCTGAA
GCTAGTTGACAAATTCCTCGAAGATGTGAAAAAGTTGTACCATTCTGAGGCTTTCACCGTCAA
CTTCGGTGACACAGAGGAGGCTAAAAAGCAGATCAACGACTACGTTGAAAAGGGTACGCAA
GGTAAAATCGTTGACCTCGTTAAAGAGCTTGATAGAGACACCGTATTTGCTCTTGTCAATTAC
ATCTTCTTTAAAGGAAAGTGGGAGAGACCTTTCGAGGTCAAGGACACCGAGGAGGAAGATTT
TCATGTAGATCAAGTCACCACTGTTAAGGTTCCAATGATGAAGCGTCTCGGTATGTTTAACAT
CCAACACTGTAAAAAGCTATCTTCTTGGGTCCTTCTGATGAAGTACCTGGGTAACGCTACTG
CAATCTTCTTCCTACCAGATGAAGGTAAGCTCCAGCACCTAGAGAACGAACTTACCCACGAC
ATCATTACTAAGTTCCTGGAAAACGAAGACCGTAGATCCGCTTCCCTGCACCTCCCAAAACT
GTCTATCACTGGTACCTATGACCTCAAGTCTGTTCTGGGTCAACTAGGTATAACCAAAGTGTT
TTCAAACGGCGCTGACCTCTCCGGTGTGACTGAAGAGGCACCTCTCAAGCTGTCCAAGGCC
GTTCACAAGGCAGTCCTTACCATTGATGAAAAGGGAACTGAAGCTGCCGGCGCTATGTTCCT
AGAGGCCATCCCAATGTCAATTCCTCCCGAGGTTAAGTTCAACAAGCCATTCGTCTTCCTAA
TGATTGAACAAAACACAAAGTCTCCTCTGTTCATGGGAAAGGTTGTTAACCCAACCCAAAAGT
AATAG

After transformation into E. *coli* TOP10F' cells (NEB-5alpha competent E. coli, C2987l, NEB), recultivation of transformants and plasmid preparations, correct insertion of the target gene was checked by restriction pattern analysis, and authenticity of the particular expression cassette was confirmed by sequencing (LGC Genomics, Berlin, Germany). An example plasmid map is shown in **Figure 1****.**

Large enough quantities of all plasmids were linearized (via *Bgl*II cut) and desalted (MCE membranes, 0.025µM, Millipore VSWP01300) for transformation into *P. pastoris.* Additionally, off-the-shelf Kanamycin/Geneticin-resistance plasmid pPK carrying PAOX1 were prepared for transformation as described above. DNA concentrations were determined by spectrophotometric measurement and adjusted to be roughly 1µg/µL.

### Example 3: Transformation into Pichia pastoris:

All media components used in experiments with *P. pastoris* during growth phases, transformation, regeneration phases (during transformation) and storage of yeast colonies are certified to be animal-free with respect to direct content or direct contact (e.g. animal-source proteinase usage in lysate digest preparation of tryptone / peptone) and possible contamination resulting thereof.

Linearized plasmid constructs are targeted for integration into the host cell genome by homologous recombination of the PAOX1-sequence into the native AOX1-locus, either as a single integrated construct (thereby, only one promoter variant is present, also called "single-copy integration") or in concatemeric form as subsequent integrations in this locus. Alternatively, one or multiple integrations into other loci in the genome can occur by non-homologous integration.

Electroporation was carried out using competent cells of the basic strain CBS7435 Mut^{s} (genotype Δ aox1; phenotype Mut^{s}), applying a modified standard procedure and standard equipment for electroporation.

After regeneration at 28°C, the preparations were plated on agar plates (YPhyD solid) containing the antibiotic Zeocin.

### Example 4: Media for transformation, restreak and cultivation:

### YPhyD liquid medium (1% yeast extract, 2% phytone, 2% dextrose)

### YPhyD solid medium (as above, plus 2% w/v agar, antibiotic supplementation as required)

### BMD liquid (1.34% YNB, 2% dextrose, 0.2M sodium phosphate buffer pH 6.0, 4x 10⁻⁵% Biotine)

**Table 3 compiles media components for solid and liquid media.**

| Ingredient | Supplier | Article # | Lot # |
|---|---|---|---|
| Yeast Extract | BD | 212720 | 8130739 |
| Yeast Nitrogen Base | BD | 291920 | 7109711 |
| Bacto-Agar | BD | 214030 | 2129076 |
| Phytone Peptone | Gibco | 210936 | 3221040 |
| D(+)-Glucose-monohydrate | Roth | 6887.5 | 278273134 |
| Biotine | Sigma-Aldrich | 14400 | BCBJ0991 |
| Sodium dihy phosphate d drogen ihydrate | Roth | 2370.2 | 287260500 |
| di-Sodium hy phosphate d drogen ihydrate | Roth | 4984.3 | 099281291 |

### Microscale fermentation examples and glycosylation analysis:

### Example 5: Microscale cultivation in 96-deep well plates:

For screening, single colonies were picked from transformation plates into single wells of 96-deep well plates filled with optimized cultivation media. Corner wells of some plates were inoculated with the particular mock strain to serve as matrix for analysis.

After an initial growth phase to generate biomass (BMD solid), expression from AOX1-promoter-variant(s) was induced by addition of an optimized liquid mixture containing a defined concentration of methanol. At defined points of time, further induction with methanol was performed.

After a total of 72 hours from the initial methanol induction, all deep well plates were centrifuged and supernatants of all wells were harvested into stock microtiter plates for subsequent analysis.

After selection of particular strains from screening results, these strains were re-streaked onto non-selective agar plates in a manner that yields single, isolated colonies per strain.

Per strain cultivated in rescreening, six of these individual colonies were inoculated into single wells of 96-deep well plates filled with optimized cultivation media, and treated as described above.

**Table 4 Workflow of microscale cultivation.**

| Item | Workflow |
|---|---|
| *Pichia pastoris* host cell line | *Pp*-mut^{s} |
| Transformations | Pool of 2 constructs (dedicated PAOX1-promoter) into *Pp-*mut^{s} |
| Microscale cultivation: screening | *Pp*-mut^{s} with rAAT (limited promoter usage): 92 transformants |
| Microscale cultivation: rescreening | *Pp*-mut^{s} with rAAT (limited promoter usage): 4 candidate strains selected after screening |

The clone selection was conducted as follows:
- Step 1:: Preparation of *Pichia pastoris* host cell line (Pp- Mut^{s})
- Step 2:: Transformation with plasmid
- Step 3:: Microscale cultivation
- Step 4:: Microscale re-cultivation
- Step 5:: Selection of lead clones

### Example 6: Analysis of target protein expression:

### By microfluidic capillary electrophoresis (mCE):

A high throughput screening method involving microfluidic capillary electrophoretic separation (GXII, CaliperLS, now Perkin Elmer) and subsequent identification of the target protein based on size was established. Briefly, several µL of all culture supernatants are fluorescently labelled and analysed according to protein size, using an electrophoretic system based on microfluidics. Internal standards (contained in supplied solutions from supplier) enable approximate allocations to size in kDa and approximate concentrations of detected signals. Bovine serum albumin (BSA) was used as calibrator for apparent molecular weight and concentration after dilution in mock strain matrix to known concentration. Different allocations of proteinaceous peaks with respect to apparent molecular weight (deduced from migration time) may differ between individual analysis plates on the same microfluidic chip, as well as on individual chips, all due to systemic variations. Thereby, deviations of apparent and nominal molecular weight (shifting to higher apparent molecular weight) are usually observed.

### Procedure:

A 5 µL sample (from de-glycosylation, see below) is admixed with 8 µL sample buffer (Perkin Elmer, LDS-containing, pH7.58) containing appropriate amount of reducing agent (then pH7.37), and heated for 5 minutes at 95°C. Subsequently, 32 µL of ddH2O is added, and samples are applied to mCE after centrifugation at 4,000 rpm for 3 minutes (to pellet potential aggregates).

### De-glycosylation:

Some samples were treated with EndoH to analyse the putative glycosylation status of the expressed target protein and provide a preliminary quantification of the amount of the glycosylated target protein. As N-glycosylated proteins tend to produce undefined peaks on mCE due to heterogeneity of the glycosylation, assignment of usually bulgy peaks as well as quantification is difficult. Hence, de-glycosylation yielding more distinct peaks for the target proteins is used to enable specific assignment of target signals and quantification.

For de-glycosylation using EndoH (NEB), 10 µL of supernatant sample were mixed with 10x denaturing buffer and heated to 70°C for 10 minutes. 10x G3-reaction buffer was added, as well as 0.5 µL EndoH for treated conditions or 0.5µL of G3-reaction buffer for untreated condition. After incubation at 37°C for 60 minutes, samples were used for analysis.

### Example 7: Result screening:

### Comparison of target supernatant to mock strain supernatant:

Mock strain supernatant and exemplarily one supernatant of a strain aiming at secreting rAAT were subjected to de-glycosylation conditions with (brown line) and without (green) addition of EndoH (Figure 2).

It appears that a putative non-glycosylated rAAT (marked with arrow 2 in brown and green lines in figure 2) was secreted to the culture supernatant, next to N-glycosylated rAAT that is visualized by de-glycosylation with EndoH (brown line, marked by arrow 1 in **Figure 2**) and not detected in the EndoH-untreated condition (green line).

### Standard setting for comparison of exemplary clones of mut^{s} strains:

A detailed view of an electropherogram overlay of EndoH-treated screening supernatants of mut^{s} strains 1B3 (blue) and 1F8 (red) secreting rAAT against BSA diluted to 125 mg/L in mock strain matrix (brown) is shown in **Figure 3****.** Arrow 1 indicates target protein peak for putative de-glycosylated rAAT, arrow 2 marks signal of putative non-glycosylated rAAT, arrow 3 shows peak for EndoH, arrow 4 highlights signal for BSA and arrows 5 point at analytical artefacts (caused by air-bubbled in the microfluidic system). The ratio of signals for rAAT in putative non-glycosylated and putative de-glycosylated fashion differed somewhat among individual supernatants. The selection of strains for rescreening was based on peak area of the putative de-glycosylated signal.

### Example 8: Result rescreening:

Supernatant pools generated from all six cultivated wells per strain (total of approx. 1.8 mL) were analyzed by mCE after 72 hours of methanol induction under reducing conditions with or without EndoH-treatment against BSA as calibrator (diluted in mock strain matrix to 125 mg/L).

A detailed view of an electropherogram overlay of EndoH-treated rescreening supernatant pools of mut^{s} -strains 1F8 (blue), 1B3 (red) and 1E2 (brown) against 1B6 in EndoH-treated (green) versus untreated fashion (pink) is shown in **Figure 4****.** All clones secret rAAT. Arrow 1 indicates target protein peak for putative de-glycosylated rAAT, arrow 2 marks signal of putative non-glycosylated rAAT, arrow 3 shows peak for EndoH and arrows 4 point at analytical artefacts (caused by air-bubbled in the microfluidic system). When reviewing peak content in green (EndoH-treated) and pink (untreated) lines, it appears that the assignment of one presumable non-glycosylated peak (present in both lines) and one presumable de-glycosylated signal (only present in green line) matched the assumption from screening. The ratio of signals for rAAT in putative non-glycoslyated and putative de-glycosylated forms differed somewhat among individual supernatants. Estimated concentrations of the putative de-glycosylated and non-glycosylated rAAT-fractions found in supernatants is compiled in table 5.

**Table 5: Estimated concentrations of rAAT (as putative de-glycosylated fraction and putatively non-glycosylated fraction) in supernatant pools from rescreening, calculated by comparison of specific peak area with peak area of BSA present in known concentration.**

| Strain | Titer rAAT [mg/L] | |
|---|---|---|
| | Putative Glycosylated | Putative Unglycosylated |
| 1F8 | 10.6 | 8.3 |
| 1B3 | 7.0 | 6.1 |
| 1B6 | 6.9 | 3.7 |
| 1E2 | 5.6 | 4.7 |

One common observation was made: supernatants contained one (presumable) non-glycosylated peak (arrow 2) and one signal present only after de-glycosylation (arrow 1), likely representing an N-glycosylated fraction of the target protein being visualized by de-glycosylation.

Given the potential titer range of around 10 mg/L in supernatant in microscale, a concentration of 150 - 200 mg/L of de-glycosylated rAAT in bioreactor cultivation is feasible.

### Protein purification examples (from 2L fermentation):

### Example 9: First purification campaign of strain mut^{s} clone ID 186:

The aim of this Example was the preparative 2-step purification of rAAT from a crude bioreactor material of strain mut^{s} clone ID 1B6. The target protein was captured by affinity chromatography using a Vantage L column packed with approx. 18 mL of Alpha-1 Antitrypsin Select resin (Cytiva, Product-No. 17547201). As a second step, SEC was performed to polish and formulate the sample to PBS (pH 7.4). In an initial run, 2 L of crude bioreactor material from reactor S2 were used for preparative purification; for a second purification, a mix of crude bioreactor material from reactor B1, B2 and S2 produced in WP4-rep were used.

### Affinity chromatography:

To initially capture the target protein, affinity chromatography was used. Chromatography was performed as recommended by the manufacturer of the resin (Cytiva, Product-No. 17547201). Prior to purification, the column was cleaned with 70% ethanol (recommended by the manufacturer) for 48 hours, washed with water and equilibrated with buffer A.

**Table 6: Chromatography system.**

| | |
|---|---|
| Chromatography System | AKTA Avant 150 |
| Column/Resin | Vantage L column, 11 x 250, packed with approx. 18 mL |
| Buffer A | 20 mM Tris-HCl. 150 mM NaCl, pH 7.4 |
| Buffer B1 | 20 mM Tris-HCl, 2 M MgCl2, pH 7.4 |
| Buffer B2 | 1 x PBS. pH 2.0 |
| Load preparation | Filtered cell-free culture supernatant, 2-fold diluted in buffer A, pH adjusted |

**Table 7: Steps of the affinity chromatography.**

| **Step** | **Buffer** | **Volume** | **Flow rate** | **Comments** |
|---|---|---|---|---|
| Equilibration | Buffer A | 10 CV | 8 mL/min | |
| Sample Load | | 1000 mL* | 4 mL/min | |
| Wash | Buffer A | 4 CV | | |
| Elution | Buffer B1 | 8CV | 8 mL/min | 10 mL fractions collected |
| Wash | Buffer B2 | 4 CV | | |
| Equilibration | Buffer A | 5CV | | |

| | | | | |
|---|---|---|---|---|
| CV: column volume * diluted in buffer A in a 1:1 ratio | | | | |

### Size exclusion chromatography (SEC):

After capture of the target protein, size exclusion chromatography (SEC) was used as a polishing step and to formulate the final sample to PBS pH 7.4.

**Table 8: Chromatography system.**

| | |
|---|---|
| Chromatography System | AKTA Pure |
| Column/Resin | HiLoad 26/600 Superdex 75pg |
| Buffer A | 1x PBS pH 7.4 |
| Load preparation | Concentrated eluate pool of the affinity chromatography |

**Table 9: Steps of SEC.**

| **Step** | **Buffer** | **Volume** | **Flow rate** | **Comments** |
|---|---|---|---|---|
| Equilibration | Buffer A | 1.2 CV | 2.6 mL/min | |
| Sample Load | - | 10-12 mL | | |
| Elution | 100% A | 1.4 CV | | 10mL fractions collected |
| Equilibration | Buffer A | 0.4 CV | | |

First purification campaign using material from reactor S2:

### Affinity chromatography of first campaign:

2 L crude bioreactor material (reactor S2 produced in WP4-rep) was 2-fold diluted in buffer A (i.e. to 4 L) and loaded onto the column in 4 separate runs (1000 mL for each run).

A well-defined peak was observed for all chromatograms (example chromatogram is shown in **Figure 5****)** and main peak fractions (2A2, 2A3, 2A4 for the example chromatogram) were pooled and used for further analysis.

Example chromatogram of the elution part of the affinity chromatography for mut^{s} clone ID 1B6 (reactor S2) is shown in **Figure 5****.** UV-signal in blue, conductivity in brown, elution gradient (% of buffer B) in green. Fractions indicated in red along the x-axis. Inset in the upper right corner shows chromatogram of the whole run.

### Size exclusion chromatography of first campaign:

Eluate pools of the first and second run, as well as of the third and fourth run were pooled and each pool concentrated by ultrafiltration (spin device, 10 kDa cut-off) to approximately 10 mL and loaded onto an SEC column in two separate runs. Column was cleaned with 0.5 M NaOH and equilibrated in buffer before loading the sample.

Chromatograms for both SEC runs are shown in **Figure 6****.** Chromatogram of the second SEC depicted one well-defined peak (like seen in WP10 previously), while the chromatogram of the first SEC run shows a left-handed shoulder peak with less UV signal measured than for the second SEC run. Fractions #37-41 of the second SEC run were pooled for further analysis, while fractions #9-10, #11 and #12-14 were analysed separately to be able to exclude potentially impure fractions. As impurities are visible for fractions #9-11, they were pooled and concentrated to 10 mL and again loaded onto an SEC column to minimize protein loss. The chromatogram shows one well-defined peak and all fractions were analysed separately and stored frozen at - 70°C as backup.

Chromatograms of the SEC for mut^{s} clone ID 1B6 (reactor S2) is shown in **Figure 6****.** The upper panel shows a first SEC run (eluate pool of affinity run 1 + 2), and the lower panel shows a second SEC run (eluate pool of affinity run 3 + 4). UV-signal in blue, conductivity in brown. Fractions indicated in red along the x-axis.

After SEC and final analysis by Western Blot and SDS-PAGE, samples of the first SEC run (eluate fractions #12-14) and second SEC run (eluate fractions #37-41) were pooled, concentrated to 22 mg/mL (by BCA, 8 mL volume) and sterile filtered. Samples were then sterile filtered and stored frozen at -70°C.

### Final analysis and discussion of first campaign:

Samples of the starting material, intermediates and final pool (prior to concentrating to 22 mg/mL) were analysed by SDS-PAGE and Western Blot (**Figure 7**). Protein concentration in the final pool was determined to be 14.4 g/L (BCA), which corresponds to 107.7 mg protein. Endotoxin levels were analyzed by LAL test using Charles River Endosafe PTS and dedicated test chips (sample dilution 1:100). Summarized parameters are listed in table 10.

**Table 10: Summarized parameters of final sample from first purification campaign.**

| Parameter | Analytical method | Unit | Result |
|---|---|---|---|
| Concentration | BCA | mg/mL | 14.4 |
| Volume | | mL | 7.5 |
| Amount | | mg | 107.7 |

SDS-PAGE and Western Blot analysis show that affinity chromatography on AAT-specific resin worked very well to capture the protein from cultures and many bands of a molecular weight between 17 and 40 kDa are cleared. Target protein loss is calculated to 19% (which is surprisingly low considering the degree of purification achieved).

To ensure no protein loss in the concentration step, spin devices with a cut-off of 10 kDa were used to concentrate the capture step eluate pool to 10 mL. No protein loss could be seen in the SDS-PAGE and only a very small band is visible in the Western Blot (which was calculated to 0.5% loss). According to the SDS-PAGE and Western Blot in **Figure 7****,** no target protein is lost during SEC, as the final sample is calculated as 100% of the SEC start sample. However, for the first SEC run impurity bands (potentially dimers / multimers) are visible in earlier fractions (#9-11) and were therefore excluded from further experiments. SEC was therefore a very helpful step to further increase the purity and to formulate the sample to PBS. For the final sample eluate fractions #12-14 (first SEC run) and eluate fractions #37-41 (second SEC run) were pooled and concentrated to 22 mg/mL.

To minimize protein loss, SEC fractions #9-11 (excluded originally, see above) were pooled, concentrated to 10 mL and again loaded onto a SEC column. **Figure 8** shows the analysis of each fraction by Western Blot and SDS-PAGE and reveals that again, the first two fractions (#9 and 10) show impurity bands, while fractions #11 and 12 contain only the target protein. Therefore, fractions #11 and 12 were pooled, sterile filtered and stored frozen at -70°C to serve as a backup sample. Summarized parameters for the final sample of fractions #11 and 12 are listed in table 11.

**Table 11: Summarized parameters of final sample of fractions #11 and 12.**

| Parameter | Analytical method | Unit | Result |
|---|---|---|---|
| Concentration | Western Blot | mg/mL | 13.5 |
| Volume | | mL | 20 |
| Amount | | mg | 270 |

### Example 10: Second purification campaign of strain mut^{s} clone ID 1B6 using pooled material from reactors B1, B2 and S2:

### Affinity chromatography of the second campaign:

To show reliability of the repeat process, a second purification campaign was performed. For the second purification campaign, the remaining amount of 280 mL crude bioreactor reactor material from reactor S2 was mixed with 345 mL from reactor B1 and 345 mL from reactor B2 (note that all three had shown very similar performance). The resulting 970 mL were diluted in buffer A to 2 L and loaded onto the column in two separate runs (1000 mL for each run). A well-defined peak was observed for both chromatograms (example chromatogram is shown in **Figure 9****)** and main peak fractions (2A2, 2A3, 2A4 for the example chromatogram) were pooled and used for further analysis.

### Size exclusion chromatography of the second campaign:

Eluate pools of both runs were pooled and concentrated by ultrafiltration (spin device, 10 kDa cut-off) to approximately 10 mL. The sample was then re-buffered to 1x PBS (pH 7.4) and loaded onto a Sartobind STIC^{®} PA. Eluate of the Sartobind STIC^{®} PA (still >22 EU/mg) was then directly loaded onto a SEC column in one single run. The column was cleaned with 0.5 M NaOH and equilibrated in buffer before loading the sample. A chromatogram of the SEC run is shown in **Figure 10** and exhibited one well-defined peak. Fraction #10-13 were sterile filtered and analyzed by Western Blot and SDS-PAGE. After final analysis by Western Blot and SDS-PAGE showed no impurity bands, samples were pooled, concentrated to 51.7 mg/mL (by BCA, 1.9 mL) and again sterile filtered. Samples were then sterile filtered and stored frozen at -70°C until shipment.

### Final analysis and discussion of the second campaign:

Samples of the starting material, intermediates and final pool (prior to concentrating) were analysed by SDS-PAGE and Western Blot (**Figure 11**). Protein concentration in the final pool was determined to be 51.7 g/L (BCA), which corresponds to 98.2 mg protein. Summarized parameters are listed in table 12.

**Table 12: Summarized parameters of final samples**

| Parameter | Analytical method | Unit | Result |
|---|---|---|---|
| Concentration | BCA | mg/mL | 51.7 |
| Volume | | mL | 1.9 |
| Amount | | mg | 98.2 |

SDS-PAGE and Western Blot analysis show that affinity chromatography on AAT-specific resin worked very well to capture the protein from cultures and many bands of a molecular weight between 17 and 40 kDa are cleared. Target protein loss is calculated to only 13%.

To ensure no protein loss in the concentration step, spin devices with a cut-off of 10 kDa were used to concentrate the capture step eluate pool to 10 mL. No protein loss could be seen neither in the SDS-PAGE nor in the Western Blot. According to the SDS-PAGE and Western Blot in **Figure 11****,** final sample is calculated to 85% yield compared to SEC starting sample. No impurity bands were seen for the four main peak fractions, but it needs to be mentioned that the sample already appeared highly pure after the affinity step. The final sample (concentration of 51.7 mg/mL by BCA) is stored frozen until shipment.

Examples 11-13 are directed to a bioreactor method for secreted production of recombinant AAT in a *Pichia pastoris* strain.

### Bioreactor examples (up to 10L):

### Example 11: Bioreactor cultivation protocol.

### Media preparation:

All media components are certified to be free of contaminants of animal origin. Purified H2O (by reverse osmosis, conductivity of 6.9 µS/cm) was used for all media preparations.

### Pre-culture medium:

**Table 13: pre-culture medium.**

| Component | Concentration | Article No. | Grade | Supplier |
|---|---|---|---|---|
| Phytone-Peptone | 20 g/L | 211906 | n/a | Becton-Dickinson |
| Bacto-Yeast Extract | 10 g/L | 2127¹ | n/a | Becton-Dickinson |
| Glycerol | 20 g/L | 7530.2 | >98% | Roth |

| | | | | |
|---|---|---|---|---|
| ¹2127 is the order number, 10, 20, 30 are the container sizes | | | | |

An appropriate amount of water is distributed among the individual components; all components are heat sterilized separately, allowed to cool down and mixed for use in laminar flow cabinet.

**Table 14: For example, for 1 L of YPG medium.**

| Component | Weight [g] | Water [mL] |
|---|---|---|
| Phytone-Peptone | 20 | 450 |
| Bacto-Yeast Extract | 10 | 450 |
| Glycerol | 20 | 100 |

### Bioreactor media:

**Table 15: Modified Basal salt medium (BSM), listed in order of addition. Each component is added after the previous one has been fully dissolved.**

| Component | Amount per liter | Article No. | Grade [% ] | Supplier |
|---|---|---|---|---|
| Glycerol | 40.0 g | 7530.2 | >98 | Roth |
| Deionized water | 800 mL | n/a | | |
| o-Phosphoric acid 85% | 13.5 mL | 2608.2 | 85 | Roth |
| Citric acid | 4 g | X863.3 | >99.5 | Roth |
| Calcium chloride dihydrate | 0.5 g | HN04.3 | >99 | Roth |
| Sodium chloride | 0.25 g | 9265.1 | >99.8 | Roth |
| Magnesium sulfate | 7.5 g | P027.2 | >99 | Roth |
| Ammonium sulfate | 10 g | 3746.4 | >99.5 | Roth |
| Potassium hydroxide | 2 g | 6751.1 | >85 | Roth |
| Potassium sulfate | 9 g | P022.2 | >99 | Roth |
| Antifoam PPG2000 | 0.1 mL | 297776T | n/a | VWR |
| Fill to 1 L with water and sterilize. | | | | |
| After sterilization and cooling down, add 4.35 mL/L PTM1 using sterile filtration. | | | | |

BSM has a pH-value of 1.8 ± 0.2 and conductivity of 24 µS/cm.

**Table 16: PTM1 Trace Elements, listed in order of addition. Slight turbidity remains present and is normal.**

| Component | Amount per liter | Article No. | Grade [%] | Supplier |
|---|---|---|---|---|
| Deionized water | 800 mL | n/a | | |
| Sulfuric acid (96%) | 5 mL | 971.2 | 96 | Roth |
| Copper(II) sulfate pentahydrate | 6.0 g | P024.1 | >99.5 | Roth |
| Potassium iodide | 0.09 g | 8491.2 | >99 | Roth |
| Manqanese(II) sulfate | 3.0 g | 4487.1 | >99 | Roth |
| Sodium molybdate dihydrate | 0.2 g | 0274.1 | >99.5 | Roth |
| Boric acid | 0.02 g | 1.00165.0100 | >99.8 | Merck |
| Cobalt(II) chloride hexahydrate | 0.92 g | 7095.1 | >99 | Roth |
| Zinc sulfate heptahydrate | 42.2 g | K301.2 | >99.5 | Roth |
| Iron(II) sulfate heptahydrate | 65 g | P015.2 | >99 | Roth |
| Biotine | 0.2 g | 14400-5G | >99,6 | Fluka |
| Fill to 1 L with water | | | | |

PTM1 has a pH-value of 1.6 ± 0.2 and conductivity of 55 µS/cm.

### Other material:

| | |
|---|---|
| Feed-Solution Glycerol: | 60% w/w+ 12 ml/L PTM1 |
| Feed-solution Methanol: | MeOH (>99.85%, CHEMRES) |
| Base: | Ammonia-solution 25% (Art. 5460.3, Roth) |
| Antifoam: | PPG2000 (added automatically using an antifoam probe) |

### Pre-culture for 1L fermenters:

The strain was inoculated from am agar plate using an inoculation loop (with a biomass amount that covers the loop end) into wide-necked, baffled, covered 300 mL shake flasks filled with 50 mL of YPG medium and shaken at 110 rpm (50 mm rotation diameter) at 28°C over night (pre-culture 1). Ideally, OD600 of pre-culture 1 reaches a value of 15-20 after 20-24 hours of growth (depends on the strain and amount of biomass used for inoculation). OD600 is optical density measured at 600 nm, measured using spectrophotometer Genesys 10S VIS (Thermo Scientific) against deionized water as blank. Pre-culture 2 is inoculated from pre-culture 1 in 1 L wide-necked, baffled, covered shake flasks filled with 100 mL YPG medium to OD600 = 2-3 and shaken at 110 rpm (50 mm rotation diameter) at 28°C for 9-10 h. Ideally, OD600 of pre-culture 2 reaches a value of 18-20 after 9-10 hours of growth (doubling time of about 2 h in the exponential growth phase), after which pre-culture 2 material is used to inoculate the bioreactor.

### Pre-culture for 10L fermenters:

The strain was inoculated from an agar plate using an inoculation loop (with a biomass amount that covers the loop end) into wide-necked, baffled, covered 300 mL shake flasks filled with 50 mL of YPG medium and shaken at 110 rpm (50 mm rotation diameter) at 28°C over night (pre-culture 1). Ideally, OD600 of pre-culture 1 reaches a value of 15-20 after 20-24 hours of growth (depends on the strain and amount of biomass used for inoculation). OD600 is optical density measured at 600 nm, measured using spectrophotometer Genesys 10S VIS (Thermo Scientific) against deionized water as blank. Pre-culture 2 is inoculated from pre-culture 1 in 2 L wide-necked, baffled, covered shake flasks filled with 200 mL YPG medium to OD600 = 2-3 and shaken at 110 rpm (50 mm rotation diameter) at 28°C for 9-10 h. Ideally, OD600 of pre-culture 2 reaches a value of 18-20 after 9-10 hours of growth (doubling time of about 2 h in the exponential growth phase), after which pre-culture 2 material is used to inoculate the bioreactor. Three flasks per strain and bioreactor were used in order to obtain sufficient inoculum amount.

Alternatively, one can inoculate the pre-culture medium directly from the glycerol stock: 200 mL of medium in a 2L-baffled (4 baffles) shake flask inoculated with 1 mL stock (prepared to OD600 = 4) and incubated at 110 rpm and 28°C reaches an OD600 = 20 ± 2 after roughly 24-28 hours (still, monitoring of OD600 is advised).

### Bioreactor cultivation conditions:

### Description for bioreactor with 1 L working volume:

A bioreactor is filled with 0.4 L BSM medium, heat sterilized, and pH is corrected to 5 with ammonia solution. Calculated volume of pre-culture 2 is used to inoculate the bioreactor to an OD600 of 2.0. The bioreactor cultivation consists of four phases:
1) Batch growth on glycerol as carbon source
2) Glycerol fed-batch phase to generate additional biomass
3) Transition/induction phase with co-feed of methanol and glycerol
4) Induction phase on methanol as sole carbon source

The temperature was set to 28°C for the entire process. pH was set to 5.0 in the initial batch phase and for the most part of the glycerol fed-batch phase. Using a ramp that starts two hours before initiating the transition phase (18 h in this case) and lasts for 1.5 hours, the pH set point is increased to 6.0 and kept at this level throughout the remaining process.

Oxygen saturation was set to 30% throughout the whole process.

### DO cascade control for reactors with 1L max working volume:

| | |
|---|---|
| stirrer: | 700 to maximum 1200 rpm |
| aeration: | flow range (air) of 1.0 to max. 2.0 L min-1 |
| oxygen supplementation: | up to 60% |

### Description for bioreactor with 10 L working volume:

A bioreactor is filled with 4.5 L BSM medium, heat sterilized (please compensate for any volume loss during sterilization) and pH is corrected to 5 with ammonia solution. Calculated volume of pre-culture 2 is used to inoculate the bioreactor to an OD600 of 2.0. The bioreactor cultivation consists of four phases:
1) Batch growth on glycerol as carbon source
2) Glycerol fed-batch phase to generate additional biomass
3) Transition/induction phase with co-feed of methanol and glycerol
4) Induction phase on methanol as sole carbon source

The temperature was set to 28°C for the entire process. pH was set to 5.0 in the initial batch phase and for the most part of the glycerol fed-batch phase. Using a ramp that starts two hours before initiating the transition phase (18 h in this case) and lasts for 1.5 hours, the pH set point is increased to 6.0 and kept at this level throughout the remaining process.

Oxygen saturation was set to 30% throughout the whole process.

### Dissolved oxygen cascade control for 10 L reactors:

| | |
|---|---|
| stirrer: | between 250 and 1500 rpm |
| aeration: | flow range (air) of 10.0 (start value) - 20.0 L min⁻¹ regulated concomitantly to stirrer increase |
| pressure | 0.3 bar basic level, maximum level 1.5 bar increase after stirrer speed reached 1,000 rpm |

### Feeding strategy:

Table 17 provides the feeding regime of the current fermentation process with methanol induction for 4.5 L of initial batch medium volume (as applied with 10 L bioreactors).

**Table 17: "Partial co-feed protocol slower" feed strategy (for 4.5 L initial batch medium volume in 10 L reactor)**

| **Process time [h]** | **Phase** | **Feed** | **Trigger for initiating next phase** |
|---|---|---|---|
| 0-12 | Batch | | Elapsed time > 12 h |
| 12-20 | Glycerol fed-batch | Glycerol FR=29+3.5*t (g/h) | 344 g of Glycerol 60% added |
| | | [Ramp 29-57 g/h in 8 h] | |
| 20-38 | Transition/induction | Co-feed | 904 g (total) of Glycerol 60% added |
| | | Glycerol FR=21 *+1.125*t (g/h) | |
| | | [Ramp 21-41 g/h in 18 h] | |
| | | MeOH FR= 6+0.375*t (g/h) | |
| | | [Ramp 6-13 g/h in 18 h] | |
| 38-end (≈110 h) | Induction | MeOH FR=19+0.15*t (g/h) | |
| | | [Ramp 19-30 g/h in 75 h] | |

For scale-up or scale-down, feed rates can be adjusted accordingly. It should be noted that glycerol feed is automatically initiated 12 h after bioreactor inoculation, a strategy that is beneficial if the bioreactor is inoculated to OD600 of 2 and if the cells are not limited by oxygen or any other parameter. In the case that the bioreactor is inoculated to OD600 of less than 2 and/or the cells are limited by oxygen or any other parameter, it is recommended to initiate the glycerol feed only after the entire glycerol that is added to the batch medium has been consumed, which is indicated by a sharp increase of dissolved oxygen (DO spike method). Preferably, the required amount of glycerol is added and consumed (DO spike) by the culture before initiating the methanol feed, in order to reduce the risk of overfeeding the cells with methanol feed.

### Sampling:

Samples were taken at indicated time points with the following procedure: the first 2-5 mL of sampled fermentation broth were discarded. 1 mL of the freshly taken sample (2-5 mL) was transferred into a 1.5 mL centrifugation tube and spun for 5 minutes at 13,200 rpm (16,100 g) and 5°C in an Eppendorf 5415 R centrifuge. Supernatants were carefully transferred into separate vials and frozen or directly analysed for the target protein content.

### Determination of cell density (wet cell weight):

1 mL of fermentation broth was centrifuged in a tared Eppendorf vial at 13,200 rpm (16,100 g) for 5 minutes and 5°C in an Eppendorf 5415 R centrifuge, and the resulting supernatant was accurately removed. The vial was weighed (accuracy 0.1 mg), and the tare of the empty vial was subtracted to obtain wet cell weights.

### Harvest and filtration:

After centrifugation of fermentation broth (20 min, 12,000 g, 5 °C, Thermo Scientific Sorvall Bios A centrifuge with Rotor F6-10x1000 LEX), the supernatant was recovered and filtered over a filter capsule Sartopure PP3 0.45 µm (Sartorius # 5051306P4-OO-B) (for supernatant from 1L reactors) or Sartopure PP3 Midicaps 0.45 µm (Sartorius #5055306P7-SO-A) (for supernatant from 10L reactors). Suitable aliquots are prepared and frozen at -70 °C.

### Example 12: Analysis of target protein expression:

### Microfluidic capillary electrophoresis (mCE):

Microfluidic capillary electrophoretic separation (GXII, CaliperLS, now Perkin Elmer) and subsequent identification of the target protein based on its size is used. Briefly, several µL of all culture supernatants are fluorescently labelled and analysed according to protein size, using an electrophoretic system based on microfluidics. Internal standards enable approximate allocations to size in kDa and approximate concentrations of detected signals.

Different allocations of proteinaceous peaks with respect to apparent molecular weight (deduced from migration time) may differ between individual analysis plates on the same microfluidic chip, as well as on individual chips, all due to systemic variations.

Reference material for bovine serum albumin (BSA) was used as calibrator (diluted to 125 mg/L).

### Procedure:

5 µL sample (from de-glycosylation, see below) is admixed with 8 µL sample buffer (Perkin Elmer, LDS-containing, pH7.58) containing appropriate amount of reducing agent (then pH7.37), and heated for 5 minutes at 95°C. Subsequently, 32 µL of ddH2O is added, and samples are applied to mCE after centrifugation at 4,000 rpm for 3 minutes (to pellet potential aggregates).

### De-glycosylation:

For de-glycosylation using EndoH (NEB), 10 µL of diluted supernatant sample were mixed with 10x denaturing buffer and heated to 70°C for 10 minutes. 10x G3-reaction buffer was added, as well as 0.7 µL EndoH. After incubation at 37°C for 60 minutes, samples were used for analysis.

### SDS-PAGE and Western Blot:

For SDS-PAGE, diluted samples were de-glycosylated as above or used non-deglycosylated (treated in the same way but without addition of EndoH) and mixed with 4 x LDS sample buffer and 10 x Novex reducing agent (both Thermo Scientific) and incubated at 70°C for 10 min. Samples were then loaded on a Bolt Bis-Tris 4-12% Gel and run with MES buffer. SeeBlue Plus2 Pre-Stained Standard was included as a molecular weight marker (all Thermo Scientific).

For Coomassie staining, gels were washed in water, stained using SimplyBlue SafeStain (Thermo Scientific) and de-stained in water according to the manufacturer's protocol.

For Western Blot, protein transfer from a pre-run gel was performed by dry blotting using the iBlot 2 Gel Transfer Device with dedicated Novex PVDF transfer stacks. The membrane was subsequently saturated with PBS, 0.1% Tween-20, 5% BSA for 30 min at RT with gentle shaking.

For AAT-specific detection the membrane was incubated for 30 min with gentle shaking at RT using the primary AAT-Antibody (SIGMA, SAB4200196-200uL) diluted 1:750 in PBS, 0.1% Tween-20, 3% BSA. After washing the membrane three times (5sec, 5min and 10min with PBS-0.1% Tween20 under vigorous agitation at ~200 rpm), the secondary antibody (abcam anti mouse IgG H&L (HRP) ab2057-19; 5,000-fold diluted in PBS, 0.1% Tween-20, 1% BSA) was added for 20 min. The membranes were then washed again and color was developed by adding TMB ultra substrate (Thermo Scientific). For all gels and blots, samples were diluted 5-fold more for Western Blot than for Coomassie stained gels.

### Example 13: Results of bioreactor cultivation of non-glyco-engineered straind Mut^{s} 1B6 in 10L-scale:

### Overview:

Estimated product titre and cell density over the process and induction times are shown in table 17 and **Figure 12****.**

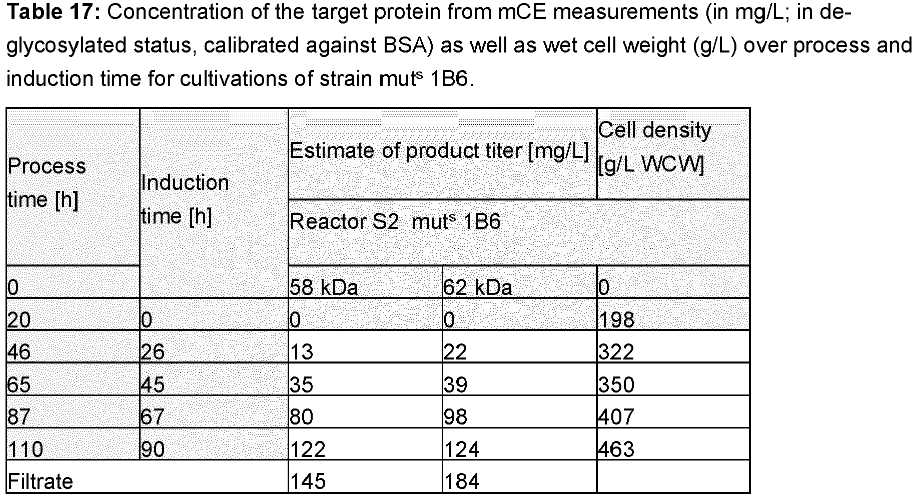

This calculation is made based on the peak at ~58 kDa apparent molecular weight in mCE as well as the peak at ~62 kDa apparent molecular weight (see Figures below).

As shown in **Figure 12****,** Strain mut^{s} 1 B6 grew well to cell density of 400-500 g/L.

**Figure 13** shows a detailed view of electropherogram overlay of 2-fold diluted, EndoH-treated supernatants from individual sampling points (45 h induced in blue; 67 h induced in red and 90 h induced in brown) against 2-fold diluted filtrate (EndoH-treated in green, untreated in pink) and BSA reference (turquoise); green arrows mark putative signals of de-glycosylated target protein, red arrows indicate main by-products, light blue arrow shows signal of EndoH and black arrow points at BSA signal. A double-peaked pattern was observed for the putative target protein under de-glycosylated conditions. Note that the left peak (migrating to lower apparent molecular weight, approx. 58 kDa) is also present in untreated condition, although at lower abundance.

**Figure 14** compares samples from the last 3 sampling points and final filtrate, with and without de-glycosylation, on SDS-PAGE and Western Blot.

**Figure 15** shows a history plot of cultivation of strain mut^{s} AAT 1B6 in 10L reactor S2 (pH 6.0, 28 °C, partial co-feed process).

Table 18 compiles the concentration of the full-length target protein estimated from SDS-PAGE by densitometric analysis of 48 kDa band signal against AAT reference (orange and red arrows in figure 3).

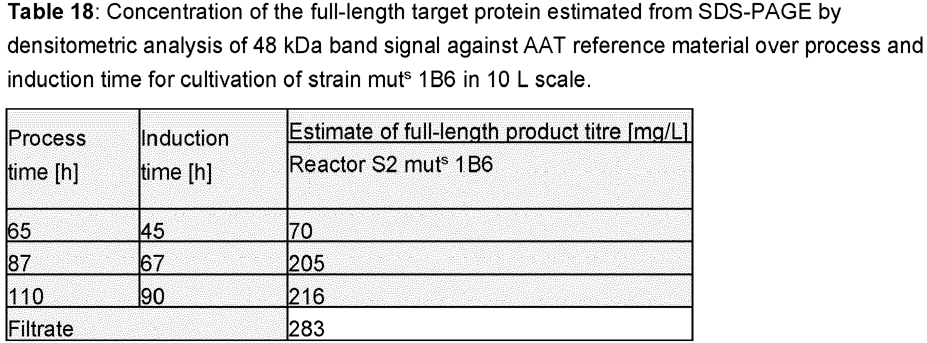

### Examples relating to analytical comparisons of rhAAT with Prolastin and a commercial reference:

Examples 14-19 are directed to comparative analysis of Prolastin (DP), a commercial reference (Ref) and the present rhAAT preparation (DS).

### Example 14: Size exclusion (SE)- HPLC analysis of DP, Ref and DS (I)

A 10 µL injection volume with 1 mg/mL DS (AATec) was diluted in the mobile phase resulting in a 10-µg load. **Figure 16** illustrates a significant shift in the elution of DS compared to the reference and Prolastin indicating potential variations in post-translational modifications. To validate this observation, unconcentrated DS (thawed and directly injected) was used as a control, which also exhibited a shifted elution time. This finding suggests that the shift is not caused by up-concentration. Additionally, two distinct high molecular weight species (HMWS) were detected as presented in Table 19.

**Table 19: Two distinct HMWS Species**

| | **Monomer** | **HMWS** | **LMWS** |
|---|---|---|---|
| DP | 78.5 % | 20.6 % | 0.9 % |
| Ref | 89.2 % | 9.9 % | 0.9 % |
| DS | 92.2 % | 7.8 % | NA |
| DS-unconcentrated | 94.2 % | 5.8 % | NA |

### Example 15: Size exclusion (SE)- HPLC analysis of stressed and unstressed DS samples

The sample was subjected to stress by storing it at 40 °C for 5 days and undergoing 5 freeze-thaw cycles, respectively. As depicted in **Figure 17****,** a new LMWS peak emerged after 40°C stress indicating fragmentation due to stress. However, it can be concluded that freeze-thaw stress does not have a significant effect on the size distribution. Table 20 provides information on the fragment distribution of unstressed and stressed samples.

**Table 20: Fragment distribution of unstressed and stressed samples**

| | | | |
|---|---|---|---|
| | unstressed | 5d 40°C | 5xFT |
| Monomer | 92.2 % | 86.4 % | 92.7 % |
| HMWS | 7.8 % | 6.3 % | 7.3 % |
| LMWS | NA | 7.3 % | NA |

### Example 16_{:} Size exclusion (SE)- HPLC analysis of monomer related peak area stability

DS sample was kept at 5 ± 1°C in the autosampler for a minimum of 48 h before analysis. **Figures 18-20** illustrate linearity of monomer's absolute peak area within the range of 5 to 20 µg.

### Example 17: Anion-exchange (AEX)-HPLC analysis of DP, Ref and DS

A 40 µL injection volume with 1 mg/mL DS (rhAAT) were diluted in mobile phase A resulting in 40 µg load. **Figure 21** reveals that the elution profile of DS lacks an obvious main peak and shoulder and it significantly differs from Prolastin and reference. The profile of DS suggests a nonhomogeneous sample, probably due to post-translational modifications, such as variations in glycosylation patterns. Main peak and shoulder assigned were based on the elution time of reference.
- Main Peak: 15.2 %, • Shoulder: 4.1 %, • Basic Var.: 64.3 %, • Acidic Var.: 16.4 %

To verify the observation, unconcentrated DS (thawed and directly injected) was used as a control. The elution profile of unconcentrated DS also exhibits differences indicating that change in profile change is not caused by up-concentration.

### Example 18: Anion-exchange (AEX)-HPLC analysis of stressed and unstressed DS samples

The DS sample was subjected to stress by storing it at 40°C for 5 days and 5 freeze-thaw cycles, respectively. **Figure 22** illustrates an increase in basic variants after 40°C incubation resulting in a changed pattern (indicated by the blue arrow). The basic variants were also slightly increased after freeze-thaw stress. Table 21 presents peak distribution of unstressed and stressed samples.

**Table 21: Peak distribution of unstressed and stressed samples**

| | unstressed | 5d 40 °C | 5 x FT |
|---|---|---|---|
| Main peak | 15.2 % | 8.7 % | 15.3 % |
| Shoulder | 4.1 % | 4.6 % | 4.0 % |
| Basic variants | 64.3 % | 71.7 % | 65.0 % |
| Acidic variants | 16.4 % | 15.0 % | 15.8 % |

### Example 19: Anion-exchange (AEX)-HPLC analysis of stability of monomer related peak area

The DS sample was kept at 5 ± 1°C in the autosampler for a minimum of 48 h before analysis. **Figures 23-25** illustrates the linearity of monomer's absolute peak area within the range of 10 to 80 µg. Therefore, DS sample remains stable at 5 ± 1°C in autosampler for at least 48 h.

### Discussion of examples 14-19:

SE-HPLC analysis of Prolastin and reference revealed the presence of multiple HMWS and a slight LMWS shoulder. In comparison, the SE-HPLC of DS demonstrated a shift in the elution implying a smaller size of the DS, potentially attributed to diverse post-translational modifications.

Furthermore, AEX-HPLC was established and exhibited enhanced resolution of charged variants compared to the original method. The profiles of Prolastin and reference displayed similar patterns, characterized by a prominent main peak accompanied by a slight shoulder and additional charged variants. In contrast, the AEX-HPLC of DS revealed multiple peaks, without a distinguishable main peak, suggesting charge non-homogeneity of the sample, possibly attributable to heterogeneous post-translational modifications.

The above methods indicate the presence of heterogeneity in the samples, particularly in terms of charge and size, with DS displaying more pronounced variations.

### Examples regarding activity and glycosylation of rhAAT:

### Example 20: Characterization of anti-proteolytic activity of recombinant rhAAT

The aim of the experiment is to evaluate the anti-proteolytic activity of rhAAT compared to Prolastin, a human plasma-derived A1AT formulation.

### Material and methods:

Protein concentration measurement: Rec human AAT (rhAAT) according to the present invention was received and protein concentration measured using Pierce^{™} Rapid Gold BCA Protein Assay Kit (Thermo Fisher #A53226) according to the manufacturer's instructions. The assay is based on the protein-dependent reduction of copper ions which can be detected colometrically at 480 nm in a VERSAMax microplate reader with SoftMax Pro 7.0.3 software. To this end, defined dilutions of the bovine serum albumin (BSA) standard of the kit in TM buffer (150 mM NaCl, 50 mM Hepes, 5 mM EDTA and 1 % Triton (v/v)) were prepared. RhAAT and a 5 mg/ml Prolastin^{®} control sample were diluted 1:1 and 1:4 in TM buffer for measurement. Concentration was calculated based on obtained BSA standard of the kit and the dilution factor.

Neutrophil Elastase activity assay: Neutrophil Elastase activity was measured by mixing 25 µl of serially diluted Prolastin^{®} (Grifols), Camostat mesylate (Sigma Aldrich #SML0057) or rhAAT with 25 µl of 2 ng/µl recombinant neutrophil elastase (Merck Millipore #324681) in assay buffer (50 mM Tris, 1 M NaCl, 0.05 % (w/v) Brij-35, pH 7.5) for 15 min at 37°C. Next, 50 µl of 200 µM of MEOSUC-Ala-Ala-Pro-Val-AMC substrate (Bachem #4005227) was added and incubated at 37°C. Fluorescence intensity was measured after 5 minutes at an excitation wavelength of 380 nm and emission wavelength of 460 nm in a SynergyTM H1 microplate reader (BioTek) with Gen5 3.04 software.

TMPRSS2 activity assay: For assessing the activity of recombinant human TMPRSS2, 25 µl of serially diluted Prolastin^{®} (Grifols), NIBSC Standard Alpha-1-Antitrypsin, plasma derived (1st International standard) (#05/162 NIBSC), Camostat mesylate (Sigma Aldrich #SML0057) or rhAAT was incubated with 25 µl of 2 µg/ml recombinant TMPRSS2 enzyme (CusaBio #YP023924HU) in assay buffer (50 mM Tris-HCl, 0.154 mM NaCl pH 8.0) for 15 min at 37 °C. In a next step, 50 µl of 20 µM BOC-Gln-Ala-Arg-AMC protease substrate (Bachem #4017019) was added and incubated for 2 h at 37°C. Fluorescence intensity was measured after 2 h at an excitation wavelength of 380 nm and emission wavelength of 460 nm in a SynergyTM H1 microplate reader (BioTek) with Gen5 3.04 software.

### Results:

An rhAAT sample and a 5 mg/ml Prolastin^{®} control sample were diluted 1:1 and 1:4 in duplicates. Concentrations were calculated based on the BSA standard of the kit and the dilution factor. Table 22 depicts the optical densities (ODs) measured and the corresponding calculated protein concentration in mg/ml. This revealed a protein concentration of ~2.5 mg/ml for the rhAAT sample.

**Table 22: Optical densities and corresponding protein concentration of the measured samples. Protein concentration of the samples were calculated based on the BSA standard curve (Figure 26) and the dilution factor of the sample.**

| **Sample (dilution)** | **OD₄₈₀ nm** | **Protein concentration** |
|---|---|---|
| Prolastin^{®} (1:1) | 1.39 | 5.1 |
| Prolastin^{®} (1:4) | 0.69 | 5.1 |
| recA1AT (1:1) | 0.72 | 2.6 |
| recA1AT (1:4) | 0.33 | 2.4 |

### rhAAT inhibits neutrophil elastase activity similar to Prolastin^{®}:

The main physiologic role of AAT is the inhibition of neutrophil elastase, a protease that is released by immune cells. The present invention provides a recombinant alternative to human plasma-derived antitrypsin for replacement therapy as well as for treatment of respiratory virus infection. To determine whether rhAAT possesses antiproteolytic activity, we evaluated rhAAT in an in-vitro neutrophil elastase activity assay along with Prolastin^{®} and Camostat mesylate, a small molecule inhibitor of the cellular protease TMPRSS2 and SARS-CoV-2 infection. The assay employs a peptide substrate linked to 7-amino-4 methyl coumarin (AMC), which is quenched by the amid bond of the adjacent amino acid. When neutrophil elastase cleaves the bond, the fluorescent AMC is released. Consequently, the addition of an inhibitor will inhibit neutrophil elastase and reduce the fluorescence signal. As depicted in **Figure 27****,** rhAAT dose-dependently inhibits neutrophil elastase activity with an IC50 of 16.6 nM, similar to Prolastin^{®}. As expected, the small molecule TMPRSS2 inhibitor Camostat mesylate did not inhibit the activity of neutrophil elastase.

### rhAAT inhibits TMPRSS2 activity similar to Prolastin^{®} and the NIBSC standard:

Besides its physiological role in regulating elastase activity, alpha-1-antitrypsin was also shown to inhibit TMPRSS2, a protease involved in viral entry of e.g. SARS-CoV-2. Therefore, the ability of rhAAT to inhibit recombinant TMPRSS2 was assessed analogous to the neutrophil elastase measurement. As an additional control, the National Institute for Biological Standard (NIBSC) antitrypsin was used as an international reference standard for the activity of alpha-1-antitrypsin formulations. As depicted in **Figure 28****,** rhAAT showed a dose-dependent inhibition of TMPRSS2 activity, similar to Prolastin^{®} and the NIBSC standard.

Table 23 summarizes the concentrations of the compounds required to achieve 50% or 90 % inhibition (IC50 and IC90) of enzyme activity in each assay. IC50 and IC90 were calculated using a non-linear regression model in GraphPad Prism 9.

**Table 23: Inhibitory concentrations (IC50 and IC90) of tested compounds in neutrophil elastase and TMPRSS2 activity assay in nanomolar. - = inactive, n.d.= not determined**

| | **TMPRSS2 IC50** | **TMPRSS2 IC90** | **Neutrophil Elastase IC50** | **Neutrophil Elastase IC90** |
|---|---|---|---|---|
| recA1AT | 74.1 nM | 330.6 nM | 16.6 nM | 36.5 nM |
| Prolastin^{®} | 79.0 nM | 340.9 nM | 14.8 nM | 31.1 nM |
| Camostat mesylate | 1.2 nM | 9.2 nM | - | - |
| NIBSC standard | 51.8 nM | 267.4 nM | n.d. | n.d. |

### Example 21: Analysis of glycosylation pattern of rhAAT produced according to the present invention

The rhAAT protein produced by preceding examples was analysed by a peptide mapping approach, which combines proteolytic digests by trypsin and trypsin/GluC double digest with RP-LC-MS/MS analysis. Digested peptides are separated by reversed phase chromatography and further analysed using high-resolution tandem mass spectrometry. N-linked glycans are subsequently analysed in a second experiment comprising enzymatic glycan release, chemical labeling, LC separation on graphitized carbon or HILIC columns and MS analysis.

### Methods:

Glycosylation Site Determination was performed by HPLC-ESI-MS and -MS/MS measurement after enzymatic digestion. The HPLC-ESI-MS/MS data were screened for N-glycosylated peptides.

To remove interfering buffer components, sample was rebuffered by ultrafiltration with Nanosep Centrifugal Ultrafiltration devices (Pall Life Science) according to the manufacturers' instructions against a denaturing buffer (0.2 M Histidine-HCl, 5.6 M Guanidine HCl, pH 6) for denaturation.

The samples were reduced with TCEP at 37°C.

The sample was rebuffered by ultrafiltration against a digestion buffer (0.02 M Histidine-HCl, 0.5 mM TCEP, pH 6.0) using Zeba^{™} Spin Desalting Columns (Thermo Scientific) according to the manufacturers' instructions. The protein concentration was determined by UV measurement (Absorption at 280nm).

The protein sample was proteolytically cleaved with following digestion strategies: (1) Trypsin and (2) Trypsin/GluC at enzyme specific conditions.

One part of the digest were enzymatically deglycosylated with EndoH (NEB) at enzyme specific conditions. A negative sample without the addition of EndoH was prepared.

HPLC-ESI-MS and -MS/MS mass spectra were obtained using the UltiMate^{®} 3000 system (Thermo Fisher Scientific) coupled to a Q Exactive Orbitrap Plus mass spectrometer (Thermo Fisher Scientific). The separation of the peptides was performed with reversed-phase (RP) chromatography on an Accucore RP - MS LC column (2.1 x 100 mm, 2.6 µm particle size, Thermo Fisher Scientific). Eluents were A: water with 0.1 % formic acid; B: acetonitrile with 0.1 % formic acid. The peptides were separated using a segmented gradient from 3 % B to 36 % B in 45 min at 30°C with a flow rate of 0.4 mL/min. MS and MS/MS spectra (produced with Higher Energy Collisional Dissociation, HCD) were recorded in positive ion mode with internal mass calibration.

The MS data sets were analyzed using the ProteinScape 2 bioinformatics platform (Bruker Daltonics, Protagen AG). Protein identification was achieved by database searching. Hereby, the fragment mass spectra were matched against an in-house database consisting of the NCBI human protein database (http://www.ncbi.nlm.nih.gov/) and the manually inserted protein sequence (as provided by the customer).

The following protein modifications were considered:
- Deamidation (Asn)
- Oxidation (Met)
- HexNAc (Asn)
- Pyro-glutamate formation (Gln/Glu at peptide N-terminus)
- Acetylation (protein N-terminus)

For each peptide covering a potential N-glycosylation site (sequence motif NXS/T, X#P), extracted ion chromatograms (XIC) were generated showing Asn as well as the Asn peptide with HexNAc. The modification levels were assigned by comparing the signal intensity of the peaks matching the peptides with and without HexNAc. The sum of the signal intensities of both peptides was set to 100 %. No correction for different ionization efficacy of the peptides was carried out; the modification level in percent does not exactly reflect the amount of the peptide with or without glycosylation.

Glycosylated peptides were identified in the HPLC-ESI-MS and -MS/MS data using in house developed algorithms resulting in the annotation of the glycosylation site and the attached glycan types. Additionally, the datasets were searched with ProteinMetrics Byonic^{™} against a sequence database consisting of the customer sequence and common contaminants (e.g. sequences of used proteases, keratin) allowing N-glycosylation as peptide modification.

The Byonic^{™} search results were imported into ProteinMetrics Byologic^{®}. Here, for each glycopeptide identified by either Byonic^{™} or the in-house algorithm, extracted ion chromatograms (XIC) were generated to determine the MS signal intensity. The quantitative level of each identified glycan structure was assigned by comparing the MS signal intensity of the corresponding glycopeptides. The sum of the signal intensities of all glycopeptides with the same peptide sequence was set to 100%. The distribution of glycans attached to each site was calculated as average of the glycan distributions for each peptide covering a glycosylation site.

### Results:

The occupancy of glycans at each N-Glycosylation position is shown in Table 24. The position N46 has been 100% occupied by the glycan, whereas the positions N83 and N274 exhibit 98.12% and 67.58% occupancy, respectively (Table 25).

**Table 24: Glycosylation occupancy of the N-glycosylated positions of rhAAT:**

| **Digest** | **Sites** | **Sequence** | **Modificatio ns** | **Start AA** | **End AA** | **z** | **Calc. m/z** | **rAAT intact mass PTG (PS-A57-2023-0001) (QP190 40, QP1904 4)** |
|---|---|---|---|---|---|---|---|---|
| Trypsin | N46 | R.QLAHQSNSTNIFFS PVSIATAFAMLSLGTK .A | NGlycan/20 3.0794 | 40 | 69 | 3 | 1128,90 97 | 100,00 |
| Trypsin | N46 | R.QLAHQSNSTNIFFS PVSIATAFAMLSLGTK .A | NG lycan/20 3.0794 | 40 | 69 | 4 | 846,934 06 | 100,00 |
| Trypsin_ GluC | N46 | R.QLAHQSNSTNIFFS PVSIATAFAMLSLGTK .A | NG lycan/20 3.0794 | 40 | 69 | 3 | 1128,90 97 | 100,00 |
| Trypsin_ GluC | N46 | R.QLAHQSNSTNIFFS PVSIATAFAMLSLGTK .A | NG lycan/20 3.0794 | 40 | 69 | 4 | 846,934 06 | 100,00 |
| Trypsin | N83 | K.ADTHDEILEGLNFN LTEIPEAQIHEGFQEL LR.T | NG lycan/20 3.0794 | 70 | 101 | 4 | 974,481 26 | 97,89 |
| Trypsin_ GluC | N83 | E.GLNFNLTEIPE.A | NG lycan/20 3.0794 | 79 | 89 | 2 | 725,359 02 | 98,34 |
| Trypsin | N247 | K.YLGNATAIFFLPDE GK.L | NG lycan/20 3.0794 | 244 | 259 | 2 | 979,990 93 | 65,04 |
| Trypsin | N247 | K.YLGNATAIFFLPDE GK.L | NG lycan/20 3.0794 | 244 | 259 | 3 | 653,663 05 | 83,49 |
| Trypsin_ GluC | N247 | K.YLGNATAIFFLPDE GK.L | NG lycan/20 3.0794 | 244 | 259 | 2 | 979,990 93 | 68,39 |
| Trypsin_ GluC | N247 | K.YLGNATAIFFLPDE GKLQHLE.N | NG lycan/20 3.0794 | 244 | 264 | 3 | 860,439 12 | 56,03 |
| Trypsin | N247 | K.YLGNATAIFFLPDE GKLQHLENELTHDIIT K.F | NGlycan/20 3.0794 | 244 | 274 | 5 | 749,589 16 | 64,94 |

**Table 25: Average glycosylation occupancy of the N-glycosylated positions of rhAAT:**

| **Summary** | **rAAT intact mass PTG (PS-A57-2023-0001) (QP19040, QP19044)** | |
|---|---|---|
| Sites | Average Percent modified | StDev Percent modified |
| N46 | 100,00 | 0,00 |
| N83 | 98,12 | 0,31 |
| N247 | 67,58 | 10,01 |

Table 26 and 27 exhibit the percentage of detected glycans at each position. For example, HexNAc1 occupies 100% of the position N46, 39% of the position N83 and 83% at the position N247. Table 28 reveals the total amount of each glycosylation according to glycan, independent of glycosylation site.

**Table 26: Glycopeptide area of each glycosylated position of rhAAT**

| **Digest** | **Sites** | **Sequence** | **Modificat ions** | **Start AA** | **End AA** | **Glycan** | **rAAT intact mass PTG (PS-AS7-2023-0001) (QP19025, QP19029)** |
|---|---|---|---|---|---|---|---|
| Trypsin | N46 | R.QLAHQSNSTNIFFS PVSIATAFAMLSLGTK .A | NGlycan/ 203.0794 | 40 | 69 | HexNAc(1) | 100 |
| Trypsin_ GluC | N83 | E.GLNFNLTEIPE.A | NGlycan/ 203.0794 | 79 | 89 | HexNAc(1) | 39,200899 |
| Trypsin_ GluC | N83 | E.GLNFNLTEIPE.A | NGlycan/ 2026.686 9 | 79 | 89 | HexNAc(2) Hex(10) | 3,167098 |
| Trypsin_ GluC | N83 | E.GLNFNLTEIPE.A | NGlycan/ 2188.739 8 | 79 | 89 | HexNAc(2) Hex(11) | 6,480683 |
| Trypsin_ GluC | N83 | E.GLNFNLTEIPE.A | NGlycan/ 2350.792 6 | 79 | 89 | HexNAc(2) Hex(12) | 14,240209 |
| Trypsin_ GluC | N83 | E.GLNFNLTEIPE.A | NGlycan/ 2512.845 4 | 79 | 89 | HexNAc(2) Hex(13) | 12,668052 |
| Trypsin_ GluC | N83 | E.GLNFNLTEIPE.A | NGlycan/ 2674.898 2 | 79 | 89 | HexNAc(2) Hex(14) | 14,193006 |
| Trypsin_ GluC | N83 | E.GLNFNLTEIPE.A | NGlycan/ 2836.951 0 | 79 | 89 | HexNAc(2) Hex(15) | 8,186455 |
| Trypsin_ GluC | N83 | E.GLNFNLTEIPE.A | NGlycan/ 2999.003 9 | 79 | 89 | HexNAc(2) Hex(16) | 1,863597 |
| Trypsin | N247 | K.YLGNATAIFFLPDE GKLQHLENELTHDIIT K.F | NGlycan/ 203.0794 | 244 | 274 | HexNAc(1) | 83,492973 |
| Trypsin | N247 | K.YLGNATAIFFLPDE GKLQHLENELTHDIIT K.F | NGlycan/ 1864.634 2 | 244 | 274 | HexNAc(2) Hex(9) | 2,141415 |
| Trypsin | N247 | K.YLGNATAIFFLPDE GKLQHLENELTHDIIT K.F | NGlycan/ 2026.687 0 | 244 | 274 | HexNAc(2) Hex(10) | 4,851544 |
| Trypsin | N247 | K.YLGNATAIFFLPDE GKLQHLENELTHDIIT K.F | NGlycan/ 2188.739 8 | 244 | 274 | HexNAc(2) Hex(11) | 2,27652 |
| Trypsin | N247 | K.YLGNATAIFFLPDE GKLQHLENELTHDIIT K.F | NGlycan/ 2350.792 6 | 244 | 274 | HexNAc(2) Hex(12) | 2,1295 |
| Trypsin | N247 | K.YLGNATAIFFLPDE GKLQHLENELTHDIIT K.F | NGlycan/ 2512.845 5 | 244 | 274 | HexNAc(2) Hex(13) | 2,575995 |
| Trypsin | N247 | K.YLGNATAIFFLPDE GKLQHLENELTHDIIT K.F | NGlycan/ 2674.898 2 | 244 | 274 | HexNAc(2) Hex(14) | 1,327615 |
| Trypsin | N247 | K.YLGNATAIFFLPDE GKLQHLENELTHDIIT K.F | NGlycan/ 2836.951 0 | 244 | 274 | HexNAc(2) Hex(15) | 0,652264 |
| Trypsin | N247 | K.YLGNATAIFFLPDE GKLQHLENELTHDIIT K.F | NGlycan/ 2999.003 9 | 244 | 274 | HexNAc(2) Hex(16) | 0,552175 |

**Table 27: Glycopeptide summary of each glycosylated position of rhAAT**

| **Summary** | | **Average Percent modified** |
|---|---|---|
| **Sites** | **Glycan** | **rAAT intact mass PTG (PS-A57-2023-0001) (QP19025, QP19029)** |
| N46 | HexNAc(1) | 100,00 |
| N83 | HexNAc(1) | 39,20 |
| N83 | HexNAc(2)Hex(10) | 3,17 |
| N83 | HexNAc(2)Hex(11) | 6,48 |
| N83 | HexNAc(2)Hex(12) | 14,24 |
| N83 | HexNAc(2)Hex(13) | 12,67 |
| N83 | HexNAc(2)Hex(14) | 14,19 |
| N83 | HexNAc(2)Hex(15) | 8,19 |
| N83 | HexNAc(2)Hex(16) | 1,86 |
| N247 | HexNAc(1) | 83,49 |
| N247 | HexNAc(2)Hex(9) | 2,14 |
| N247 | HexNAc(2)Hex(10) | 4,85 |
| N247 | HexNAc(2)Hex(11) | 2,28 |
| N247 | HexNAc(2)Hex(12) | 2,13 |
| N247 | HexNAc(2)Hex(13) | 2,58 |
| N247 | HexNAc(2)Hex(14) | 1,33 |
| N247 | HexNAc(2)Hex(15) | 0,65 |
| N247 | HexNAc(2)Hex(16) | 0,55 |

**Table 28: Glycopeptide summary for total amount of each glycosylation according to glycan, independent of glycosylation site.**

| **Glycan** | **% amount of each glycan, independent of glycosylation site** |
|---|---|
| HexNAc(1) | 73,35% |
| HexNAc(2)Hex(9) | 0,54% |
| HexNAc(2)Hex(10) | 2,40% |
| HexNAc(2)Hex(11) | 2,97% |
| HexNAc(2)Hex(12) | 5,80% |
| HexNAc(2)Hex(13) | 5,33% |
| HexNAc(2)Hex(14) | 5,58% |
| HexNAc(2)Hex(15) | 3,19% |
| HexNAc(2)Hex(16) | 0,83% |

### Example 22: rhAAT for the treatment of acute respiratory distress syndrome (ARDS)

### Study design

In vitro: In vitro, the binding ability as well as capacity of the recombinant alpha-1 antitrypsin to reduce IL-6 and IL- 8 as well as to TNF-α is demonstrated.

In vivo: This is an animal experimental setup using a mouse model. Acute lung failure is provoked by inhalation of lipopolysaccharide (LPS). rhAAT therapy or comparative Prolastine therapy is administered at different doses at different time points. After sacrifice of the experimental animals at different time points, the concentration of cytokines is measured in the blood as well as in the lining fluid of the lungs and the lung tissue. Simultaneously, histological examination is performed to quantify the cellular migration of monocytes and macrophages. In the course of this histological workup, a morphological quantification of the interstitial oedema and the degree of destruction of the lung tissue is performed as well as the detection of micro thrombi in the small blood vessels. A group of surviving test animals is preserved for six months. At this time, histological examination of the lung tissue is performed to quantify any pulmonary fibrosis that has developed.

Each group consists of six experimental animals.
Group 1: Inhalation daily once 50 µg rhAAT/data collection on the third day.
Group 2: Inhalation daily twice 50 µg rhAAT/data collection on the third day.
Group 3: Inhalation daily once 50 µg rhAAT/data collection on the fifth day.
Group 4: Inhalation daily twice 50 µg rhAAT/data collection on the fifth day
Group 5: control group (no inhalation of rhAAT)/data collection on the third day
Group 6: control group (no inhalation of rhAAT)/data collection on the fifth day
Group 7: inhalation daily once 50 µg rhAAT for one week/data collection after 6 months
Group 8: inhalation daily twice 50 µg rhAAT for one week/data collection after 6 months
Group 9: control group (no inhalation of rhAAT), data collection after 6 months

*Primary endpoints:* The primary endpoints are the laboratory and histological data collected at the respective study time points.

*Secondary endpoints:* The secondary endpoints are the general health observations of the study animals. This includes a transdermal measurement of oxygen saturation.

### Reduction of inflammatory cytokines

*In vitro:* after rhAAT treatment vs. prior to rhAAT treatment, or rhAAT treatment in comparison to no rhAAT treatment or Prolastine treatment (controls)
IL-1Ra: 0.5 ng/mL from 6 ng/mL
IL-6: 10 ng/mL from 50 ng/mL
IL-8: 5 ng/mL, from 70 ng/mL
IL-1B: 0.5 ng/mL, from 1 ng/mL

Treatment with rhAAT *in vitro* is expected to result in a 10- to 15-fold reduction of the concentration of IL-1Ra in whole blood, e.g., a 12-fold reduction from 6 ng/mL to 0.5 ng/mL. Treatment with rhAAT *in vitro* is expected to result in a 1.5- to 10-fold reduction of the concentration of IL-6 in whole blood, e.g., a 5-fold reduction from 50 ng/mL to 10 ng/mL. Treatment with rhAAT *in vitro* is expected to result in a 10- to 20-fold reduction of the concentration of IL-8 in whole blood, e.g., a 14-fold reduction from 70 ng/mL to 5 ng/mL. Treatment with rhAAT *in vitro* is expected to result in a 1.5- to 10-fold reduction of the concentration of IL-1B in whole blood, e.g., a 2-fold reduction from 1 ng/mL to 0.5 ng/mL.

### Pathomechanism of ARDS and treatment with rhAAT:

In the early phase of ARDS, there is a significant increase in proinflammatory cytokines and in the infiltration of leukocytes into the lung tissue. Here, a positive feedback loop occurs between tumor necrosis factor alpha (TNF-α) and IL-1β, IL-6, and IL-8. A rapid increase correlates with a poor prognosis for patients (Pugin et al., Am J Respir Crit Care Med. 1996). TNF-α as well as IL-1β stimulate IL-6 and IL-8 production which attract leukocytes into the alveolar cleft. At the same time, TNF-α disrupts the alveolar barrier and leads to pulmonary oedema (Dada et al, Adv Exp Med Biol. 2007). Already in the early phase of the disease, there is a strong accumulation of neutrophils in the alveolar space leading to cell death of lung tissue. The concentration of neutrophil elastase correlates directly with the severity of the disease and its prognosis (Perl et al., Expert Rev Respir Med. 2011). In the extracellular fluid there is an accumulation of neutrophil extracellular traps (NET) which act as condensation nuclei for the formation of micro thrombi (Jarrahi et al., J Thromb Haemost. 2023). In the further course there is an interstitial accumulation of macrophages with the task of transforming the lung tissue damaged by neutrophil elastase into scarred structures. If the patient survives the early phase of the disease, a persistent, severe disturbance of lung function develops along this pathway.

Inhalation therapy of acute respiratory failure with alpha-1 antitrypsin is thus a promising treatment approach, for example by application using an inhaler, such as a vibrating mesh inhaler with a defined droplet size and flow, allowing sufficiently high concentrations of rhAAT to be achieved even in deep sections of the lung. The pharmacological effect unfolds on the cellular level as well as by influencing immunological cascades. IL-6, IL-8 and TNF-α are influenced as central elements of the inflammatory response. Thus, in the early phase of the disease, interstitial pulmonary oedema is reduced and thus gas exchange is improved. At the same time, the integrity of the endothelial cells is protected. In the long term, the development of pulmonary fibrosis is prevented or at least contained.

### Example 23: rhAAT for the treatment of Bronchiolitis obliterans syndrome (BOS)

A cytokine-induced inflammation system using the human lung epithelial cells (A549) is established and the effect of rhAAT on the expression of inflammatory cytokines is analyzed.

The cytokine-induced inflammation system using the human lung epithelial cells (A549) includes:
- Set up of a human lung epithelial cell (A549)-stimulation system using a cytokine mixture (IL-1β, IFN-_{Y} and TNF-α).
- Measurement of inflammatory cytokines by cytometric bead array (IL-6, IL-8, etc.).
- Measurement of cell viability after stimulation by XTT-assay.
- Analysis of the effect of rhAAT on inflammatory cytokines and cell viability in the A549-stimulation system in a time and dose dependent manner.
- Analysis of the effect of AAT on the global miRNA expression profile next generation sequencing.

### Set up a human lung epithelial cell (A549)-stimulation using a cytokine mixture.

A549 cells are cultured in RPMI 1640 +2 mM Glutamine + 10-20% Fetal Calf Serum (FCS) and stimulated by increasing concentrations of a cytokine mixture (CM) containing IL-1β, IFN-_{Y} and TNF-α for 24 hours (Fig.32). The cytokines are used in the following concentrations: IL-1β 0.01, 0.05, 0.1, 0.5, 1.0, 5.0, 10, 50, and 100 U/ml; IFN-_{Y} 0.08, 0.4, 0.8, 4.0, 8.0, 40, 80, 400, and 800 U/ml and TNF-α 0.004, 0.02, 0.04, 0.2, 0.4, 20, and 40 ng/ml. Supernatants are removed and stored at -80o C until analysis. Cell survival is analyzed by XTT assay.

### Measurement of inflammatory cytokines by cytometric bead array (CBA):

The inflammatory cytokines IL-6 and IL-8 in the supernatants of the cell culture system are analyzed by the Cytometric Bead Array (CBA) technology, using the BD^{™} CBA Flex Set System (BD Bioscience-PharMingen, San Diego, CA, USA). Samples are measured using the BD FACSVerseTM flow cytometer and analyzed using Array Software v3.0 (BD Bioscience-PharMingen, San Diego, CA, USA). A second aliquot is kept for a possible measurement of further cytokines e.g., IL-10, IL-12, IL-15, IL-18, IP10, MCP1, RANTES or others.

### Measurement of cell viability after stimulation by XTT-assay:

Viability of the A549 cells after stimulation with the CM and/or treatment with Prolastin and rhAAT is analyzed by XTT assay (2,3-Bis-(2-Methoxy-4-Nitro-5-Sulfophenyl)-2H-Tetrazolium-5-Carboxanilide) after stimulation by XTT-assay according to manufacturer's instructions.

### Analysis of the effect of rhAAT in comparison to Prolastin on inflammatory cytokines and cell viability in the A549-stimulation system in a time and dose dependent manner:

A549 cells are cultured in RPMI 1640 + 2 mM Glutamine + 10-20% Fetal Calf Serum (FCS) RPMI and stimulated with selected concentrations of the cytokine mixture (CM) for 24 hours. To test the effect of the AATs on inflammatory cytokines and cell viability, different doses of Prolastin or rhAAT are added to the CM-stimulated A549. II-6 and IL-8 are analyzed in the supernatants by Cytometric Bead Array (CBA) and cell viability by the XTT assay, respectively.

IL-8 Production of A549 cells after stimulation with increasing concentration of the cytokine Mix (CM) containing IL-1β, IFN-βand TNF-α and incubation with different concentration of rhAAT was analyzed by CBA (Fig. 33). Upon increasing concentration of the cytokine mix (CM6 and CM8) a reduction of the concentration of IL-8 was observed upon treatment with rhAAT in comparison to no treatment. The concentration of IL-8 was reduced more strongly at higher concentration of rhAAT.

### Reduction of inflammatory cytokines:

*In vitro:* after rhAAT treatment vs. prior to rhAAT treatment or rhAAT treatment in comparison to no rhAAT treatment or Prolastine (controls):
IL-1Ra: 0.5 ng/mL from 6 ng/mL
IL-6: 10 ng/mL from 50 ng/mL
IL-8: 5 ng/mL, from 70 ng/mL
IL-1B: 0.5 ng/mL, from 1 ng/mL

*In patients:* after rhAAT treatment vs. prior to rhAAT treatment or rhAAT treatment in comparison to no rhAAT treatment (control):
IL-1B: 1 pg/mL vs 1.6 pg/mL
II-6: 50 ng/L vs 100 ng/L
IL-6: 17 pg/mL vs 70 pg/mL

Treatment with rhAAT *in vitro* is expected to result in a 10 to 15-fold reduction of the concentration of IL-1Ra in whole blood, e.g., a 12-fold reduction from 6 ng/mL to 0.5 ng/mL. Treatment with rhAAT *in vitro* is expected to result in a 1.5- to 10-fold reduction of the concentration of IL-6 in whole blood, e.g., a 5-fold reduction from 50 ng/mL to 10 ng/mL. Treatment with rhAAT *in vitro* is expected to result in a 10- to 20-fold reduction of the concentration of IL-8 in whole blood, e.g., a 14-fold reduction from 70 ng/mL to 5 ng/mL. Treatment with rhAAT *in vitro* is expected to result in a 1.5- to 10-fold reduction of the concentration of IL-1B in whole blood, e.g., a 2-fold reduction from 1 ng/mL to 0.5 ng/mL.

Treatment with rhAAT in patients is expected to result in a in a 1.5- to 5-fold reduction of the concentration of IL-1B in serum, e.g., a 1.6-fold reduction from 1.6 pg/mL to 1 pg/mL. Treatment with rhAAT in patients is expected to result in a 1.5- to 10-fold reduction of the concentration of IL-6 in serum or plasma, e.g., a 2-fold reduction from 100 ng/L to 50 ng/L in serum and a 4-fold reduction from 70 pg/mL to 17 pg/mL in plasma.

### Analysis of the effect of AAT on the global miRNA expression profile using next generation sequencing:

Total RNA, including miRNA, is isolated using the Qiagen miRNA Kit (Qiagen, Hilden Germany) according to the manufacturer's instructions. RNA concentrations are determined using the Nanodrop Lite spectrometry (Thermo Scientific, Dreieich, Germany) and RNA quality is assessed using the Agilent RNA 6000 Nano Kit and Agilent 2100 Bioanalyzer (Agilent Technologies, Santa Carla, CA, USA). The miRNA libraries are prepared using QIAseq miRNA Library Kit (Qiagen, Hilden, Germany). Then, cDNA concentration is measured using the Qubit dsDNA Assay Kit, Qubit Assay Tubes, and Qubit 3.0 Fluorometer (all from Thermo Fisher Scientific, Dreieich, Germany). DNA quality is validated with Agilent High Sensitivity DNA Reagents and Agilent 2100 Bioanalyzer (Agilent Technologies, Santa Carla, CA, USA) using High Sensitivity DNA Chips. Next generation sequencing (NGS) is performed using the MiSeq Regent Kit v3, PhiX Sequencing Control v3, and the MiSeq ^{™} Desktop Sequencer (All Illumina Inc., San Diego, CA, USA), and data are preprocessed using the Quiagen Web Portal Service, which converted coverage files into raw data matrices (https://geneglobe.qiagen.com/de/analyze). Differential expression analysis is performed in RStudio 1.2.1335 (https://cran.r-project.org/).

### Investigations on the inflammatory pathomechanism of post infectious BO (PiBO) patients:

A pathomechanism of BO such as PiBO is conceivable in which an infiltration of the submucosa of the airways through leukocytes occurs. A cascade of inflammatory cytokines and mediators leads to an accumulation of inflammatory cells. Rosewich et al. showed a predominant neutrophilic inflammatory reaction in the bronchioles with increased concentrations of inflammatory cytokines such as IL-1β, IL-6, IL-8 and TNF-α (Rosewich et al., Cytokine 2015). In addition, observations in induced sputum of PiBO patients demonstrated ongoing neutrophilic inflammation over time without termination (Eckrich et al., Lung 2016 (Figure 29). Recent investigations also showed an upregulation of IL-33 in the sputum of the patients pointing to an involvement of alveolar epithelial type II cells (AEC-II) in the inflammatory process (Kriszeleit et al., doctoral thesis, Wolfgang Goethe Universität Frankfurt am Main 2023) (Figure 29 E). AEC-II cells are damaged by many cellular and humoral inflammatory mediators in the development and progression of inflammatory induced lung injury. Vice versa, AEC-II are themselves able to amplify lung inflammation by producing inflammatory cytokines and chemokines, leading to the activation and recruitment of phagocytes and neutrophils (Standifors et al., J Clin Invest 1990).

Next generation sequencing (NGS) analysis in the sputum of BO patients revealed dysregulated miRNAs which are functionally implicated in cytokine-cytokine receptor interaction, TGF-β signalling and FoxO signalling pathway and significantly correlated with lung function values (FEV1) (Duecker et al., Clin Trans Immunology 2022). Analysis revealed a strong correlation of the miRNAs miR-335-5p, miR-30b-5p, miR-186-5p and miR-30c-5p and the lung function parameter FEV1. The data also showed a significant up-regulation of miR-335-5p and miR-186-5p in sputum of these patients compared to controls (Figure 30). The role of these miRNAs in the regulation in inflammatory processes was confirmed by in vitro experiments on IL-6 protein levels in stimulated A549 cells. Using an in vitro inflammation stimulation model, increasing IL-6 production as well as up-regulated expression of miR-335-5p, miR-30b-5p, miR-186-5p and miR-30c-5p in a dose dependent manner were observed (Figure 31)

Given the uncontrolled inflammation in BO lung disease, resolution of the chronic inflammatory response is of critical importance (Jerkic et al., Clin Trans Immunology 2022). In this regard inhalative therapy with rhAAT is a promising treatment approach due to its effect on neutrophile elastase activity and anti-inflammatory capabilities that extend beyond its antiprotease role.

### Example 24: rhAAT for the treatment of Asthma:

### Ovalbumin (OVA)-induced mouse model of asthma:

Female BALB/c mice (10-12 weeks-old) are randomly divided into two groups, the control and asthma groups. The mice are sensitized by intraperitoneal injections of 20 µg of OVA and 1 mg of alum on day 0 and day 7. Two weeks after OVA sensitization, the mice are administered a 1% OVA aerosol by inhalation on days 21, 22, 23, and 24. For the control group, PBS is used instead of OVA.

rhAAT therapy or comparative Prolastine therapy is administered at different doses at different time points by inhalation or intraperitoneal injection. After sacrifice of the experimental animals at different time points, the concentration of cytokines is measured in the blood as well as in the lining fluid of the lungs and the lung tissue. The concentrations of IL-4, IL-5 and IFN-γ in the bronchoalveolar lavage fluid (BALF) are determined using ELISA. For bronchoalveolar lavage PBS is loaded into a syringe, injected and aspirated four times in the catheter. The BALF is stored on ice. Total BALF is centrifuged and the supernatant is used for the assessment of IL-4, IL-5 and IFN-y.

Simultaneously, histological examination is performed to quantify the cellular migration of monocytes and macrophages. In the course of this histological workup, a morphological quantification of the interstitial oedema and the degree of destruction of the lung tissue is performed as well as the detection of micro thrombi in the small blood vessels. A group of surviving test animals is preserved for six months. At this time, histological examination of the lung tissue is performed to quantify any pulmonary fibrosis that has developed.

Each group consists of six experimental animals.
Group 1: Inhalation daily once 50 µg rhAAT/data collection on the third day.
Group 2: Inhalation daily twice 50 µg rhAAT/data collection on the third day.
Group 3: Inhalation daily once 50 µg rhAAT/data collection on the fifth day.
Group 4: Inhalation daily twice 50 µg rhAAT/data collection on the fifth day
Group 5: control group (no inhalation of rhAAT)/data collection on the third day
Group 6: control group (no inhalation of rhAAT)/data collection on the fifth day
Group 7: inhalation daily once 50 µg rhAAT for one week/data collection after 6 months
Group 8: inhalation daily twice 50 µg rhAAT for one week/data collection after 6 months
Group 9: control group (no inhalation of rhAAT), data collection after 6 months

*Primary endpoints:* The primary endpoints are the laboratory and histological data collected at the respective study time points.

*Secondary endpoints:* The secondary endpoints are the general health observations of the study animals. This includes a transdermal measurement of oxygen saturation.

### Reduction of inflammatory cytokines

*In vivo (OVA mice model):* after rhAAT treatment vs. prior to rhAAT treatment or rhAAT treatment in comparison to no rhAAT treatment or Prolastin (controls):
IL-4: 200 pg/mL from 500 pg/mL
IL-5: 100 pg/mL from 250 pg/mL
IFN-y: 125 pg/mL, from 200 pg/mL

Treatment with rhAAT *in vivo* is expected to result in a 1.5- to 10-fold reduction of the concentration of IL-4 in BALF, e.g., a 2.5-fold reduction from 500 pg/mL to 200 pg/mL. Treatment with rhAAT *in vivo* is expected to result in a 1.5- to 10-fold reduction of the concentration of IL-5 in BALF, e.g., a 2.5-fold reduction from 250 pg/mL to 100 pg/mL. Treatment with rhAAT *in vivo* is expected to result in a 1.2- to 10-fold reduction of the concentration of IFN-γ in BALF, e.g., a 1.6-fold reduction from 200 pg/mL to 125 pg/mL.

## Claims

1. A human recombinant AAT (rhAAT) protein or fragment thereof, expressed in a genetically modified yeast, for use in the treatment and/or prevention of a non-viral disease of the lung associated with inflammation and/or a pathological immune response.

2. The rhAAT protein or fragment thereof for use according to claim 1, wherein the disease of the lung associated with inflammation and/or a pathological immune response is associated with an increased level of inflammatory cytokines, such as IL-1β, IL-1Ra, IL-4, IL-5, IL-6, IL-8, IFN-_{Y} and/or TNF-α, an increased level of one or more transcription factors such as NF-_{K}B and/or increased immune cell infiltration in the lung tissue of a subject compared to a healthy subject.

3. The rhAAT protein or fragment thereof for use according to any one of the preceding claims, wherein the disease of the lung as is asthma.

4. The rhAAT protein or fragment thereof for use according to any one of claims 1-2, wherein the disease of the lung is acute respiratory distress syndrome (ARDS).

5. The rhAAT protein or fragment thereof for use according to any one of claims 1-2, wherein the disease of the lung is bronchiolitis obliterans (BO).

6. The rhAAT protein or fragment thereof for use according to the preceding claim, wherein the bronchiolitis obliterans (BO) is associated with lung transplantation and/or allogenic hematopoietic stem cell transplantation (chronic graft-versus host disease).

7. The rhAAT protein or fragment thereof for use according to any one of the preceding claims, wherein the rhAAT protein or fragment thereof comprises a sequence according to SEQ ID NO 4, or is encoded by SEQ ID NO 1, 2 or 3, or a sequence with an at least 80% identity thereto.

8. The rhAAT protein or fragment thereof for use according to any one of the preceding claims, wherein the rhAAT protein or fragment thereof has a serum half-life, pulmonary half-life and/or activity not less than AAT purified from human plasma.

9. The rhAAT protein or fragment thereof for use according to any one of the preceding claims, wherein the rhAAT protein or fragment thereof is expressed in a yeast of the family *Saccharomycetaceae,* preferably the family *Pichia.*

10. The rhAAT protein or fragment thereof for use according to any one of the preceding claims, wherein the rhAAT protein or fragment thereof comprises post-translational modifications.

11. The rhAAT protein or fragment thereof for use according to any one of the preceding claims, wherein the post-translation modification is N-glycosylation, O-glycosylation, N-terminal methionine removal, N-acetylation and/or phosphorylation, or any combination thereof, preferably comprising one or more N-linked glycosylations, comprising at least one HexNAc1 glycosylation.

12. The rhAAT protein or fragment thereof for use according to any one of the preceding claims, wherein the rhAAT protein or fragment thereof is administered by inhalation, preferably as a solution suitable for inhalation, soluble powder suitable for inhalation or dry powder suitable for inhalation.

13. The rhAAT protein or fragment thereof for use according to any one of the preceding claims, wherein the rhAAT protein or fragment thereof is administered by inhalation of a nebulized solution.

14. A pharmaceutical composition comprising the rhAAT protein or fragment thereof according to any one of the preceding claims and a pharmaceutically acceptable carrier, for use in the treatment of a non-viral disease of the lung associated with inflammation and/or a pathological immune response.

15. The pharmaceutical composition according to the preceding claim, wherein the composition is a solution suitable for inhalation, a soluble powder suitable for inhalation or a dry powder suitable for inhalation.
